# EUROPEAN PATENT SPECIFICATION

(11) **EP 1 721 898 B1**
(45) Date of publication and mention of the grant of the patent: **16.03.2011**
(21) Application number: 06252509.2
(22) Date of filing: 12.05.2006
(51) Int. Cl.: C07D 215/56, C07D 471/04, A61K 31/435, A61K 31/495, A61P 31/00

(54) **Quinoline and pyridoquinoxaline derivatives as antibacterial agents**
Chinolin- und Pyridochinoxalinderivate als antibakterielle Mittel
Dérivés de quinoline et pyridoquinoxaline en tant qu'agents antibactériens

(30) Priority: 12.05.2005 JO 602005
(43) Date of publication of application: 15.11.2006
(73) Proprietor: University of Jordan, 11942 Amman (JO)
(72) Inventor: Qaisi, A.M.A., University of Jordan Amman 11942 (JO); Al-Hiari, Y.M.A., University of Jordan Amman 11942 (JO); Zahra, J.A.Y., University of Jordan Amman 11942 (JO); El-Abadelah, M.M.M., University of Jordan Amman 11942 (JO)
(74) Representative: Wallace, Sheila Jane

(56) References cited:
- EP-A- 0 945 435
- NISHIMURA ET AL.: "An intramoleular cyclization of 7-Substituted 6-Fluoro-1,8-Naphthyridine and -quinoline Derivatives" J. HET. CHEM., vol. 25, 1988, pages 479-485, XP002390479

## Description

The present invention relates to certain novel pyrido[2,3-*f*]quinoxaline-8-carboxylic acid derivatives, their isomeric pyrido[2,3-*g*]quinoxaline-carboxylic acid derivatives, corresponding 4-oxo-1,4-dihydroquinoline-3-carboxylate derivatives and physiologically tolerated salts and esters thereof, a process for their preparation and these compounds for use as antibacterial agents.

The following literature references describe quinoxalinone derivatives for treating antibacterial infections:
A. Carta, M. Loriga, S. Zanetti and L. A. Sechi, Farmaco, 2003, 58, 1251-1255;
A. Carta, P. Sanna, M. Loriga, M. G. Setzu, P. La Colla and R. Loddo, Farmaco, 2002, 57, 19-25.
M. M. Ali, M. M. F. Ismail, M. S. A. El-Gaby, M. A. Zahran and Y. A. Ammar, Molecules, 2000, 5, 864-873.
P. Sanna, A. Carta, M. Loriga, S. Zanetti and L. Sechi, Farmaco 1999, 54, 161-168;
P. Sanna, A. Carta, M. Loriga, S. Zanetti and L. Sechi, Farmaco 1999, 54, 169-177;
P. Sanna, A. Carta, M. Loriga, S. Zanetti and L. Sechi, Farmaco 1998, 53, 455-461. Quinoxalinone derivatives. Nissan Chemical Industries, Ltd., Japan., *Jpn*. *Kokai Tokkyo Koho* (1982), JP 57002277**;** Chem. Abstr., 1982, 97, 438954.

EP 0 945 435 A1 discloses certain pyridonecarboxylic acid derivatives or salts thereof which exhibit antibacterial action and peroral absorbability.

Nishimura et al in J.Het.Chem., Vol. 25, 1988, pp.479-485 describe a synthesis of 1,4-oxazine and pyrazine ring systems by an intramolecular cyclization of 7-substituted 1-ethyl-6-fluoro-1,4-dihydro-4-oxo-1,8-naphthyridine and -quinoline derivatives having a nitrogen or oxygen nucleophilic site in the C-7 appendage. The in vitro antibacterial activities of compounds prepared by this method were also tested.

It has now been discovered that certain substituted pyrido[2,3-*f*]quinoxaline-8-carboxylic acid derivatives, substituted pyrido[2,3-g]quinoxaline-8-carboxylic acid derivatives, corresponding 4-oxo-1,4-dihydroquinoline-3-carboxylate derivatives and physiologically tolerated salts and esters thereof are useful as antibacterial compounds, in particular against Gram positive bacteria.

According to the invention there is therefore provided a compound of the general formula 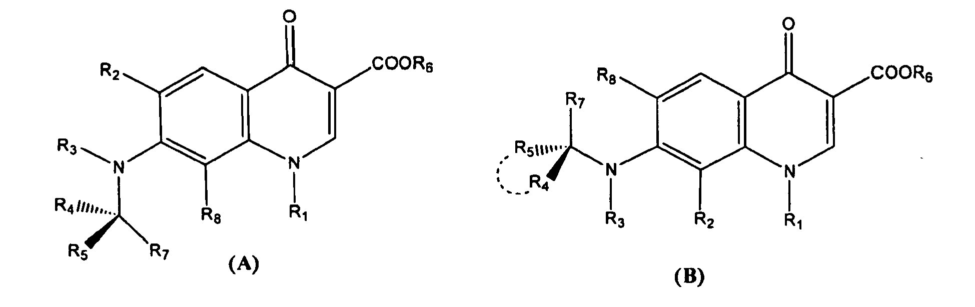 or a physiologically tolerated salt or ester thereof,
in which
R₁ represents an optionally substituted C₁₋₁₂ alkyl, C₂₋₁₂ alkenyl, C₃₋₁₂ cycloalkyl, C₁₋₁₂ alkoxy, amino, C₁₋₁₂ alkylamino, di-C₁₋₁₂ alkylamino, C₆₋₂₄ aryl or C₇₋₃₀ aralkyl group;
R₂ represents a halogen atom, a hydroxyl group or an optionally substituted C₁₋₂ alkoxy group;
R₃ represents a hydrogen atom or an optionally substituted C_{I}-₁₂ alkyl group;
R₄ and R₅ each independently represent a hydrogen atom or an optionally substituted C₁₋₁₂ alkyl, C₆₋₂₄ aryl, C₇₋₃₀ aralkyl, 3- to 18-membered heterocyclyl or 3- to 18-membered heterocyclyl-C₁₋₆ alkyl group, or
R₄ and R₅ together represent an optionally substituted alkylene group;
R₆ represents a hydrogen atom or an optionally substituted C₁₋₁₂ alkyl group;
R₇ represents a group -COOR₆, where R₆ is as defined above, and
R₈ represents a nitro group, or R₇ and R₈ together represent a group -C(O)-NR₉-, where R₉ is a hydrogen atom or an optionally substituted C₁₋₁₂ alkyl group;
optional substituents being selected from halogen atoms, nitro, cyano, hydroxyl, C₃₋₈ cycloalkyl, C₁₋₁₂ alkyl, C₁₋₁₂ haloalkyl, C₁₋₁₂ alkoxy, C₁₋₁₂ haloalkoxy, amino, C₁₋₁₂ alkylamino, di-(C₁₋₁₂ alkyl)amino, formyl, C₁₋₁₂ alkoxycarbonyl, carboxyl, C₁₋₁₂ alkanoyl, C₁₋₁₂ alkylthio, C₁₋₁₂ alkylsulphinyl, C₁₋₁₂ alkylsulphonyl, C₁₋₁₂ alkylsulphonato, carbamoyl, C₁₋₁₂ alkyamido, C₆₋₁₀ aryl and C₇₋₁₁ aralkyl groups.

Any alkyl or alkenyl group, unless otherwise specified, may be linear or branched and may contain up to 12, preferably up to 6, and especially up to 4 carbon atoms. Preferred alkyl groups are methyl, ethyl, propyl and butyl. Preferred alkenyl groups include ethenyl (vinyl), propenyl and butenyl groups. When an alkyl moiety forms part of another group, for example the alkyl moiety of an aralkyl group, it is preferred that it contains up to 6, especially up to 4, carbon atoms. Preferred alkyl moieties are methyl and ethyl.

An aryl group may be any aromatic hydrocarbon group and may contain from 6 to 24, preferably 6 to 18, more preferably 6 to 16, particularly 6 to 14, and especially 6 to 10 carbon atoms. Preferred aryl groups include phenyl, naphthyl, anthryl, phenanthryl and pyryl groups, especially a phenyl or naphthyl, and particularly a phenyl, group. When an aryl moiety forms part of another group, for example the aryl moiety of an aralkyl group, it is preferred that it is a phenyl, naphthyl, anthryl, phenanthryl or pyryl, especially phenyl or naphthyl, and particularly a phenyl, moiety.

An aralkyl group may be any alkyl group substituted by an aryl group. A preferred aralkyl group contains from 7 to 30, more preferably 7 to 24, particularly 7 to 18 and especially 7 to 11, carbon atoms, particularly preferred aralkyl groups being benzyl, naphthylmethyl, anthrylmethyl, phenanthrylmethyl and pyrylmethyl groups. A particularly preferred aralkyl group is a benzyl group.

A cycloalkyl group may be any saturated cyclic hydrocarbon group and may contain from 3 to 12, preferably 3 to 8, and especially 3 to 6, carbon atoms. Preferred cycloalkyl groups are cyclopropyl, cyclopentyl and cyclohexyl groups.

A heteroaryl group may be any aromatic monocyclic or polycyclic ring system which contains at least one heteroatom. Preferably, a heteroaryl group is a 5- to 18-membered, particularly a 5- to 14-membered, and especially a 5- to 10-membered, aromatic ring system containing at least one heteroatom selected from oxygen, sulphur and nitrogen atoms. Preferred heteroaryl groups include pyridyl, pyrylium, thiopyrylium, pyrrolyl, furyl, thienyl, indolyl, isoindolyl, indolizinyl, imidazolyl, pyridonyl, pyronyl, pyrimidinyl, pyrazinyl, oxazolyl, thiazolyl, purinyl, quinolinyl, isoquinolinyl, quinoxalinyl, pyridazinyl, benzofuranyl, benzoxazolyl and acridinyl groups. Particularly preferred heteroaryl groups include indolyl, imidazolyl, pyridyl, thienyl and furyl groups, especially 3-indolyl, 4-imidazolyl, 2-pyridyl, 2-thienyl and 2-furyl groups.

A heterocyclic group may be any monocyclic or polycyclic ring system which contains at least one heteroatom and may be unsaturated or partially or fully saturated. The term "heterocyclic" thus includes heteroaryl groups as defined above as well as non-aromatic heterocyclic groups. Preferably, a heterocyclic group is a 3- to 18-membered, more preferably a 3- to 14-membered, particularly a 3- to 10-membered, especially a 5- to 10-membered, ring system containing at least one heteroatom selected from oxygen, sulphur and nitrogen atoms. Preferred heterocyclic groups include the specific heteroaryl groups named above as well as pyranyl, piperidinyl, pyrrolidinyl, indolinyl, isoindolinyl, dioxanyl, piperazinyl, morpholinyl, thiomorpholinyl, morpholinosulphonyl, tetrahydroisoquinolinyl and tetrahydrofuranyl groups.

A heterocyclylalkyl group may be any alkyl group substituted by a heterocyclic group. Preferably, the heterocyclic moiety is a 3- to 18-membered, more preferably a 3- to 14-membered, particularly a 3- to 10-membered and especially a 5- to 10-membered, heterocyclic group as defined above and the alkyl moiety is a C₁₋₆ alkyl, preferably C₁₋₄ alkyl, and especially methyl, group.

When any of the foregoing substituents are designated as being optionally substituted, these optional substituents are selected from halogen atoms, nitro, cyano, hydroxyl, cycloalkyl, alkyl, haloalkyl, alkoxy, haloalkoxy, amino, alkylamino, dialkylamino, formyl, alkoxycarbonyl, carboxyl, alkanoyl, alkylthio, alkylsulphinyl, alkylsulphonyl, alkylsulphonato, carbamoyl, alkylamido, aryl and aralkyl groups.

When any of the foregoing optional substituents represents or contains an alkyl substituent group, this may be linear or branched and may contain up to 12, preferably up to 6, and especially up to 4, carbon atoms. A cycloalkyl group may contain from 3 to 8, preferably from 3 to 6, carbon atoms. An aryl group or moiety may contain from 6 to 10 carbon atoms, phenyl groups being especially preferred. A halogen atom may be a fluorine, chlorine, bromine or iodine atom and any group which contains a halo moiety, such as a haloalkyl group, may thus contain any one or more of these halogen atoms.

Preferred optional substituents include halogen atoms, nitro, cyano, hydroxyl, C₁₋₆ alkyl, C₁₋₆ haloalkyl, C₁₋₆ alkoxy, C₁₋₆ haloalkoxy, amino, C₁₋₆ alkylamino, di-(C₁₋₆ alkyl)amino, formyl, C₁₋₆ alkoxycarbonyl, carboxyl, C₁₋₆ alkanoyl, C₁₋₆ alkylthio, C₁₋₆ alkylsulphinyl, C₁₋₆ alkylsulphonyl, carbamoyl and C₁₋₆ alkylamido groups. Particularly preferred optional substituents include halogen atoms, nitro, cyano, hydroxyl, C₁₋₄ alkyl, C₁₋₄ haloalkyl, C₁₋₄ alkoxy and C₁₋₄ haloalkoxy groups with halogen atoms being especially preferred.

Preferably, R₁ represents a C₁₋₆ alkyl, C₂₋₆ alkenyl, C₃₋₆ cycloalkyl, C₁₋₆ alkoxy, C₁₋₆ alkylamino or C₆₋₁₄ aryl group, each group being optionally substituted by one or more substituents selected from the group consisting of halogen atoms, nitro, cyano, hydroxyl, C₁₋₄ alkyl, C₁₋₄ haloalkyl, C₁₋₄ alkoxy and C₁₋₄ haloalkoxy groups. More preferably, R₁ represents a C₁₋₄ alkyl, C₂₋₄ alkenyl, C₃₋₆ cycloalkyl, C₁₋₄ alkoxy, C₁₋₄ alkylamino or phenyl group, each group being optionally substituted by one or more substituents selected from the group consisting of halogen atoms, hydroxyl and C₁₋₄ alkoxy groups. When R₁ is a substituted phenyl group, it is preferred that substitution is at the ortho and/or para positions of the phenyl ring with para-substitution being especially preferred. It is particularly preferred that R₁ represents an ethyl, vinyl, cyclopropyl, methylamino, methoxy, hydroxyphenyl (especially parahydroxyphenyl), methoxyphenyl (especially paramethoxyphenyl), fluorophenyl (epecially ortho- or parafluorophenyl) or difluorophenyl (especially 2,4-difluorophenyl) group. It is especially preferred that R₁ represents a cyclopropyl, ethyl or 2,4-difluorophenyl group, particularly a cyclopropyl group.

It is preferred that R₂ represents a halogen atom, a hydroxyl group or a C₁₋₄ alkoxy group. More preferably, R₂ represents a fluorine or chlorine atom or a hydroxyl or C₁₋₃ alkoxy group, especially a methoxy group. It is particularly preferred that R₂ represents a fluorine atom.

Preferably, R₃ represents a hydrogen atom or a C₁₋₄ alkyl group, especially a methyl group. It is especially preferred that R₃ is a hydrogen atom.

It is preferred that R₄ and R₅ each independently represent a hydrogen atom or a C₁₋₆ alkyl, C₆₋₁₄ aryl, C₇₋₁₈ aralkyl, 3- to 10-membered heterocyclyl or 3- to 10-membered heterocyclyl-C₁₋₆ alkyl group, each group being optionally substituted by one or more substituents selected from the group consisting of halogen atoms, hydroxyl, C₁₋₄ haloalkyl, C₁₋₄ alkoxy, C₁₋₄ haloalkoxy, C₁₋₄ alkylthio, C₁₋₄ haloalkylthio, carboxyl, amino, carbamoyl, C₁₋₄ alkylamido and -NH-CO-R' groups, where R' is a hydrogen, C₁₋₄ alkyl or phenyl group, or R₄ and R₅ together represent a group -(CH₂)ₙ- where n is an integer from 2 to 5. More preferably, R₄ and R₅ each independently represent a hydrogen atom or a C₁₋₄ alkyl, C₆₋₁₀ aryl, C₇₋₁₁ aralkyl, 5- to 10-membered heterocyclyl or 5- to 10-membered heterocyclyl-C₁₋₄ alkyl group, each group being optionally substituted by one or more substituents selected from the group consisting of halogen atoms, hydroxyl, C₁₋₄ alkoxy, C₁₋₄ alkylthio, carboxyl, carbamoyl, amino, C₁₋₄ alkylamido and -NH-CO-R' groups where R' is a hydrogen, methyl or phenyl group, or R₄ and R₅ together represent a group - (CH₂)ₙ- where n is an integer from 2 to 4. When R₄ and/or R₅ represents a substituted phenyl group, it is preferred that substitution is at the ortho and/or para position of the phenyl ring with para-substitution being especially preferred.

It is particularly preferred that R₄ and R₅ each independently represent a hydrogen atom; a C₁₋₄ alkyl group optionally substituted by one or more hydroxyl, C₁₋₄ alkoxy, C₁₋₄ alkylthio, carboxyl, carbamoyl, amino or -NH-CO-R' groups where R' is a hydrogen, methyl or phenyl group; a phenyl group optionally substituted, preferably at the 2- and/or 4- position, by one or more halogen atoms, hydroxyl or C₁₋₄ alkoxy groups; a benzyl group optionally substituted, preferably at the 2- and/or 4-position of the phenyl ring, by one or more halogen atoms, hydroxyl or C₁₋₄ alkoxy groups; or a pyridyl, thienyl, furyl, indolylmethyl or imidazolylmethyl group; or R₄ and R₅ together represent a group -(CH₂)ₙ- where n is an integer from 2 to 4.

More preferably, R₄ and R₅ each independently represent a hydrogen atom; a C₁₋₄ alkyl group optionally substituted by one or more hydroxyl, C₁₋₄ alkoxy or C₁₋₄ alkylthio groups; a group -(CH₂)ₘ-COOH or -(CH₂)ₘ-CONH₂ where m is 1 or 2; a group -(CH₂)₄-NHCOR' where R' is hydrogen, methyl or phenyl; a phenyl group optionally substituted at the 2- and/or 4-position by a fluorine, chlorine, hydroxyl or methoxy group; a benzyl group optionally substituted at the 2- and/or 4-positiion by a hydroxyl group; or a 2-pyridyl, 2-thienyl, 2-furyl, 3-indolylmethyl or 4-imidazolylmethyl group.

It is especially preferred that R₄ represents a hydrogen atom or a methyl, ethyl, methylethyl (isopropyl), 1-methylpropyl, 2-methylpropyl (isobutyl), hydroxymethyl, 1-hydroxyethyl, 2-(methylthio)ethyl, carboxymethyl, 2-carboxyethyl, 4-(ethanamido)butyl, phenyl or benzyl group and R₅ represents a hydrogen atom, or R₄ and R₅ together represent a -(CH₂)₄- group.

Preferably, R₆ represents a hydrogen atom or a C₁₋₄ alkyl group, especially a methyl group.

It is preferred that R₇ represents a group -COOR₆, where R₆ is a hydrogen atom or a C₁₋₄ alkyl, especially a methyl, group, and R₈ represents a nitro group, or R₇ and R₈ together, that is, -R₇-R₈-, represent a group -C(O)-NH- thereby forming a quinoxaline ring in conjunction with the adjacent ring.

One preferred group of compounds has the Formula I or II: 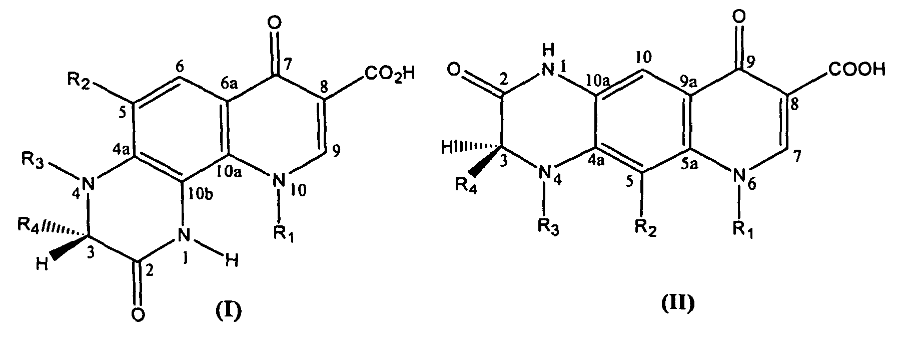 in which
R₁ = Et, vinyl, *c*-C₃H₅ (i.e. cyclopropyl), NHMe, OMe, *p*-(OH)C₆H₄, *p*-(MeO)C₆H₄, *p*-(F)C₆H₄, *o*-(F)C₆H₄, 2,4-(F)₂C₆H₃,preferably Et, *c*-C₃H₅ or 2,4-(F)₂C₆H₃, R₂ = fluorine, chlorine, hydroxyl or C₁-C₃-alkoxy, preferably F Cl, OH or OMe, R₃ = H, Me,
R₄ = H, C₁-C₄-alkyl which is optionally substituted by hydroxyl, C₁-C₄-alkoxy or C₁-C₄-alkylthio, CH₂CO₂H, CH₂CONH₂, CH₂CH₂CO₂H, CH₂CH₂CONH₂, (CH₂)₄NHCOR' [R' = H, Me, Ph], Ph, *p*-(X)C₆H₄ [X = F, Cl, OH, OMe], PhCH₂, *p*-(OH)C₆H₄CH₂, 2,4-(OH)₂C₆H₃CH₂, 3-(indolyl)CH₂ or 4-(imidazolyl)CH₂.

Another preferred group of compounds has the formula IA or IIA: 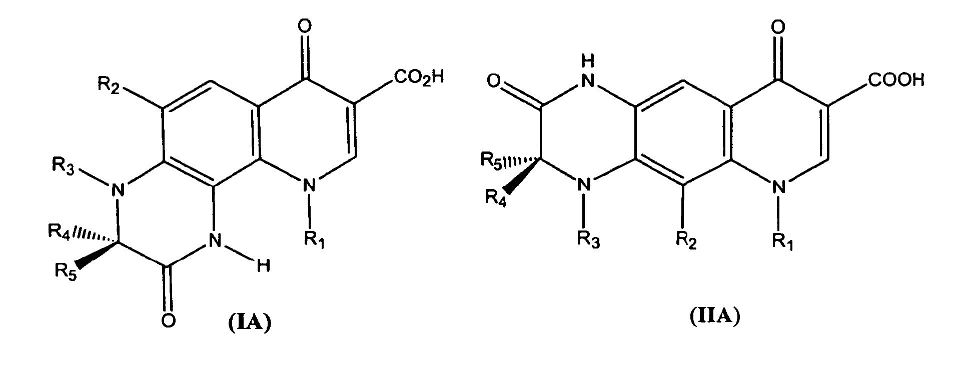 in which
R₁, R₂ and R₃ have the same meaning as mentioned in formulae **I** and **II** above, R₄ = R₅: Me, Ph, 2-Py, 2-thienyl or 2-furyl.

A further preferred group of compounds has the formula IB or IIB: 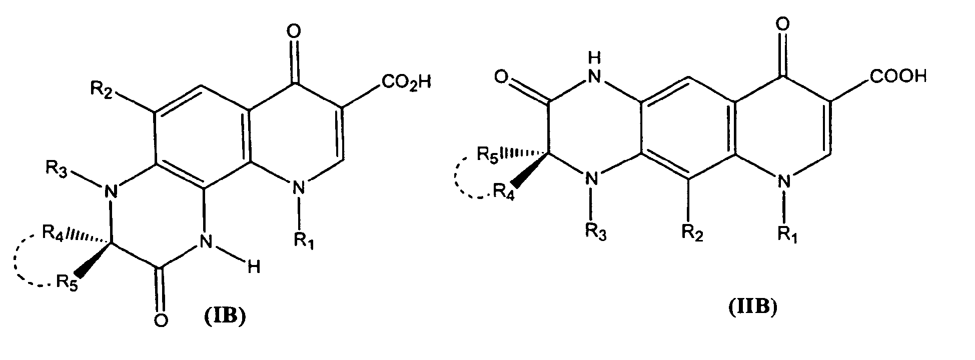 in which
R₁, R₂ and R₃ have the same meaning as mentioned in formula **I** and **II** above, R₄ and R₅ = [CH₂]ₙ, where n = 2, 3, 4 or 5.

In another a preferred group of compounds of formulae **I** and **II:**
R₁ = *c*-C₃H₅ or Et,
R₂ = F, Cl, OH or C₁-C₃-alkoxy,
R₃ = H, Me,
R₄ = C₁-C₄-alkyl which is substituted by amino, hydroxyl, C₁-C₄-alkoxy or C₁-C₄-alkylthio; Ph, Bz, and other alpha amino acid residues (such as CH₂CO₂H, CH₂CH₂CO₂H and CH₂-Hetaryl groups).

In another preferred group of compounds of formulae **IA** and **IIA:**
R₁, R₂ and R₃ have the same meaning as mentioned in formulae **I** and **II** in the preceding paragraph,
R₄ = R_{5 =} Me or Ph.

In another preferred group of compounds of formulae **IB** and **IIB:**
R₁, R₂ and R₃ have the same meaning as mentioned in formulae **I** and **II** in the antepreceding paragraph,
R₄ and R₅ together = [CH₂]₂; [CH₂]₃ or [CH₂]₄.

In another preferred group of compounds of formulae **I** and **II:**
R₁ = *c*-C₃H₅,
R₂ = F, Cl, OH or OMe,
R₃ = H, Me,
R₄= C₁-C₄-alkyl which is substituted by hydroxyl, C₁-C₄-alkoxy or C₁-C₄-alkylthio.

In another preferred group of compounds of formulae **IA** and **IIA:**
R₁, R₂ and R₃ have the same meaning as mentioned in formulae **I** and **II** in the preceding paragraph,
R₄ = R₅: Me.

In another preferred group of compounds of formulae **IB** and **IIB:**
R₁, R₂ and R₃ have the same meaning as mentioned in formulae **I** and **II** in the antepreceding paragraph,
R₄ and R_{5 =} [CH₂]₂, [CH₂]₃ or [CH₂]₄.

Compounds of the formulae **I, IA** or **IB** and compounds of the formulae **II, IIA** or **IIB** as described above, are of very particular importance when the said substituents have the following meanings:
R₁ is cyclopropyl or ethyl,
R₂ is fluorine, chlorine, methoxy or ethoxy,
R₃ is hydrogen or methyl,
R₄ (of formulae **I** and **II)** is C₁-C₄-alkyl which is substituted by hydroxyl, C₁-C₄-alkoxy or C₁-C₄-alkylthio.

R₄ = R₅ (of formulae **IA** and **IIA)** is methyl or phenyl,

R₄ and R₅ (of formulae **IB** and **IIB)** which together with the interjacent carbon atom constitute spiro-cyclopropyl, -cyclobutyl or -cyclopentyl.

The compound (*S*)-10-Cyclopropyl-3-ethyl-5-fluoro-2,7-dioxo-1,2,3,4,7,10-hexahydro-4-methyl-pyrido[2,3-*f*]quinoxaline-8-carboxylic acid (**III-2a** / Example 1) is of very particular importance. The compounds of formulae **I** and **II** possess a stereocenter which is the C-3 carbon atom having the so-called (*S*)-configuration.

The invention also provides a process for the preparation of a compound of the general formula A or B as defined above which comprises reacting a compound of the general formula 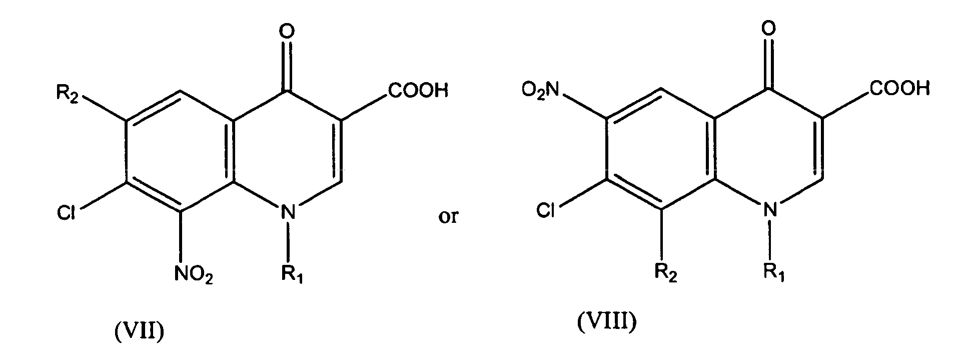 in which R₁ and R₂ are as defined above, with a compound of the general formula 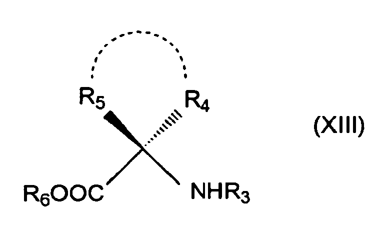 in which R₃, R₄, R₅ and R₆ are as defined above, to form a compound of general formula A or B in which R₆ is a hydrogen atom, R₇ represents a group -COOR₆ and R₈ represents a nitro group;
if desired, subjecting the resultant compound to reductive cyclization to form a compound of general formula A or B in which R₇ and R₈ together represent a group -C(O)-NR₉-, where R₉ is as defined above;
if desired, reacting the resultant compound with an alkylating agent to form a compound of general formula A or B in which R₆ is an alkyl group;
and/or if desired, converting the resultant compound to a physiologically tolerated salt of ester thereof.

Preferably, the reaction between a compound of formula VII or VIII and a compound of formula XIII is carried out in the presence of a solvent, preferably water and/or an alcohol such as ethanol. It is also preferred that the reaction is carried out in the presence of a base such as potassium hydrogen carbonate. Ideally, the reaction is carried out in aqueous ethanolic potassium hydrogen carbonate solution. The reaction is preferably carried out at a temperature from 50-100°C, more preferably 60-90°C, for a period of ½-6 days, preferably 1-5 days.

The optional reductive cyclization step is preferably carried out in the presence of a solvent, such as water. It is also preferred that the reaction is carried out in the presence of a base such as potassium carbonate solution. Preferably, the reductive aspect of the process is accomplished by the use of a reducing agent, such as sodium dithionite. It is preferred that the reaction is carried out at room temperature, that is, 15 to 40°C, preferably 20 to 30°C, more preferably around 25°C.

The optional alkylating step is preferably carried out using an alkylating agent, such as diazomethane etherate. Preferably, the reaction is carried out at a temperature of 0 to 20°C, more preferably 0 to 10°C, for 10 to 90, preferably 20 to 60 minutes.

The optional conversion to a corresponding physiologically tolerated salt or ester can be carried out by known methods involving the reaction of the compound with a suitable acid or base. Suitable acids include organic acids and mineral acids. Suitable bases include inorganic and organic bases.

The present invention furthermore relates to compounds of formulae **I, IA** and **IB,** and compounds of formulae **II, IIA** and **IIB** as potential antibacterial compounds which are preferably used for treating Gram positive and Gram negative bacterial strains, exemplified by S. *aureus* and E. *coli.*

Pure compounds of formulae **III, IIIA** and **IIIB** can be prepared by reductive cyclization of the respective quinolone precursors of the formulae **V, VA** and **VB,** wherein R₁, R₂, R₄ and R₅ are as defined above (Schemes 1-3).

Pure compounds of formulae **IV, IVA** and **IVB** can be prepared by reductive cyclization of the respective quinolone precursors of the formulae **VI, VIA** and **VIB,** wherein R₁, R₂, R₄ and R₅ are as defined above (Schemes 4-6).

Compounds of the formulae **V, VA, VB** and **VI, VIA, VIB** can be prepared by analogy to known methods or by modifications thereof (see, for example, R. Chicharro, S. de Casto, R. L. Reino and J. V. Aran, Eur. J. Org. Chem., 2003, 2314-2326).

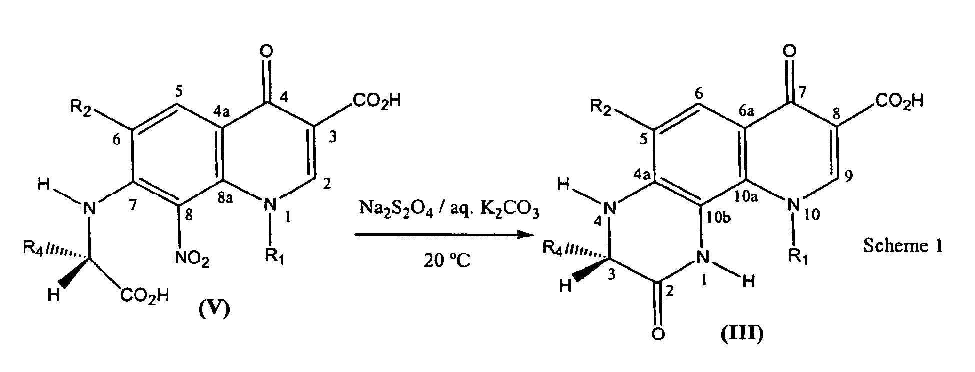

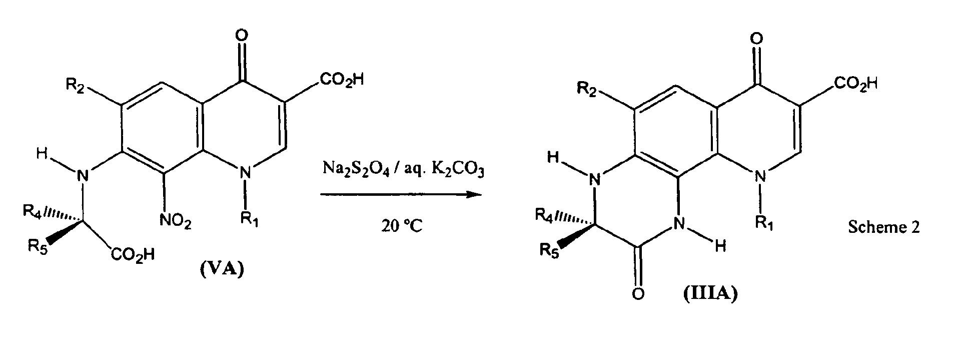

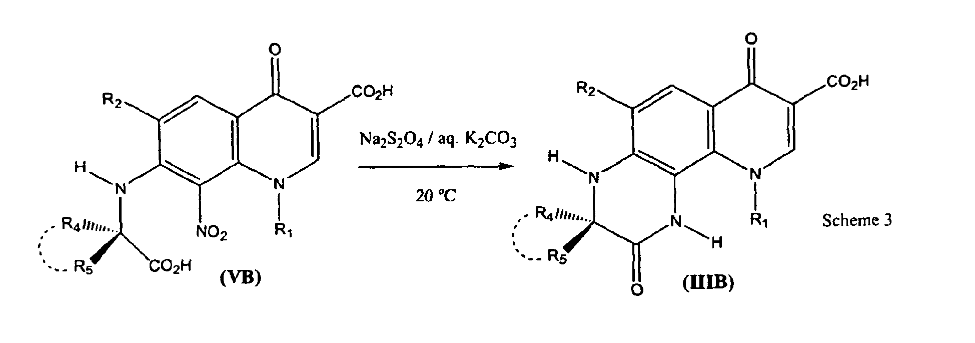

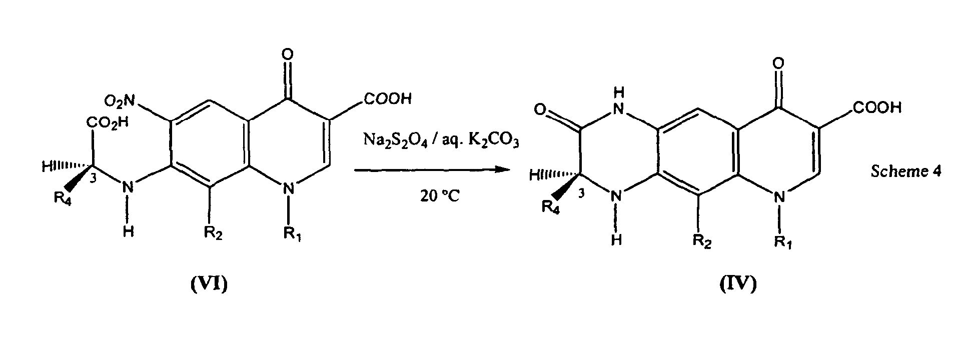

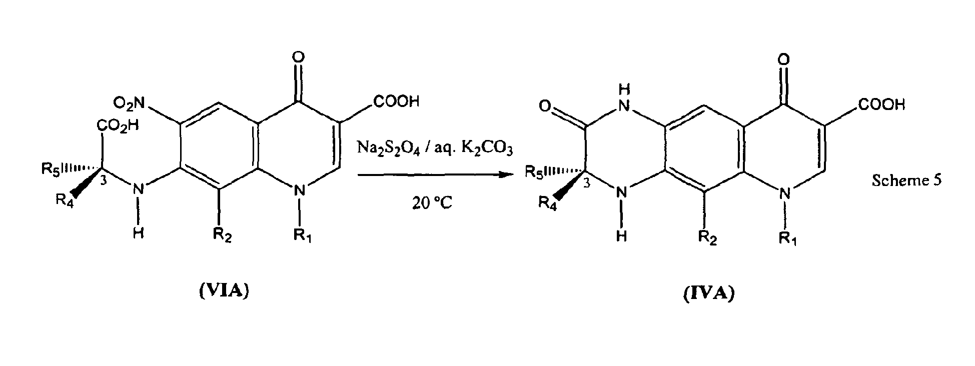

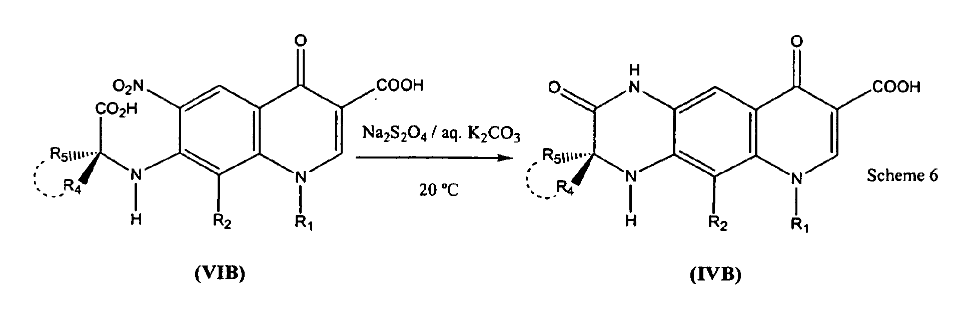

The (*S*)-enantiomers of the chiral pyridoquinoxalinones of the formulae **III** and **IV,** possessing high optical purity (≥98% ee), can be obtained utilizing the particular (*S*)-enantiomer of formulae **V** and **VI,** where R₁, R₂, and R₄ have the same meaning as in the desired products **I** and **II.**

Optically pure enantiomers are understood to mean those compounds in which the enantiomer ratio is at least 95 : 5, preferably 97 : 3 (that is % ee ≥ 98.5).

Compounds of the formulae **V, VA, VB** (R₁ = cyclopropyl, R₂ = F) can be prepared *via* interaction of 7-chloro-1-cyclopropyl-6-fluoro-8-nitro-4-oxo-1,4-dihydroquinoline-3-carboxylic acid (**VII** / R₁ = cyclopropyl, R₂ = F) with a particular primary α-amino acid (Schemes 7-9). The methodology adopted herein is analogous to known methods (or modifications thereof) reported for the preparation of N-(2,4-dinitrophenyl)-α-amino acids (see, for example, R. Chicharro, S. de Casto, R. L. Reino and J. V. Aran, Eur. J. Org. Chem., 2003, 2314-2326, and references cited therein). The compounds (**VII** / R₁ = cyclopropyl, R₂ = F), used as starting materials, are known and can be prepared according to published procedures [ prep. of cipro Grohee].

Compounds of the formulae **VI, VIA, VIB** (R₁ = cyclopropyl, R₂ = F) can be prepared *via* interaction of 7-chloro-1-cyclopropyl-8-fluoro-6-nitro-4-oxo-1,4-dihydroquinoline-3-carboxylic acid **(VIII** / R₁ = cyclopropyl, R₂ = F) with a particular primary α-amino acid (Schemes 10-12). Again, the methodology adopted herein is analogous to known methods (or modifications thereof) reported for the preparation of related systems (see, for example, R. Chicharro, S. de Casto, R. L. Reino and J. V. Aran, Eur. J. Org. Chem., 2003, 2314-2326, and references cited therein). The compounds (**VIII** / R₁ = cyclopropyl, R₂ = F), used as starting materials, are known and can be prepared according to published procedures [ prep. of isocipro/ jala]. 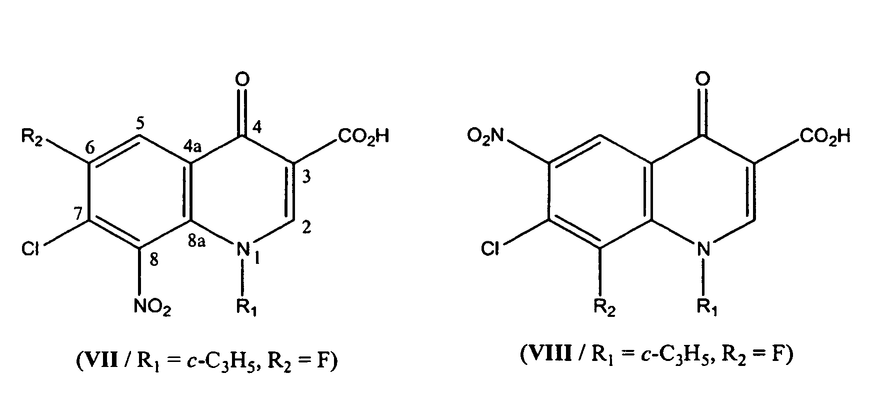 The primary α-amino acids (formula XIII) are well known compounds which can be prepared by known processes or processes analogous to known processes.

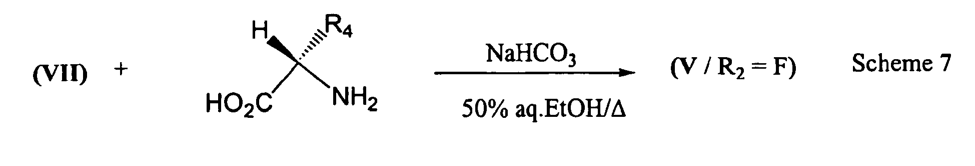

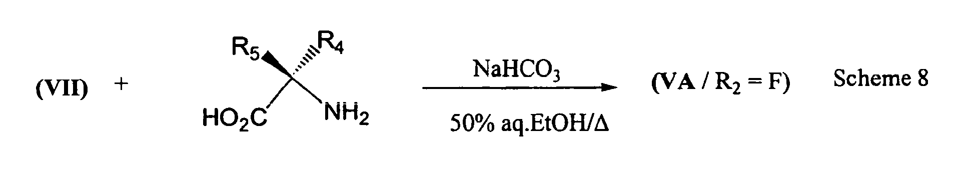

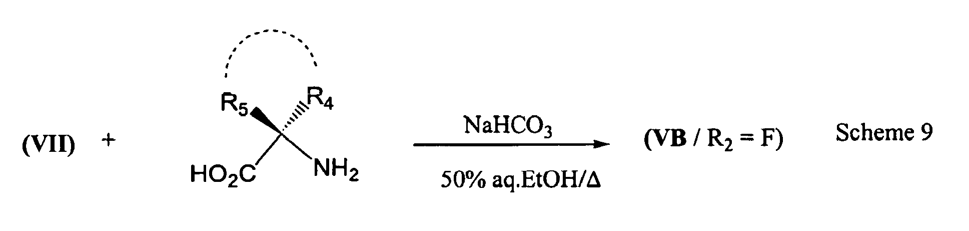

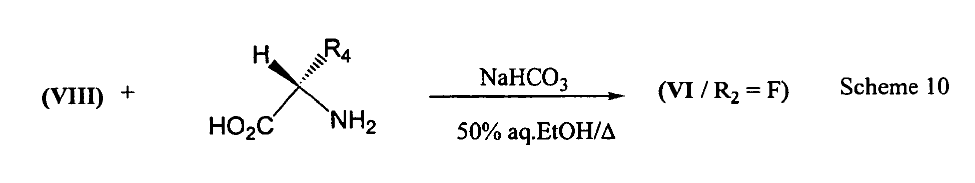

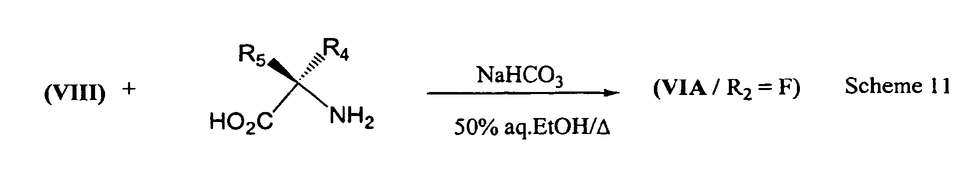

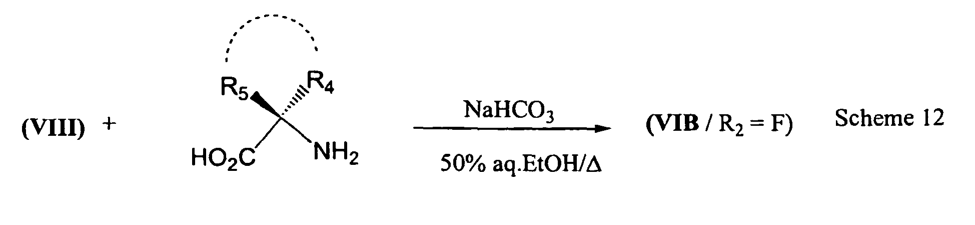

The present invention furthermore relates to compounds of formulae **IX,** and **X** as potential antibacterial compounds which are preferably used for treating Gram positive and Gram negative bacterial strains, exemplified by S. *aureus* and E. *coli.* 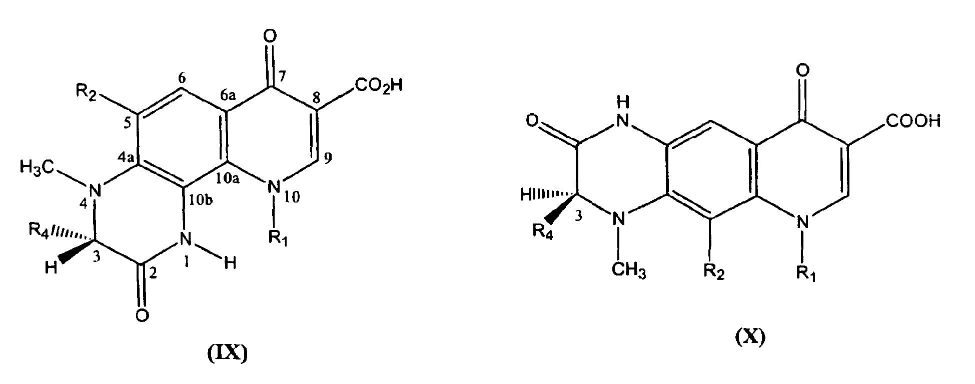 In a preferred group of compounds of the formulae **IX** and **X**:
R₁ = *c*-C₃H₅ or Et,
R₂ = F, Cl, OH or C₁-C₃-alkoxy,
R₄ = C₁-C₄-alkyl which is substituted by amino, hydroxyl, C₁-c₄-alkoxy or C₁-C₄-alkylthio; Ph, Bz, and other alpha amino acid residues (such as CH₂CO₂H, CH₂CH₂CO₂H and CH₂-Hetaryl groups).

In another preferred group of compounds of the formulae **IX** and **X**:
R₁ = *c*-C₃H₅,
R₂=F,
R₄ = hydrogen (N-sarcosine), methyl (N-methylalanine), isopropyl (N-methylvaline) and benzyl (N-methylphenylalanine).

Pure compounds of formulae **IX** and **X** are prepared by reductive cyclization of the respective quinolone precursors of the formulae **XI** and **XII** wherein R₁, R₂ and R₄ are as defined above (Schemes 13-14).

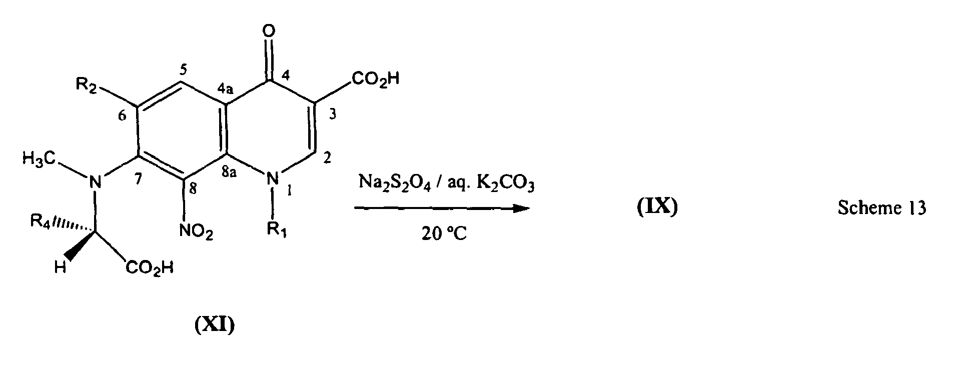

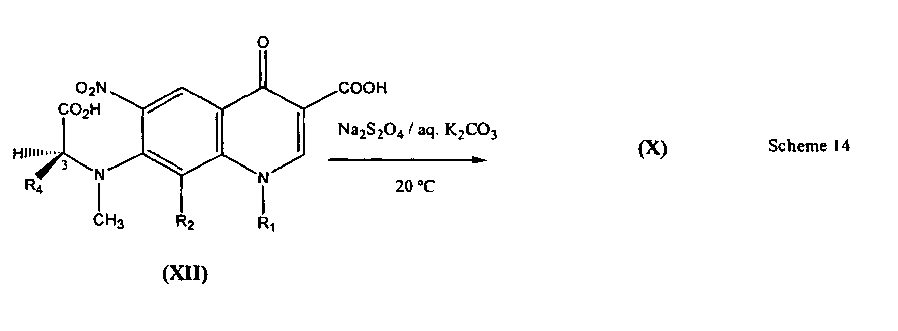

The compounds of the formulae **XI** and **XII** can be prepared by analogy to known methods or by modifications thereof (see, for example, R. Chicharro, S. de Casto, R. L. Reino and J. V. Aran, Eur. J. Org. Chem., 2003, 2314-2326).

The (*S*)-enantiomers of the chiral pyridoquinoxalinones of the formulae **IX** and **X**, possessing high optical purity (≥98% ee), can be obtained utilizing the particular (*S*)-enantiomer of the formulae **XI** and **XII,** where R₁, R₂, and R₄ have the same meaning as in the desired products **IX** and **X.**

Optically pure enantiomers are understood to mean those compounds in which the enantiomer ratio is at least 95 : 5, preferably 97 : 3 (that is % ee ≥ 98.5).

Compounds of the formula **XI** (R₁ = cyclopropyl, R₂ = F) can be prepared *via* interaction of 7-chloro-1-cyclopropyl-6-fluoro-8-nitro-4-oxo-1,4-dihydroquinoline-3-carboxylic acid **(VII** / R₁ = cycloproyl, R₂ = F) with a particular N-methyl α-amino acid (Scheme 15). The methodology adopted herein is analogous to known methods (or modifications thereof) reported for the preparation of N-(2,4-dinitrophenyl)-α-amino acids (see, for example, R. Chicharro, S. de Casto, R. L. Reino and J. V. Aran, Eur. J. Org. Chem., 2003, 2314-2326, and references cited therein). The compounds (**VII** / R₁ = cyclopropyl, R₂ = F), used as starting materials, are known and can be prepared according to published procedures [prep. of cipro Grohee].

Compounds of the formula **XII** (R₁ = cyclopropyl, R₂ = F) can be prepared *via* interaction of 7-chloro-1-cyclopropyl-8-fluoro-6-nitro-4-oxo-1,4-dihydroquinoline-3-carboxylic acid (**VIII** / R₁ = cyclopropyl, R₂ = F) with a particular N-methyl α-amino acid (Scheme 16). Again, the methodology adopted herein is analogous to known methods (or modifications thereof) reported for the preparation of related systems (see, for example, R. Chicharro, S. de Casto, R. L. Reino and J. V. Aran, Eur. J. Org. Chem., 2003, 2314-2326, and references cited therein). The compounds (**VIII** / R₁ = cyclopropyl, R₂ = F), used as starting materials, are known and can be prepared according to published procedures [ prep. of isocipro/ jalal]. 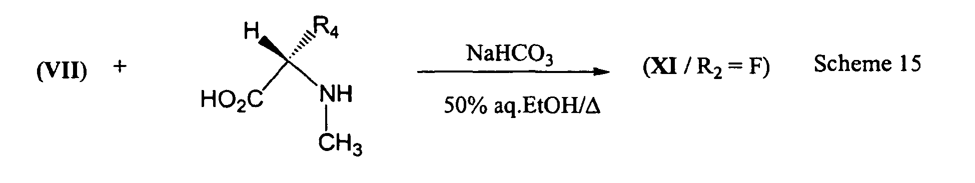 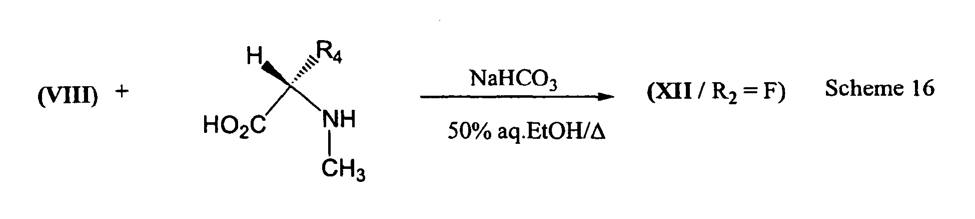 The N-methyl α-amino acids are well known compounds which can be prepared by known processes or processes analogous to known processes.

The compounds of general formulae A and B which encompass the compounds of formulae I, IA, IB, II, IIA, IIB, III, IIIA, IIIB, IV, IVA, IVB, V, VA, VB, VI, VIA, VIB, IX, X, XI and XII, have been found to be effective as antibacterial agents. They are therefore useful in the treatment and/or prophylaxis of diseases caused by bacteria or other microbes. They are particularly useful in combating Gram positive and Gram negative strains, such as *S.aureus* and *E.coli.* Moreover, since the compounds of general formula V, VA, VB, VI, VIA, VIB, XI and XII can be readily converted into compounds of general formula III, IIIA, IIIB, IV, IVA, IVB, IX and X respectively, the compounds of general formula V, VA, VB, VI, VIA and VIB function as prodrugs.

In view of the above, the present invention also provides a compound of the general formula A or B or a physiologically tolerated salt or ester thereof as defined above for use in medicine, particularly as an antibacterial agent.

The invention also includes the use of a compound of the general formula A or B or a physiologically tolerated salt or ester thereof as defined above for the manufacture of a medicament for use as an antibacterial agent, especially against Gram positive and Gram negative bacteria, such as *S.aureus* and *E.coli.*

In another aspect, the compounds of the invention can be used in a method for preventing a a disease caused by a bacterium or a microbe which comprises administering to a patient a therapeutically or prophylactically effective amount of a compound of the general formula A or B or a physiologically tolerated salt or ester thereof as defined above.

The invention also provides a pharmaceutical composition which comprises a carrier and, as active ingredient, a compound of the general formula A or B or a physiologically tolerated salt or ester thereof as defined above. A process for the preparation of a pharmaceutical composition as defined above is also provided which comprises bringing a compound of the general formula A or B or a physiologically tolerated salt or ester thereof as defined above into association with a carrier.

A pharmaceutically acceptable carrier may be any material with which the active ingredient is formulated to facilitate administration. A carrier may be a solid or a liquid, including a material which is normally gaseous but which has been compressed to form a liquid, and any of the carriers normally used in formulating pharmaceutical compositions may be used. Preferably, compositions according to the invention contain 0.5 to 95% by weight of active ingredient.

The compounds of general formula A and B can be formulated as, for example, tablets, capsules, suppositories or solutions. These formulations can be produced by known methods using conventional solid carriers such as, for example, lactose, starch or talcum or liquid carriers such as, for example, water, fatty oils or liquid paraffins. Other carriers which may be used include materials derived from animal or vegetable proteins, such as the gelatins, dextrins and soy, wheat and psyllium seed proteins; gums such as acacia, guar, agar, and xanthan; polysaccharides; alginates; carboxymethylcelluloses; carrageenans; dextrans; pectins; synthetic polymers such as polyvinylpyrrolidone; polypeptide/protein or polysaccharide complexes such as gelatin-acacia complexes; sugars such as mannitol, dextrose, galactose and trehalose; cyclic sugars such as cyclodextrin; inorganic salts such as sodium phosphate, sodium chloride and aluminium silicates; and amino acids having from 2 to 12 carbon atoms such as a glycine, L-alanine, L-aspartic acid, L-glutamic acid, L-hydroxyproline, L-isoleucine, L-leucine and L-phenylalanine.

Auxiliary components such as tablet disintegrants, solubilisers, preservatives, antioxidants, surfactants, viscosity enhancers, colouring agents, flavouring agents, pH modifiers, sweeteners or taste-masking agents may also be incorporated into the composition. Suitable colouring agents include red, black and yellow iron oxides and FD & C dyes such as FD & C blue No. 2 and FD & C red No. 40 available from Ellis & Everard. Suitable flavouring agents include mint, raspberry, liquorice, orange, lemon, grapefruit, caramel, vanilla, cherry and grape flavours and combinations of these. Suitable pH modifiers include sodium hydrogencarbonate, citric acid, tartaric acid, phosphoric acid, hydrochloric acid and maleic acid. Suitable sweeteners include aspartame, acesulfame K and thaumatin. Suitable taste-masking agents include sodium hydrogencarbonate, ion-exchange resins, cyclodextrin inclusion compounds, adsorbates or microencapsulated actives.

The invention is further illustrated by the following examples.

### Examples

Numbering of Compounds III and V as Included in the Examples:

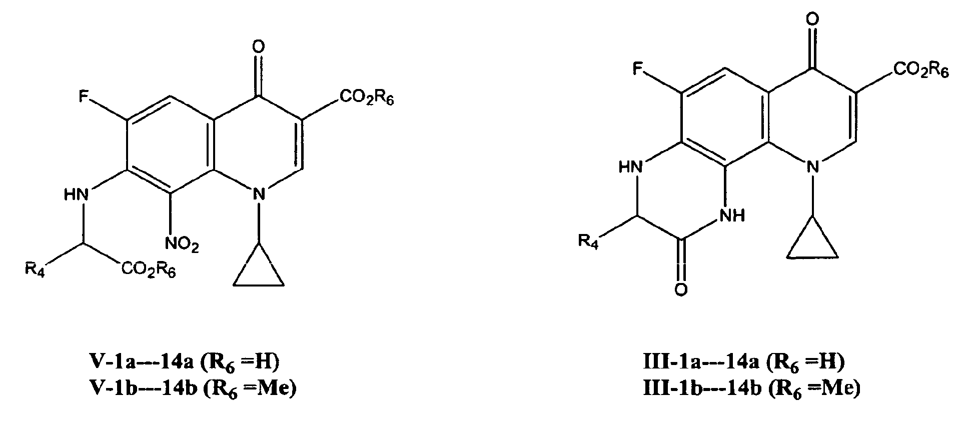

| **Entry** **III/ V** | **α-Amino** **acid (L-)** | **R₄** |
|---|---|---|
| 1 | Gly | H |
| 2 | Ala | Me |
| 3 | Val | (Me)₂CH |
| 4 | Leu | (Me)₂CH₂CH |
| 5 | Ile | Et(Me)CH |
| 6 | ser | CH₂OH |
| 7 | Thr | MeCHOH |
| 8 | Met | MeSCH₂CH₂ |
| 9 | N-Ac Lys | Ac-NH(CH₂)₄ |
| 10 | Asp | CH₂CO₂H |
| 11 | Glu | CH₂CH₂CO₂H |
| 12 | Phe | CH₂Ph |
| 13 | Phenyl glycine* | Ph* |
| 14 | Amino butyric acid* | Et* |

| | | |
|---|---|---|
| * non-proteinic | | |

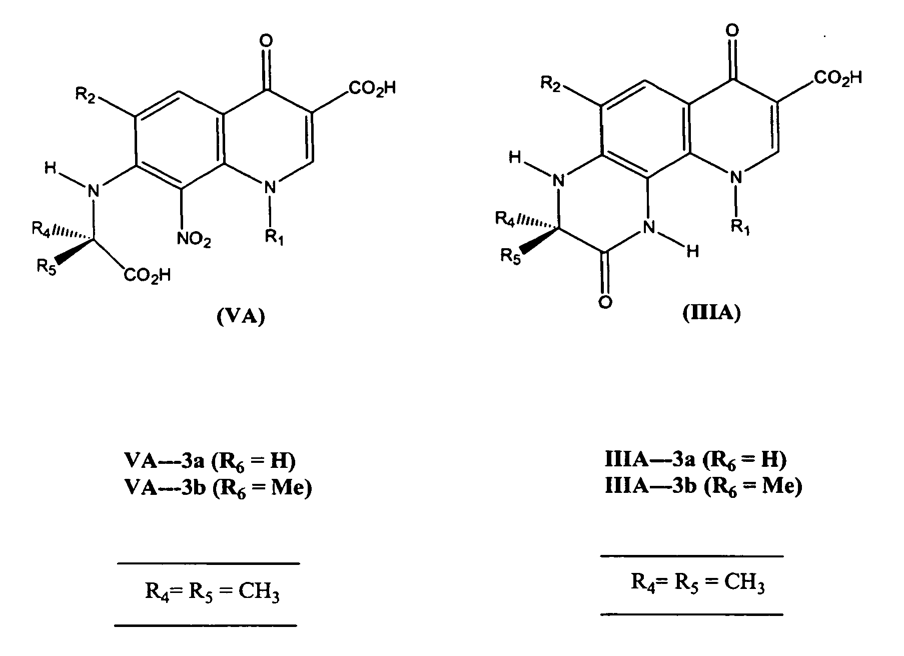

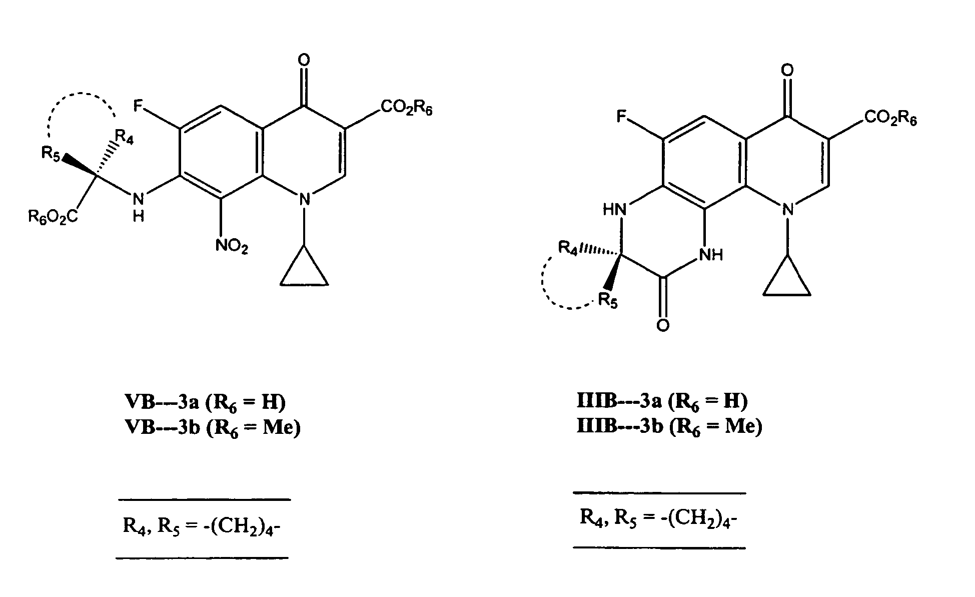

**Numbering of Compounds IV and VI as Included in the Examples:**

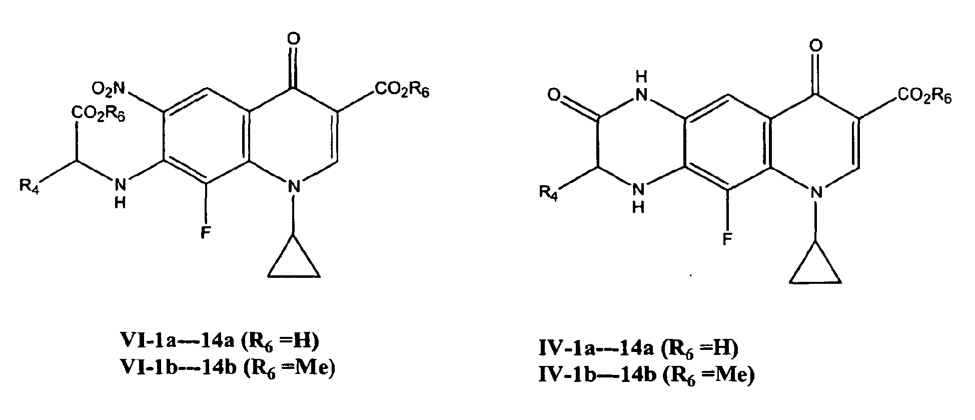

| **Entry** **IV/VI** | **α-Amino** **acid (L-)** | **R₄** |
|---|---|---|
| 1 | Gly | H |
| 2 | Ala | Me |
| 3 | Val | (Me)₂CH |
| 4 | Leu | (Me)2_{C}H2_{C}H |
| 5 | Ile | Et(Me)CH |
| 6 | ser | CH₂OH |
| 7 | Thr | MeCHOH |
| 8 | Met | MeSCH₂CH₂ |
| 9 | N-Ac Lys | Ac-NH(CH₂)₄ |
| 10 | Asp | CH₂CO₂H |
| 11 | Glu | CH₂CH₂CO₂H |
| 12 | Phe | CH₂Ph |
| 13 | Phenyl glycine* | Ph* |
| 14 | Amino butyric acid* | Et* |

| | | |
|---|---|---|
| * non-proteinic | | |

**Numbering of Compounds IX and XI as Included in the Examples:**

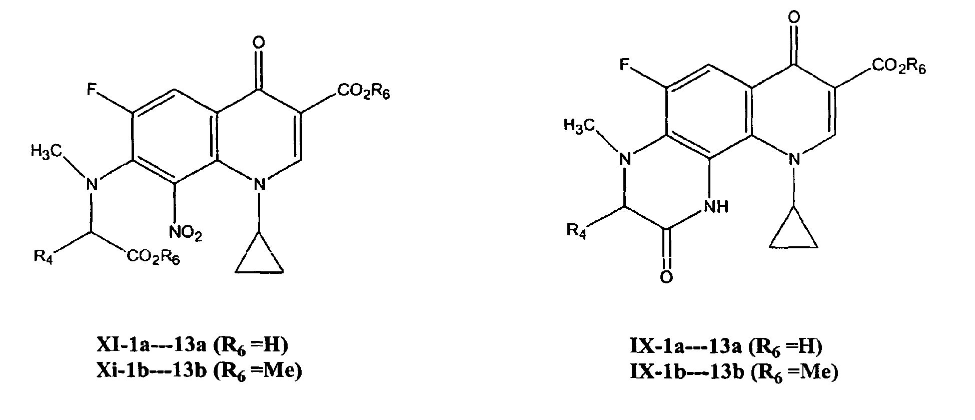

| **Entry** **IX / XI** | **N-Me α-Amino** **acid (L-)** | **R₄** |
|---|---|---|
| 1 | N-Me Gly (Sarcosine) | H |
| 2 | N-Me Ala | Me |
| 3 | N-Me Val | (Me)₂CH |
| 4 | N-Me Leu | (Me)₂CH₂CH |
| 5 | N-Me Ile | Et(Me)CH |
| 6 | N-Me Ser | CH₂OH |
| 7 | N-Me Thr | MeCHOH |
| 8 | N-Me Met | MeSCH₂CH₂ |
| 9 | N-Me Asp | CH₂CO₂H |
| 10 | N-Me Glu | CH₂CH₂CO₂H |
| 11 | N-Me Phe | CH₂Ph |
| 12 | N-Me Phenyl glycine* | Ph* |
| 13 | N-Me Amino butyric acid* | Et* |

| | | |
|---|---|---|
| * non-proteinic | | |

**Numbering of Compounds X and XII as Included in the Examples:**

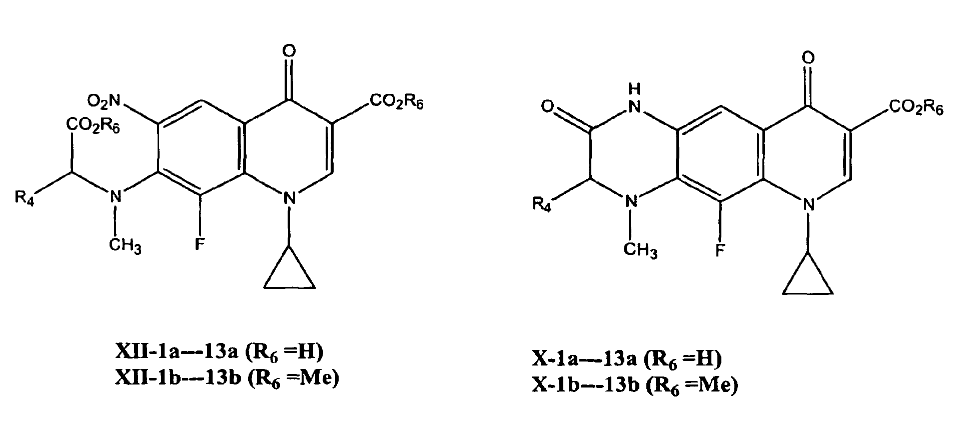

| **Entry** **X / XII** | **N-Me α-Amino** **acid (L-)** | **R₄** |
|---|---|---|
| **1** | N-Me Gly (Sarcosine) | H |
| **2** | N-Me Ala | Me |
| **3** | N-Me Val | (Me)₂CH |
| **4** | N-Me Leu | (Me)₂CH₂CH |
| **5** | N-Me Ile | Et(Me)CH |
| **6** | N-Me Ser | CH₂OH |
| **7** | N-Me Thr | MeCHOH |
| **8** | N-Me Met | MeSCH₂CH₂ |
| **9** | N-Me Asp | CH₂CO₂H |
| **10** | N-Me Glu | CH₂CH₂CO₂H |
| **11** | N-Me Phe | CH₂Ph |
| **12** | N-Me Phenyl glycine* | Ph* |
| **13** | N-Me Amino butyric acid* | Et* |

| | | |
|---|---|---|
| * non-proteinic | | |

### Experimental part:

The primary α-amino acids, employed in this study, are biochemical grades (Aldrich) and were used as received. 2,4-Dichloro-5-fluoro-3-nitrobenzoic acid was purchased from Acros. Tris [3-(heptafluoropropylhydroxymethylene)-(*d*)-camphorato]europium (III), [(+)- Eu (hfc)₃] was purchased from Aldrich. Melting points (uncorrected) were determined on a Gallenkamp electrothermal melting temperature apparatus. Optical rotations were measured on a Perkin-Elmer 141 polarimeter with a micro cell (100mm path length, 1 mL), at 20°C as solutions in DMSO (*c∼*1) or 5% aq. NaHCO₃ (*c*∼1) for the acids, and in CHCl₃ (*c*∼1) for the esters.

¹H- and ¹³C-NMR spectra were recorded on a Bruker DPX-300 instrument with Me₃Si as internal reference. EIMS spectra were obtained using a Finnigan MAT TSQ-70 spectrometer at 70 eV; ion source temperature = 200 °C. High resolution MS-ESI data were obtained with Bruker Bio TOF III. IR spectra were recorded as KBr discs on a Nicolet Impact-400 FT-IR spectrophotometer. Microanalyses were preformed at the Microanalytical Laboratory - Medicinal Chemistry devision, Faculty of Pharmacy, Jordan University, Amman.

### Example 1

### 7-[(Carboxymethyl)amino]-1-cyclopropyl-6-fluoro-8-nitro-4-oxo-1,4-dihydroquinoline-3-carboxylic acid (V-1a)

A well-stirred mixture of glycine (0.68 g, 9 mmol), 7-chloro-1-cyclopropyl-6-fluoro-8-nitro-4-oxo-1,4-dihydroquinoline-3-carboxylic acid **VII** (1.0 g, 3 mmol) and sodium hydrogencarbonate (1.5 g, 18 mmol) in aqueous ethanol (140 ml, 1:1 v/v) was heated at 70-80 °C. The reaction mixture slowly developed a light yellow color that changed into bright yellow, then into clear orange solution. The progress of the reaction was monitored by TLC, and was completed within 40-48 h. The resultant orange solution was first extracted with dichloromethane (50 ml), then the aqueous layer was separated, acidified with 3N HCl to pH 6.5, and re-extracted with dichloromethane (50 ml). The aqueous layer was separated and acidified again with 3N HCl to pH 2-3, whereby the title compound was precipitated as fluorescent yellowish solid which was filtered, washed with cold water (3 x 20 ml) and recrystallized from ethanol.

Yield 0.90 g (82 %), mp 236- 238 °C (decomposition). *Anal*.Calcd for C₁₅H₁₂FN₃O₇ (365.27): C, 49.32; H,3.31; N, 11.50; found C,49.02; H,3.22; N,11.41;¹H NMR (300 MHz, DMSO-d₆): δ 0.95(m, 4H, H₂-2'/ H₂-3'), 3.67(br m, 1H, H-1'), 4.25(br dd, *J=* 5.4 Hz, 6.2 Hz, 2H, α-C*H*₂-NH), 7.57(br s, 1H, N-H), 7.95(d, ³*J*_{H-F}=13.9 Hz, 1H, H-5), 8.72(s, 1H, H-2), 13.43(br s, 1H, CH₂-CO₂*H*), 14.63(br s, 1H, C(3)-CO₂H); ¹³C NMR (75 MHz, DMSO-d₆): δ 10.2(C-2'/ C-3'), 40.6(C-1'), 47.1(d, *J*_{C-F} = 12.5 Hz, α-CH_{Z}NH), 109.5(C-3), 114.4(d, ²*J*_{C-F}= 23.0 Hz, C-5), 116.6(d, ³*J*_{C-F} = 7.1 Hz, C-4a), 128.0(d, ³*J*_{C-f}= 5.6 Hz, C-8), 135.5(C-8a), 138.8(d, ²*J*_{C-f}= 14.3 Hz, C-7), 150.4(d, ¹*J*_{C}-_{F} = 247 Hz, C-6), 152.0(C-2), 165.4(C(3)-*C*O₂H), 171.7(d, *J*_{C-F}= 2.5 Hz, CH₂-*C*O₂H), 175.5(d, ⁴*J*_{C-F} = 2.6 Hz, C-4).

### Example 2

### Methyl 1-Cyclopropyl-6-fluoro-7-(methoxycarbonylmethyl-amino)-8-nitro-4-oxo-1,4-dihydroquinoline-3-carboxylate (V-1b)

This compound is prepared via interaction of **V-1a** with diazomethane etherate.

Yield (87 %); MS (TOF ES ⁺): *m*/*z* 394 (M+ H)⁺, HRMS calcd for C₁₇H₁₇FN₃O₇ 394.1050, found 394.1045; *m*/*z* 416 (M+ Na)⁺, HRMS calcd for C₁₇H₁₆FN₃O₇Na 416.0870, found 416.0864; ¹H NMR (300 MHz, CDCl₃): δ 0.91(m, 4H, H₂-2'/H₂-3'), 3.51(m,1H, H-1'), 3.63(s, 3H, CH₂-CO₂C*H*₃), 3.72(s, 3H, C(3)-CO₂C*H*₃), 4.28(dd, *J*_{H-F} = 5.8, 5.1 Hz, 2H, α-C*H*₂-NH), 7.19(dd, *J* = 5.8, 5.6 Hz, 1H, N-H), 7.82(d, ³*J*_{H-F} = 14.3 Hz, 1H, H-5), 8.49(s,1H, H-2); ¹³C NMR (75 MHz, CDCl₃): δ 10.2(C-2'/ C-3'), 39.3(C-1'), 47.0(d, *J*_{C-F}= 11.9 Hz, α-CH₂NH), 52.0(CH₂-CO₂*C*H₃), 52.6(C(3)-CO₂*C*H₃), 111.9(C-3), 115.3(d, ²*J*_{C-F} = 22.6 Hz, C-5), 119.9(d, ³*J*_{C-F} = 5.9 Hz, C-4a), 129.0(d, ³*J*_{C-F}= 5 Hz, C-8), 134.4(d, ⁴*J*_{C-F} = 1.8 Hz, C-8a), 137.0(d, ²*J*_{C-F} = 14.1 Hz, C-7), 149.7(d, *J*_{C-F} = 244 Hz, C-6), 151.7(C-2), 164.6(C(3)-*C*O₂Me), 170.4(d, ⁴*J*_{C-F} = 2 Hz, C-4), 171.2(d, *J*_{C-F} = 2.3 Hz, CH₂-CO₂Me).

### Example 3

### (S)-7-[(1-Carboxyethyl)amino]-1-cyclopropyl-6-fluoro-8-nitro-4-oxo-1,4-dihydroquinoline-3-carboxylic acid [(S) -V-2a]

A stirred mixture of (*S*)- α-alanine [(*S*)- / 0.8 g, 9 mmol], **VII** (1.0 g, 3 mmol) and sodium hydrogen carbonate (1.5 g, 18 mmol) in 50 % aqueous ethanol (140 ml) was heated at 75-80 °C for 24-30 h under reflux conditions. Work-up of the reaction mixture as described for **V1a** above, produced the title compound as fluorescent yellow solid which was recrystallized from ethanol.

Yield 0.86 g (76 %), mp 219- 221 °C (decomposition); MS(EI): *m*/*z* (% rel.int.): 379 (6, M⁺), 359(2), 335(20), 301(9), 289(24), 257(50), 244(58), 217(30), 188(21), 174(13), 147(11), 107(9), 85(61), 83(100); HRMS: Calcd for C₁₆H₁₄FN₃O₇ 379.08153, found 379.08219; ¹H NMR (300 MHz, DMSO-d₆): δ 0.94(m, 4H, H₂-2'/ H₂-3'), 1.46(d, *J* =6.8 Hz, 3H, CH₃), 3.67(m, 1H, H-1'), 4.62(dq, *J* = 6.8 Hz, 7.1 Hz, 1H, α-C*H*Me), 7.36(d, *J =* 7.1 Hz, 1H, N-H), 8.03(d, ³*J*_{H-F} = 13.7 Hz, 1H, H-5), 8.75(s, 1H, H-2), 13.30(br s, 1H, CH- CO₂*H*), 14.59(br s, 1H, C(3)-CO₂H). ¹³C NMR (75 MHz, DMSO-d₆): δ 10.1(C-2'/ C-3'), 19.8(d, *J*_{C-F} = 2.9 Hz, CH₃), 40.7(C-1'), 53.8(d, *J*_{C-F} = 12 Hz, α-CHMe), 109.7 (C-3), 114.9(d, ²*J*_{C-F} = 22.9 Hz, C-5), 117.3(d, ³*J*_{C-F} = 7.1 Hz, C-4a), 128.7(d, ³*J*_{C-F} = 5 Hz, C-8), 135.7 (C-8a), 137.9(d, ²*J*_{C-F} = 14.7 Hz, C-7), 150.2(d, ¹*J*_{C-F} = 248 Hz, C-6), 152.1(C-2), 165.3(C(3)-*C*O₂H), 174.2 (CH-CO₂H), 175.5(d, ⁴*J*_{C-F} = 2.2 Hz, C-4).

### Example 4

### (S) -7-(1-Carboxy-2-methylpropylamino)-1-cyclopropyl-6-fluoro-8-nitro-4-oxo-1,4-dihydroquinoline-3-carboxylic acid [(S)-V-3a]

A solution of L-valine (1.0 g, 9 mmol) and sodium hydrogen carbonate (NaHCO₃) (1.5 g, 18 mmol) in aqueous ethanol (140 ml, 1:1 v/v) was added to **VII** (1.0 g, 3 mmol) of. The resulting stirred mixture was heated at 60-65 °C. The mixture slowly developed a light yellow color that changed into bright yellow, then into clear orange solution. The progress of the reaction was monitored by TLC, and was completed within 100-120 h. The orange solution was extracted with chloroform (CHCl₃, 2 x 50 ml). The aqueous layer was cooled, adjusted with 3.5N HCl addition to get a solution of pH 6-7 and another extraction with CHCl₃ (50 ml) was carried out. Further acidification of the leftover of the aqueous layer was performed with 3.5N HCl to pH = 1-2, whereby the title compound was precipitated as yellowish solid which was filtered, washed with cold water (2 x 10 ml) and dried. Re-crystallization of the formed product was carried out from a mixture of chloroform and ethanol (1:1).

Yield 1.1 g (90 %), mp 217- 222 °C (decomposition); *Anal.*Calcd for C₁₈H₁₈FN₃O₇ (407.35): C, 53.07; H, 4.45; N, 10.32; found: C, 52.83; H, 4.69; N, 10.68; IR (KBr): *v* 3308, 3070, 2971, 1733, 1700, 1629, 1549, 1518, 1469, 1321, 1257, 1216, 1144, 1033 cm⁻¹; MS(FAB): *m*/*z* ( % rel. int.): 408[100, (M+H)⁺ / calcd. for C₁₈H₁₈FN₃O₇ 407(M)]; ¹H NMR (300 MHz, DMSO-d₆): δ 0.89, 0.95(2d, *J =* 6.8 Hz, 6.0 H, (C*H*₃)₂-CH), 0.92(m, 4H, H₂-2'/H₂-3'), 2.21(m, 1H, CHMe₂), 3.68(m, 1H, H-1'), 4.50(br d, *J* = 7.2 Hz, CH-CO₂H), 7.21(d, *J =* 8.0 Hz, 1H, *N*-H), 8.07(d, ³*J*_{H-F} = 13.5 Hz, 1H, H-5), 8.77(s, 1H, H-2), 13.39(br s, 1H, CH-CO₂*H*), 14.55(br s, 1H, C₃-CO₂H); ¹³C NMR (75 MHz, DMSO-d₆): δ 10.1(C-2'/C-3'),18.0, 18.5((CH₃)₂), 31.6(*C*HMe₂), 40.9(C-1'), 63.2(d, *J*_{C-F} = 11.3 Hz, CH-*N*H), 109.8(C-3), 115.2(d, ²*J*_{C-F} = 22.7 Hz, C-5), 117.8(d, ³*J*_{C-F} = 7.1 Hz, C-4a), 129.3(d, ³*J*_{C-F} = 5.4 Hz, C-8), 135.8 (C-8a), 138.1(d, ²*J*_{C-F} = 14.9 Hz, C-7), 150.4(d, *J*_{C-F} = 248 Hz, C-6), 152.3(C-2), 165.3(C₃-*C*O₂H), 172.7 (CH-*C*O₂H), 175.6(d, ⁴*J*_{C-F}= 2.5 Hz, C-4).

### Example 5

### (S)-7-(1-Carboxy-3-methyl-butylamino)-1-cyclopropyl-6-fluoro-8-nitro-4-oxo-1,4-dihydroquinoline-3-carboxylic acid [(S) -V-4a]

This compound is prepared from interaction of **VII** and (*S*)-Leucine

Yield 1.03 g (79%), mp 210- 212 °C (decomposition) ; [α]D = +124.1° (DMSO, c∼1); *Anal*.Calcd for C₁₉H₂₀FN₃O₇ (421.38): C, 54.16; H, 4.78; N, 9.97; found C,53.83; H,4.74; N,9.80; IR (KBr): ν 3319, 3064, 2970, 2902, 1736, 1694, 1643, 1532, 1481, 1328, 1209, 1140, 1030 cm⁻¹; MS(FAB): *m*/*z* ( % rel. int.): 422 [100, M+H)⁺/ calcd. for C₁₉H₂₀FN₃O₇ 421 (M)]; ¹H NMR (300 MHz, DMSO-d₆): δ 0.85, 0.90 (2d, *J* = 5.5 Hz, 6H, (C*H*₃)₂-CH), 0.94(m, 4H, H₂-2'/H₂-3'), 0.98(m, 1H, C*H*Me₂), 1.70(m, 2H, β-*C*H₂-CH), 3.67(m, 1H, H_1'), 4.50 (br m, 1H, α-CH-CO₂H), 7.06 (d, *J* = 7.9 Hz, 1H, *N*-H), 8.04 (d, ³*J*_{H-F} = 13.7 Hz, 1H, H-5), 8.75 (s, 1H, H-2 ), 13.28( br s, 1H, CH-CO₂*H*), 14.53 (br s, 1H, C₃-CO₂H ); ¹³C NMR (75 MHz, DMSO-d₆): δ 10.2(C-2'/ C-3'), 22.1, 23.1[(CH₃)₂], 25.0(*C*HMe₂), 40.7(C-1'), 41.7(β-*C*H₂-CH), 57.0(d, *J*_{C-F} = 11.2 Hz, α-CH-*N*H), 109.7(C-3), 115.0(d, ²*J*_{C-F} = 23.0 Hz, C-5), 117.7(d, ³*J*_{C-F} = 7.1 Hz, C-4a), 129.3(d, ³*J*_{C-F}= 5.3 Hz, C-8), 135.5 (C-8a), 138.0(d, ²*J*_{C-F}= 14.8 Hz, C-7), 150.4(d, ¹*J*_{C-F} = 248 Hz, C-6), 152.2(C-2), 165.3(C₃-*C*O₂H), 173.9 (CH-*C*O₂H), 175.6(d, ⁴*J*_{C-F} = 2.6 Hz, C-4).

### Example 6

### (S)-Methyl7-(1-carboxy-3-methyl-butylamino)-1-cyclopropyl-6-fluoro-8-nitro-4-oxo-1,4-dihydroquinoline-3-carboxylate [(S)-V-4b]

This ester is prepared fom interaction of diazomethane etherate with (*S*)-**V-4a.**

Yield (88 %); [α]_{D} = +214.2° (CHCl₃, c∼1); *Anal*.Calcd for C₂₁H₂₄FN₃O₇ (449.43): C, 56.12; H, 5.38; N, 9.35; found C,55.95; H,5.17; N,9.45; MS (EI): *m*/*z* (% rel.int.): 449 (18, M**⁺),** 418(8), 391(100), 372(40), 343(22), 316(31), 302(35), 275(42), 259(32), 203(26), 168(17), 174(18), 147(8); HRMS: calcd for C₂₁ H₂₄ F N₃ O₇ 449.15978 (M⁺), found 449.16004; ¹H NMR (300 MHz, CDCl₃): δ 0.83, 1.06(2m, 4H, H₂-2'/H₂-3'), 0.94, 0,96(2d, *J*= 6.6 Hz, 6.5 Hz, 6H, CH(C*H*₃)₂, 1.64(m, 1H, β-CH_{A}-C*H*Me₂), 1.73(m, 1H, β-C*H*_{B}-CHMe₂),1.80(m, 1H, C*H*Me₂), 3.57(m, 1H, H-1'), 3.67(s, 3H, CH-CO₂C*H*₃), 3.87(s, 3H, C(3)-CO₂C*H*₃), 4.60(m, 1H, α-C*H*-NH), 6.52(d, *J* = 9.7 Hz, 1H, N-H), 8.09(d, ³*J*_{H-F} = 13.5 Hz, 1H, H-5), 8.55(s, 1H, H-2); ¹³C NMR (75 MHz, CDCl₃): δ 10.0(C-2'/ C-3'), 11.8(*C*H₃CH₂), 21.7, 22.8(CH(*C*H₃)₂), 24.9(CHMe₂), 38.9(C-1'), 42.7(β-CH₂-CHNH), 52.3(C(3)-CO₂*C*H₃), 52.7(CH-CO₂*C*H₃), 57.0(d, *J*_{C-F} = 11.9 Hz, α-CH-NH), 112.5(C-3), 116.8(d, ²*J*_{C-F} = 22.7 Hz, C-5), 121.5(d, ³*J*_{C-F} = 6.2 Hz, C-4a), 130.1(d, ³*J*_{C-F} = 5 Hz, C-8), 134.2(C-8a), 136.6(d, ²*J*_{C-F} = 14.8 Hz, C-7), 149.9(d, ¹*J*_{C}-_{F} = 247 Hz, C-6), 151.4(C-2), 165.2(C(3)-*C*O₂Me), 171.2(d, ⁴*J*_{C-F} = 1.9_Hz, C-4), 173.1(CH-*C*O₂Me).

### Example 7

### (1"S, 2"S)-7-(1"-Carboxy-2"-methylbutylamino)-1-cyclopropyl-6-fluoro-8-nitro-4-oxo-1,4-dihydroquinoline-3-carboxylic acid [(S)-V-5a]

This compound is prepared from interaction of **VII** and (*S*)-isoleucine

Yield 0.95 g (74%), mp 233- 235 °C (decomposition) ; [α]_{D} = - 88.1° (5% aq. NaHCO₃, *c*∼1); *Anal*.Calcd for C₁₉H₂₀FN₃O₇ (421.38): C, 54.16; H, 4.78; N, 9.97; found C,54.02; H,4.79; N,10.08; IR (KBr): *v* 3302, 3055, 2962, 2936, 2877, 1736, 1702, 1626, 1523, 1472, 1328, 1200, 1038 cm⁻¹; MS(FAB): *m*/*z* ( % rel. int.): 422 [100, M+H)⁺ / calcd. for C₁₉H₂₀FN₃O₇ 421 (M)]; ¹H NMR (300 MHz, DMSO-d₆): δ 0.84(d, *J* = 6.7 Hz, 3H, C*H*₃-CH), 0.90(t, *J* = 7.4 Hz, 3H, C*H*₃-CH₂), 0.95(m, 4H, H₂-2'/ H₂-3'), 1.26(m, 1H, C*H_{A}*Me), 1.47(m, 1H, C*H_{B}*Me), 1.90(m, 1H, C*H*-Me), 3.67(m, 1H, H.1'), 4.56 ( br m,1H, α-C*H*-CO₂H ), 7.31 ( d, *J* = 7.8 Hz, 1H, *N-*H), 8.03 (d, ³*J*_{H-F} = 13.6 Hz, 1H, H-5 ), 8.75 ( s, 1H, H-2 ), 13.46( br s, 1H, CH-CO₂*H*), 14.51 (br s, 1H, C₃-CO₂*H* ); ¹³C NMR (75 MHz, DMSO-d₆): δ 10.1(C-2'/ C-3'), 12.1(*C*H₃CH₂), 15.2(*C*H₃CH), 25.4(*C*H₂Me), 38.5(*C*HMe), 40.8(C-1'), 62.0(d, *J*_{C-F} = 11.2 Hz, α-CH-*N*H), 109.8(C-3), 115.2(d, ²*J*_{C-F} = 23.0 Hz, C-5), 117.7(d, ³*J*_{C-F} = 6.9 Hz, C-4a), 129.2(d, ³*J*_{C-F} = 5.6 Hz, C-8), 135.8 (C-8a), 137.9(d, ²*J*_{C-F} = 14.9 Hz, C-7), 150.4(d, ¹*J*_{C-F} = 248 Hz, C-6), 152.2(C-2), 165.3(C₃-*C*O₂H), 172.6 (CH-*C*O₂H), 175.5(d, ⁴*J*_{C-F} = 2.4 Hz, C-4).

### Example 8

### (1"S, 2"S)- Methyl 7-(1"-carboxy-2"-methylbutylamino)-1-cyclopropyl-6-fluoro-8-nitro-4-oxo-1,4-dihydroquinoline-3-carboxylate [(S)-V-5b]

This ester is prepared fom interaction of diazomethane etherate with (*S*)-**V-5a**.

Yield (86%), [α]p = +120.2° (CHCl₃, c∼1); *Anal.Calcd* for C₂₁H₂₄FN₃O₇ (449.43): C, 56.12; H, 5.38; N, 9.35; found C,56.50; H,5.37; N,9.45; MS (EI): *m*/*z* (% rel.int.): 449 (23, M⁺), 418(10), 391(100), 390(63), 372(31), 343(23),304(52), 275(48), 272(54), 243(30), 203(38), 188(10), 174(16), 162(11), 133(10), 114(3); HRMS: calcd for C₂₁ H₂₄ F N₃ O₇ 449.15978(M⁺), found 449.16053; ¹H NMR (300 MHz, CDCl₃): δ 0.83, 1.06(2m, 4H, H₂-2'/H₂-3'), 0.94(t, *J* = 7.1 Hz, 3H, C*H*₃CH₂), 0.96(d, *J* = 7 Hz, 3H, C*H*₃CH), 1.28(m, 1H, MeC*H*_{A}), 1.47(m, 1H, MeC*H*_{B}), 1.95(br m, 1H, β-C*H*Me), 3.57(m, 1H, H-1'), 3.69(s, 3H, CH-CO₂C*H*₃), 3.88(s, 3H, C(3)-CO₂C*H*₃), 4.56(m, 1H, α-C*H*-NH), 6.82(d, *J* = 8.5 Hz, 1H, N-H), 8.09(d, ³*J*_{H-F} = 13.6 Hz, 1H, H-5), 8.55(s, 1H, H-2); ¹³C NMR (75 MHz, CDCl₃): δ 10.0(C-2'/C-3'), 11.8(*C*H₃CH₂), 15.5(*C*H₃CH), 25.0(γ-*C*H₂Me), 38.7(β-CHMe), 38.9(C-1'), 52.3(C(3)-CO₂*C*H₃), 52.5(CH-CO₂*C*H₃), 62.7(d, *J*_{C-F} = 11.6 Hz, α-CH-NH), 112.5(C-3), 116.8(d, ²*J*_{C-F} = 22.7 Hz, C-5), 121.3(d, ³*J*_{C-F} = 5.9 Hz, C-4a), 129.9(d, ³*J*_{C-F} = 4.4 Hz, C-8), 134.3(C-8a), 136.6(d, ²*J*_{C-F} = 14.6 Hz, C-7), 149.9(d, ¹*J*_{C-F} = 247 Hz, C-6), 151.4(C-2), 165.3(C(3)-*C*O₂Me), 171.2(d, ⁴*J*_{C-F} = 2 Hz, C-4), 172.0(CH-*C*O₂Me).

### Example 9:

### (S)-7-(1-Carboxy-2-hydroxyethylamino)-1-cyclopropyl-6-fluoro-8-nitro-4-oxo-1,4-dihydroquinoline-3-carboxylic acid [(S) -V-6a]

This compound is prepared from interaction of **VII** and (*S*)-Serine

Yield 0.68 g (56%), mp 170- 171 °C (decomposition. Softens at 130 °C and resolidifies 135 °C) ; [α]_{D} = +39.2° (DMSO, c∼1); *Anal*.Calcd for C₁₆H₁₄FN₃O₈ (395.30): C, 48.61; H, 3.57; N, 10.63; found C,48.83; H,3.61; N,10.97; IR (KBr): *v* 3438, 3328, 3064, 2953, 1728, 1634, 1515, 1447, 1319, 1217, 1157, 1064 cm⁻¹; MS(FAB): *m*/*z* ( % rel. int.): 396 [100, (M+H)⁺ / calcd. for C₁₆H₁₄FN₃O₈ 395 (M)]; ¹H NMR (300 MHz, DMSO-d₆): δ 0.92, 0.98(2m, 4H, H₂-2'/H₂-3', 3.70(m, 1H, CH_{A}OH), 3.75(m, 1H, C*H*_{B}OH), 3.91(m,1H, H.1'), 4.65 (br m, 1H, C*H*-CO₂H), 5.47 (br s, 1H, CH₂O-*H*), 7.62 (d, *J =* 7.6 Hz, 1H, *N*-H), 8.03 (d, ³*J*_{H-F} = 13.8 Hz, 1H, H-5 ), 8.76 (s, 1H, H-2 ), 13.23(br s, 1H, CH-CO₂*H*), 14.61 (br s, 1H, C₃-CO₂H ); ¹³C NMR (75 MHz, DMSO-d₆): δ 10.1(C-2'/C-3'), 40.8(C-1'), 59.8(d, *J*_{C-F} = 11.3 Hz, CH-*N*H), 61.9(CH₂OH), 109.7(C-3), 114.9(d, ²*J*_{C-F} = 22.9 Hz, C-5), 117.2(d, ³*J*_{C-F} = 7.1 Hz, C-4a), 128.5(d, ³*J*_{C-F} = 5.7 Hz, C-8), 135.9 (C-8a), 138.5(d, ²*J*_{C-F} = 14.9 Hz, C-7), 150.9(d, ¹*J*_{C-F} = 248 Hz, C-6), 152.2(C-2), 165.4(C₃-*C*O₂H), 172.3 (CH-*C*O₂H), 175.5(d, ⁴*J*_{C-F} = 2.6 Hz, C-4).

### Example 10

### (S)- Methyl 7-(1-carboxy-2-hydroxyethylamino)-1-cyclopropyl-6-fluoro-8-nitro-4-oxo-1,4-dihydroquinoline-3-carboxylate [(S)-V-6b]

Yield (90 %) [α]_{D} = +60.9° (CHCl₃, *c*∼1); MS (EI): *m*/*z* (% rel.int.): 423 (17, M⁺), 392(14), 365(100), 346(49), 304(27), 275(34), 258(38), 243(47), 229(43), 203(47), 174(33), 162(27), 134(22), 121(15), 107(11); HRMS: calcd for C₁₈ H₁₈ F N₃ O₈ 423.10774(M⁺), found 423.10461; ¹H NMR (300 MHz, CDCl₃): δ 0.75(m, 2H) and 0.96, 1.04(2m, 2H) (H₂-2'/H₂-3'), 3.51(m, 1H, H-1'), 3.82, 3.83(2s overlapped, 6H, C(3)-CO₂CH₃ and CH-CO₂C*H*₃), 4.11(m, 1H, CH_{A}-OH), 4.33(br s, 1H, O-H), 4.35(m, 1H, CH_{B}-OH), 4.74(m, 1H, α-C*H*-NH), 7.72(d, *J* = 8 Hz, 1H, N-H), 7.76(d, ³*J*_{H-F} = 15.1 Hz, 1H, H-5), 8.47(s, 1H, H-2); ¹³C NMR (75 MHz, CDCl₃): δ 9.5, 10.1(C-2'/ C-3'), 39.2(C-1'), 52.2(C(3)-CO₂*C*H₃), 53.1(CH-CO₂*C*H₃), 60.1(d, *J*_{C-F} = 10.7 Hz, α-CH-NH), 62.9(CH₂-OH), 111.6(C-3), 116.0(d, ²*J*_{C-F} = 22.7 Hz, C-5), 119.6(d, ³*J*_{C-F} = 6.2 Hz, C-4a), 128.3(d, ³*J*_{C-F}= 5 Hz, C-8), 134.5(C-8a), 136.5(d, ²*J*_{C-F} = 14.8 Hz, C-7), 149.5(d, ¹*J*_{C-F} = 246 Hz, C-6), 151.4(C-2), 164.8(C(3)-*C*O₂Me), 170.6(d, ⁴*J*_{C-F} = 2.2 Hz, C-4), 171.2(CH-*C*O₂Me).

### Example 11

### (S) -7-(1-Carboxy-2-hydroxypropylamino)-1-cyclopropyl-6-fluoro-8-nitro-4-oxo-1,4-dihydroquinoline-3-carboxylic acid [(S)-V-7a]

This compound is prepared from interaction of **VII** and (*S*)-Threonine

Yield 0.80 g (65%), mp 230- 231°C (decomposition); [α]_{D} = +143.9° (DMSO, c∼1); *Anal*.Calcd for C₁₇H₁₆FN₃O₈ (409.32): C, 49.88; H, 3.94; N, 10.27; found C,49.70; H,3.90; N,10.10; IR (KBr): *v* 3549, 3370, 3336, 3064, 2987, 1745, 1711, 1634, 1523, 1438,1311,1268,1191,1132cm⁻¹;MS(FAB): *m*/*z* (% rel. int.): 410 [100, (M+H)⁺ / calcd. for C₁₇H₁₆FN₃O₈ 409 (M)]; ¹H NMR (300 MHz, DMSO-d₆): δ 0.93, 0.99(2m, 4H, H₂-2'/H₂-3'), 1.19(d, , *J* = 6.4 Hz, 3H, CH₃), 3.69(m, 1H, H_1', 4.29(m, 1H, C*H-*OH ), 4.45 (dd, *J* ≈ 8.6 Hz, 1H, C*H-N*H), 5.56 (br s, 1H, O-*H*), 7.39(d, *J* = 8.6 Hz, 1H, *N*-H ), 8.03 (d, ³*J*_{H-F} = 13.7 Hz, 1H, H-5 ), 8.76 (s, 1H, H-2 ), 13.14(br s, 1H, CH-CO₂*H*), 14.52(br s, 1H, C₃-CO₂H ); ¹³C NMR (75 MHz, DMSO-d₆): δ 10.1, 10.2 (C-2'/C-3'), 21.6(CH₃), 40.8(C-1'), 63.7(d, *J*_{C-F} = 11.3 Hz, CH-*N*H), 66.6(CH-OH), 109.8(C-3), 114.8(d, ²*J*_{C-F} = 22.7 Hz, C-5), 117.4(d, ³*J*_{C-F} =7.1 Hz, C-4a), 129.0(d, ³*J*_{C-F} = 5.4 Hz, C-8), 135.7 (C-8a), 139.3(d, ²*J*_{C-F}= 14.4 Hz, C-7), 150.0(d, ¹*J*_{C-F} = 248 Hz, C-6), 152.2(C-2), 165.4(C₃-*C*O₂H), 172.5 (CH-*C*O₂H), 175.6(d, ⁴*J*_{C-F} = 2.6 Hz, C-4).

### Example 12

### (S) - Methyl 7-(1-Carboxy-2-hydroxypropylamino)-1-cyclopropyl-6-fluoro-8-nitro-4-oxo-1,4-dihydroquinoline-3-carboxylate [(S)-V-7b]

Yield (85%); [*α*]_{D} = +182.5° (CHCl₃, *c*∼1); MS (EI): *m*/*z* (% rel.int.): 437 (26, M⁺), 406(16), 393(32), 379(38), 345(78), 316(52), 288(56), 285(64), 246(72), 230(80), 203(100), 188(64), 174(52), 162(43), 133(32), 121(190), 107(12); HRMS: calcd for C₁₉ H₂₀ F N₃ O₈ 437.12339(M⁺), found 437.12157; ¹H NMR (300 MHz, CDCl₃): δ 0.84, 1.07(2m, 4H, H₂-2'/ H₂-3'), 1.37(d, *J* = 6.2 Hz, 3H, CH₃-CHOH), 2.59(br s, O-H), 3.59(m, 1H, H-1'), 3.69(s, 3H, CH-CO₂CH₃), 3.88(s, 3H, C(3)-CO₂C*H*₃), 4.48(overlapped m, 2H, α-C*H*-NH, and β-C*H*-OH), 6.98(d, *J* = 9.4 Hz, 1H, N-H), 8.09(d, ³*J*_{H-F} = 13.6 Hz, 1H, H-5), 8.56(s, 1H, H-2); ¹³C NMR (75 MHz, CDCl₃): δ 10.1(C-2'/ C-3'), 20.5 CH₃-CHOH), 38.9(C-1'), 52.3(C(3)-CO₂*C*H₃), 52.9(CH-CO₂CH₃), 63.4(d, *J*_{C-F} *=* 11.3 Hz , α-CH-NH), 67.8(β-CH-OH), 112.4(C-3), 116.6(d, ²*J*_{C-F} = 22.7 Hz, C-5), 121.4(d, ³*J*_{C-F} = 6.2 Hz, C-4a), 130.4(d, ³*J*_{C-F} = 4.5 Hz, C-8), 134.2(d, ⁴*J*_{C-F} = 1.8 Hz, C-8a), 137.1 (d, ²*J*_{C-F} = 14.3 Hz, C-7), 150.2(d, ¹*J*_{C-F} = 246 Hz, C-6), 151.4(C-2), 165.3(C(3)-*C*O₂Me), 171.3(d, ⁴*J*_{C-F} = 2 Hz, C-4), 171.5(CH-*C*O₂Me).

### Example 13

### (S)- 7-(1-Carboxy-3-methylsulfanyl-propylamino)-1-cyclopropyl-6-fluoro-8-nitro-4-oxo-1,4-dihydroquinoline-3-carboxylic acid [(S) -V-8a]

This compound is prepared from interaction of **VII** and (*S*)-Methionine

Yield 0.95 g (71%), mp 205 - 206°C (decomposition) ; [*α*]_{D} = +41.6° (DMSO, c~1); *Anal*.Calcd for C₁₈H₁₈FN₃O₇S (439.42): C, 49.20; H, 4.13; N, 9.59; found C,49.22; H,4.19; N,9.36; IR (KBr): ν 3370, 3055, 2945, 2902, 1745, 1694, 1626, 1515, 1455, 1319, 1217, 1183 cm⁻¹; MS(FAB): *m* / *z* ( % rel. int.): 440 [100, (M+H)⁺ / calcd. for C₁₈H₁₈FN₃O₇S 439 (M)]; MS(EI): 405(2), 377(4), 359(21), 318(30), 317(66), 315(55), 284(30), 269(35), 246(100), 218(96), 203(58), 189(23), 174(21), 122(16); ¹H NMR (300 MHz, DMSO-d₆): δ 0.94(m, 4H, H₂-2'/ H₂-3'), 1.99(s, 3H, CH₃-S), 2.20(m, 2H, -C*H*₂CH₂-S), 2.81(m, 2H, C*H*₂-S), 3.68(m, 1H, H-1'), 4.66(m, 1H, *α-*C*H-*NH), 7.19(d, *J* = 7.6 Hz, 1H, N-H), 8.07(d, ³*J*_{H-F} = 13.8 Hz, 1H, H-5), 8.76(s, 1H, H-2), 13.33(br s, 1H, CH-CO₂*H*), 14.60(br s, 1H, C(3)-CO₂H); ¹³C NMR (75 MHz, DMSO-d₆): δ 10.1(C-2'/C-3'), 15.0(CH₃), 29.6(*β*-CH₂), 32.1(*γ*-CH₂), 40.6(C-1'), 57.5(d, *J*_{C-F} = 11.4 Hz, *α*-CH), 109.5(C-3), 114.9(d, ²*J*_{C-F} = 23.0 Hz, C-5), 117.6(d, ³*J*_{C-F} *=* 7.1 Hz, C-4a), 129.4(d, ³*J*_{C-F} = 5.2 Hz, C-8), 135.3(C-8a), 137.9(d, ²*J*_{C-F} = 14.8 Hz, C-7), 150.3(d, ¹*J*_{C-F} = 249 Hz, C-6), 152.0(C-2), 165.7(C₃-*C*O₂H), 173.4 (*α-C*O₂H), 175.4(d, ⁴*J*_{C-F} = 2.3 Hz, C-4).

### Example 14

### (S)- Methyl 1-Cyclopropyl-6-fluoro-7-(1-methoxycarbonyl-3-methylsulfanylpropylamino)-8-nitro-4-oxo-1,4-dihydroquinoline-3-carboxylate [(S) -V-8b]

Yield (92 %), [*α*]_{D} = +174.8° (CHCl₃, *c*∼1); *Anal.* Calcd for C₂₀H₂₂FN₃O₇S (467.47): C, 51.39; H, 4.74; N,8.99; found C,51.27; H,4.75; N,8.86; MS (EI): *m*/*z* (% rel.int.): 468 (5, M+1)⁺, 436(7), 409(35), 408(16), 360(13), 342(47), 306(14), 288(20), 275(22), 243(26), 217(26), 172(16), 162(16), 147(27), 133(9), 61(100); HRMS: calcd for C₂₀H₂₃FN₃O₇S 468.12403 (M+H)⁺, found 468.11952; ¹H NMR (300 MHz, CDCl₃): δ 0.83, 1.07(2m, 4H, H₂-2'/ H₂-3'), 2.04(m, 1H, -S-CH₂CH_{A}), 2.08(s, 3H, CH₃-S), 2.21(m, 1H, S-CH₂C*H*_{B}), 2.58(m, 2H, S-CH₂), 3.58(m, 1H, H-1'), 3.71(s, 3H, CH-CO₂C*H*₃), 3.88(s, 3H, C(3)-CO₂CH₃), 4.74(m, 1H, C*H*-CO₂Me), 6.66(d, *J* = 8.7 Hz, 1H, N-H), 8.12(d, ³*J*_{H-F} = 13.4 Hz, 1H, H-5), 8.56(s, 1H, H-2); ¹³C NMR (75 MHz, CDCl₃): δ 10.1(C-2'/ C-3'), 15.5(CH₃-S), 29.7(S-CH₂), 32.8(S-CH₂CH₂), 38.9(C-1'), 52.4(C(3)-CO₂*C*H₃), 52.9(CH-CO₂*C*H₃), 57.4(d, *J*_{C-F} = 11.7 Hz, α-CH-NH), 112.5(C-3), 116.9(d, ²*J*_{C-F} = 22.7 Hz, C-5), 121.7(d, ³*J*_{C-F} = 6.1 Hz, C-4a), 130.3(d, ³*J*_{C-F} = 4.5 Hz, C-8), 134.1(d, ⁴*J*_{C-F} = 1.7 Hz, C-8a), 136.2(d, ²*J*_{C-F}= 14.6 Hz, C-7), 149.9(d, *¹J*_{C-F} = 246 Hz, C-6), 151.5(C-2), 165.2(C(3)-*C*O₂Me),171.1(d, ⁴*J*_{C-F} = 2.2_Hz, C-4), 172.3(CH-*C*O₂Me).

### Example 15

### (S)- 7-(5-Acetylamino-1-carboxypentylamino)-1-cyclopropyl-6-fluoro-8-nitro-4-oxo-1,4-dihydroquinoline-3-carboxylic acid [(S)-V-9a]

This compound is prepared from interaction of **VII** and (*S*)-N-Ac-Lysine

Yield 0.75 g (52 %), mp 124- 126°C (decomposition); *Anal*.Calcd for C₂₁H₂₃FN₄O₈ (478.43): C, 52.72; H, 4.85; N, 11.71; found C,52.56; H,4.99; N,11.45; IR (KBr): *∨* 3345, 3072, 2936, 2851, 1728, 1626, 1540, 1515, 1455, 1311, 1217, 1140 cm⁻¹; MS(FAB): *m*/*z* ( % rel. int.): 479 [100, (M+H)⁺ / calcd. for C₂₁H₂₃FN₄O₈ 478 (M)]; ¹H NMR (300 MHz, DMSO-d₆):δ 0.92, 0.97(2m, 4H, H₂-2'/H₂-3'), 1.25(m, 2H, *γ-*CH₂), 1.34(m, 2H, *δ*-CH₂), 1.71(s, 3H, CH₃), 1.82(m, 2H, *β*-CH₂), 2.93(m, 2H, *ε-*CH₂), 3.69(m, 1H, H.1'), 4.55 (m, 1H, α-C*H*-CO₂H), 7.27 (d, *J* = 7.3 Hz, 1H, Ar-N-*H* ), 7.76 (t, *J* = 5.2 Hz, 1H, O=C-NH), 8.04(d, ³*J*_{H-F} = 13.6 Hz, 1H, H-5), 8.75 (s, 1H, H-2 ), 13.88(br s, 1H, CH-CO₂*H*), 14.57 (br s, 1H, C₃-CO₂H ); ¹³C NMR (75 MHz, DMSO-d₆): δ 10.2(C-2'/C-3'), 22.4(*γ*-CH₂), 23.0(CH₃), 29.2(*δ*-CH₂), 32.5(*β*-CH₂), 38.2(*ε*-CH₂), 40.7(C-1'), 58.1(d, *J*_{C-F} = 11.4 Hz, *α*-CH-*N*H), 109.6(C-3), 115.1(d, ²*J*_{C-F} = 22.7 Hz, C-5), 117.5(d, ³*J*_{C-F} = 7.2 Hz, C-4a), 129.0(d, ³*J*_{C-F} = 5.5 Hz, C-8), 135.7 (C-8a), 138.0(d, ²*J*_{C-F} = 15.0 Hz, C-7), 150.3(d, ¹*J*_{C-F} = 248 Hz, C-6), 152.2(C-2), 165.4(C₃-*C*O₂H), 169.4(O=*C*-Me), 173.5 (CH-*C*O₂H), 175.5(d, ⁴*J*_{C-F} = 2.4 Hz, C-4).

### Example 16

### (S)-Methyl 7-(5-Acetylamino-1-carboxypentylamino)-1-cyclopropyl-6-fluoro-8-nitro-4-oxo-1,4-dihydroquinoline-3-carboxylate [(S)-V-9b]

Yield (85 %), MS(EI): *m* / *z* ( % rel. int.): 507 [13, (M+H)⁺], HRMS calcd. for C₂₃H₂₈FN₄O₈ 507.18906, found 507.19360; 489 (24), 457(30), 447(58), 416(59), 402(30), 305(11), 258(17), 245(26), 217(19), 142(22), 126(24), 100(100); ¹H NMR (300 MHz, CDCl₃): δ 0.84, 1.07(H₂-2'/ H₂-3'), 1.40(m, 2H, γ-CH₂), 1.51(m, 2H, δ-CH₂), 1.82(m, 2H, β-CH₂), 1.94(s, 3H, CH₃-C=O), 3.21(m, 2H, ε-CH₂), 3.58(m, 1H, H-1'), 3.71(s, 3H, CH-CO₂C*H*₃), 3.88(s, 3H, C(3)-CO₂CH₃), 4.59(m, 1H, α- C*H-*CO₂Me), 5.69(br s, 1H, H-N-C=O), 6.71(d, *J =* 8.4 Hz, 1H, Ar-N*H*), 8.10(d, ³*J*_{H-F} = 13.5 Hz, 1H, H-5), 8.57(s, 1H, H-2); ¹³C NMR (75 MHz, CDCl₃): δ 10.1(C-2'/ C-3'), 22.4(γ-CH₂), 23.4(CH₃-C=O), 29.1(δ-CH₂), 33.1(β-CH₂), 39.0(C-1'), 39.1(ε-CH₂-NH), 52.4(C(3)-CO₂CH₃), 52.8(CH-CO₂*C*H₃), 58.1(d, *J*_{C-F} = 14.9 Hz, α-*C*H-CO₂Me), 112.3(C-3), 116.8(d, ²*J*_{C-F} = 22.7 Hz, C-5), 121.4(d, ³*J*_{C-F} = 5.8 Hz, C-4a), 130.0(d, ³*J*_{C-F} = 4.5 Hz, C-8), 134.3(C-8a), 136.3(d, ²*J*_{C-F} = 14.7 Hz, C-7), 149.9(d, ¹*J*_{C-F} = 247 Hz, C-6), 151.4(C-2), 165.2(C(3)-*C*O₂Me), 170.3 (O=C-NH), 171.3(d, ⁴*J*_{C-F} = 2 Hz, C-4), 172.4(CH-*C*O₂Me).

### Example 17

### (S)-2-[(3-Carboxy-1-cyclopropyl-6-fluoro-8-nitro-4-oxo-1,4-dihydroquinolin-7-yl)amino] succinic acid [(S)-V-10a]

This compound is prepared from interaction of **VII** and (*S*)-aspartic acid

Yield (74 %), mp 213- 214°C (decomposition); [*α*]_{D} = +142.3° (DMSO, *c*∼1); *Anal*.Calcd for C₁₇H₁₄FN₃O₉ (423.31): C, 48.24; H, 3.33; N, 9.93; found C,48.31; H,3.38; N,9.70; IR (KBr): *ν* 3481, 3336, 3072, 1736, 1626, 1515, 1447, 1319, 1208, 1030 cm⁻¹; MS(FAB): *m*/*z* ( % rel. int.): 424 [100, M+H)⁺ / calcd. for C₁₇H₁₄FN₃O₉ 423 (M)]; ¹H NMR (300 MHz, DMSO-d₆): δ 0.79(m, 3H) and 1.02(m, 1H) (H₂-2'/ H₂-3'), 3.14(dd, *J =* 17.1 Hz, 4.8 Hz, 1H, *β*-C*H*_{A}-CO₂H), 3.21(dd, *J =* 17.1 Hz, 4.6 Hz, 1H, *β*-C*H*_{B}-CO₂H), 3.66(m, 1H, H-1'), 4.82(m, 1H, *α*-C*H*-(NH)), 7.48(d, *J =* 8.8 Hz, 1H, N-H), 8.05(d, ³*J*_{H-F} = 13.6 Hz, 1H, H-5), 8.75(s, 1H, H-2), 12.65-13.35(overlapped 2br s, 2H, *α*-CH-CO₂*H* and *β*-CH₂CO₂*H*), 14.31(br s, 1H, C(3)-CO₂*H*); ¹³C NMR (75 MHz, DMSO-d₆): δ 10.2(C-2'/ C-3'), 37.2(*C*H₂-CO₂H), 40.6(C-1'), 55.0(d, *J*_{C-F} = 12.1 Hz, *α*-*C*H(NH)CO₂H), 109.7(C-3), 115.0(d, ²*J*_{C-F} = 23.0 Hz, C-5), 117.8(d, ³*J*_{C-F} = 7.2 Hz, C-4a), 129.7(d, ³*J*_{C-F} = 5.1 Hz, C-8), 135.4(C-8a), 138.1(d, ²*J*_{C-F} = 14.2 Hz, C-7), 150.6(d, ¹*J*_{C-F} = 248 Hz, C-6), 152.3(C-2), 165.3(C(3)-*C*O₂H), 172.6(CH₂-*C*O₂H and CH-*C*O₂H / superimposed), 175.6(d, ⁴*J*_{C-F} = 2.5 Hz, C-4).

### Example 18

### (S) - Dimethyl 2-{[1-cyclopropyl-6-fluoro-3-(methoxycarbonyl)- 8-nitro -4-oxo-1,4-dihydroquinolin-7-yl]amino}succinate [(S)-V-10b]

Yield (88%), mp 135 - 136 °C (decomposition); [*α*]_{D} = +327.5° (CHCl₃, *c*∼1); *Anal*.Calcd for C₂₀H₂₀FN₃O₉ (465.39): C, 51.62; H, 4.33; N, 9.03; found C,51.72; H,4.33; N,9.18; MS (EI): *m*/*z* (% rel.int.): 465 (8, M⁺), 434(6), 407(100), 374(21), 359(9), 345(9), 314(23), 275(33), 242(15), 215(21), 188(15), 161(10), 147(8), 113(20), 101(10); HRMS: calcd for C₂₀ H₂₀ F N₃ O₉ 465.11830 (M⁺), found 465.11734;

¹H NMR (300 MHz, CDCl₃): δ 0.84, 1.07(2m, 4H, H₂-2'/ H₂-3'), 2.95(dd, *J* = 16.9 Hz, 3.8 Hz, 1H, *β*-C*H*_{A}-CO₂Me), 3.09(dd, *J* = 16.9 Hz, 4.4 Hz, 1H, *β*-C*H*_{B}-CO₂Me), 3.58(m, 1H, H-1'), 3.69(s, 3H, CH-CO₂C*H*₃), 3.70(s, 3H, CH₂-CO₂C*H*₃), 3.88(s, 3H, C(3)-CO₂C*H*₃), 4.76(m, 1H, α- C*H*-NH), 7.02(d, *J* = 9.5 Hz, 1H, N-H), 8.13(d, ³*J*_{H-F} = 13.4 Hz, 1H, H-5), 8.56(s, 1H, H-2); ¹³C NMR (75 MHz, CDCl₃): δ 10.1, 10.2(C-2'/ C-3'), 37.2(*C*H₂-CO₂Me), 38.9(C-1'), 52.3(C(3)-CO₂*C*H₃ and CH₂-CO₂*C*H₃ / superimposed), 53.2(CH-CO₂*C*H₃), 55.0(d, *J*_{C-F} = 11.9 Hz, α-CH-NH), 112.4(C-3), 116.7(d, ²*J*_{C-F} = 22.7 Hz, C-5), 122.1 (d, ³*J*_{C-F} = 6.3 Hz, C-4a), 131.2(d, ³*J*_{C-F} = 5 Hz, C-8), 133.9(d, ⁴*J*_{C-F} = 1.8 Hz, C-8a), 136.1 (d, ²*J*_{C-F} = 14.3 Hz, C-7), 150.3(d, ¹*J*_{C-F} = 246 Hz, C-6), 151.5(C-2), 165.2(C(3)-*C*O₂Me), 170.7(CH₂-*C*O₂Me), 171.2(d, ⁴*J*_{C-F} = 2.1 Hz, C-4), 171.3(CH-*C*O₂Me).

### Example 19

### (S) - 2-[(3-carboxy-1-cyclopropyl-6-fluoro-8-nitro-4-oxo-1,4-dihydroquinolin-7-yl)amino] pentanedioic acid [(S)-V-11a]

This compound is prepared from interaction of **VII** and (*S*)-Glutamic acid

Yield (77 %), mp 207- 207 °C (decomposition) ; [*α*]_{D} = +89.5° (DMSO, c∼1); *Anal*.Calcd for C₁₈H₁₆FN₃O₉ (437.33): C, 49.43; H, 3.69; N, 9.61; found C,49.10; H,3.66; N,9.26; IR (KBr): *ν* 3515, 3345, 3174, 3098, 2962, 2919, 1736, 1711, 1626, 1516, 1464, 1328, 1209, 1192 cm⁻¹; MS(FAB): *m*/*z* (% rel. int.): 438 [100, (M+H)⁺ / calcd. for C₁₈H₁₆FN₃O₉ 437 (M)]; ¹H NMR (300 MHz, DMSO-d₆): δ 0.96 ( m, 4H, H₂-2'/ H₂-3'), 2.10 ( m, 2H, *β*-CH₂), 2.33 (2d, *J* = 7.0 Hz, 6.9 Hz, 2H, γ-CH₂), 3.69 ( m, 1H, H-1'), 4.60 ( m, 1H, *α*-CH ), 7.75 ( d, *J =* 7.8 Hz, 1H, *N-*H ), 8.05 ( d, ³*J*_{H-F} = 13.6 Hz, 1H, H-5) 8.75 ( s, 1H, H-2 ), 12.10-12.80 ( overlapped 2 br s, 2H, *α*-CO₂H, γ-CO₂H), 14.47 (br s, 1H, C₃-CO₂H); ¹³C NMR (75 MHz, DMSO-d₆): δ 10.2 (C-2'/C-3'), 27.9(C-*β*), 30.1(C-*γ*), 40.6(C-1'), 57.8(d, *J*_{C-F}= 11.5 Hz, C-*α*), 109.6 (C-3), 114.9(d, ²*J*_{C-F} = 23.0 Hz, C-5), 117.7(d, ³*J*_{C-F} = 7.1 Hz, C-4a), 129.5(d, ³*J*_{C-F} = 5.1 Hz, C-8), 135.4 (C-8a), 138.1(d, ²*J*_{C-F} = 14.6 Hz, C-7), 150.4(d, ¹*J*_{C-F} = 248.0 Hz, C-6), 152.2(C-2), 165.4(C₃-*C*O₂H), 173.3(*α*-CO₂H), 174.2 (*γ*-CO₂H), 175.6(d, ⁴*J*_{C-F} = 2.6 Hz, C-4).

### Example 20

### (S) - Dimethyl 2-{[1-cyclopropyl-6-fluoro-3-(methoxycarbonyl)-8-nitro -4-oxo-1,4-dihydroquinolin-7-yl]amino}pentanedioate [(S)-V-11b]

Yield (82 %), mp 114- 115°C (decomposition); [*α*]_{D} = +218.8° (CHCl₃, *c*∼1); *Anal*.Calcd for C₂₁H₂₂FN₃O₉ (479.41): C, 52.61; H, 4.63; N, 8.76; found C, 52.54; H, 4.75; N, 8.85; IR (KBr): *ν* 3339, 3092, 3012, 2956, 1721, 1616, 1511, 1462, 1437, 1326, 1258, 1162 cm⁻¹; MS (EI): *m*/*z* (% rel.int.): 479 (5, M⁺), 448(10), 431(12), 421(100), 402(27), 388(27), 370(62), 342(40), 332(24), 289(35), 256(24), 243(24), 230(27), 216(24), 188(20), 159(35), 127(23), 115(27); HRMS: calcd for C₂₁ H₂₂ F N₃ O₉ 479.13395(M⁺), found 479.12798; ¹H NMR (300 MHz, CDCl₃): δ 0.83, 1.07(2m, 4H, H₂-2'/ H₂-3'), 2.09(m, 1H, *β*-C*H*₂-CHNH), 2.28(m, 1H, β-C*H*_{B}-CHNH), 2.48(overlapped 2d, 2H, *J =* 6.9, 7.1 Hz, C*H*₂-CO₂Me), 3.57(m,1H, H-1'), 3.66(s, 3H, CH₂-CO₂C*H*₃), 3.70(s, 3H, CH-CO₂C*H*₃), 3.88(s, 3H, C(3)-CO₂C*H*₃), 4.63(m, 1H, α-C*H*-NH), 6.59(d, *J* = 8.8 Hz, 1H, N-H), 8.11(d, ³*J*_{H-F}= 13.5 Hz, 1H, H-5), 8.55(s, 1H, H-2); ¹³C NMR (75 MHz, CDCl₃): δ 10.0, 10.1(C-2'/ C-3'), 28.4(β-CH_{A}-CHNH), 29.9(*C*H₂-CO₂Me), 38.9(C-1'), 52.0(CH₂-CO₂*C*H₃), 52.3(C(3)-CO₂*C*H₃), 52.9(CH-CO₂*C*H₃), 57.7(d, *J*_{C-F} = 11.8 Hz, α-CH-NH), 112.4(C-3), 116.8(d, *²J*_{C-F}= 22.8 Hz, C-5), 121.6(d, *³J*_{C-F}= 6.2 Hz, C-4a), 130.5(d, ³*J*_{C-F} = 4.4 Hz, C-8), 134.1(d, ⁴*J*_{C-F} = 1.7 Hz, C-8a), 136.1 (d, ²*J*_{C-F} = 14.6 Hz, C-7), 149.9(d, *J*_{C-F} = 247 Hz, C-6), 151.5(C-2), 165.2(C(3)-*C*O₂Me), 171.1(d, ⁴*J*_{C-F} = 1.9 Hz, C-4), 172.1(CH-*C*O₂Me), 172.7(CH₂-*C*O₂Me).

### Example 21

### (S) - 7-[(1-Carboxy-2-phenylethyl)amino]-1-cyclopropyl-6-fluoro-8-nitro-4-oxo-1,4-dihydroquinoline-3-carboxylic acid [(S)-V-12a]

A stirred mixture of (*S*)-phenylalanine [1.65 g, 10 mmol], **VII** (1.0 g, 3 mmol) and sodium hydrogen carbonate (1.6 g, 19 mmol) in 50 % aqueous ethanol (150 ml) was heated at 78-80 °C for 72-84 h under reflux conditions. Work-up of the reaction mixture as described for **V-1a** above, gave the title compound as fluorescent yellow solid that was recrystallized from chloroform / methanol (2:1, v/v).

Yield 0.97 g (71 %), mp 233- 234 °C (decomposition); MS(FAB): *m*/*z* ( % rel. int.): 456[100, (M+H)⁺ / calcd. for C₂₂H₁₈FN₃O₇ 455(M)]; ¹H NMR (300 MHz, DMSO-d₆): δ 0.91(m, 4H, H₂-2'/ H₂-3'), 3.17(m, 2H, *β*-C*H*₂Ph), 3.61(m, 1H, H-1'), 4.88(m, 1H, *α*-C*H*-NH), 7.09(m, 2H, H-2"/ H-6"), 7.15(m, 3H, H-3"/ H-5", H-4"), 7.23(d, *J* = 6.5 Hz, 1H, N-H), 8.01(d, ³*J*_{H-F} = 13.7 Hz, 1H, H-5), 8.73 (s, 1H, H-2), 13.8(br s, 1H, CH- CO₂*H*), 14.56(br s, 1H, C(3)-CO₂H); ¹³C NMR (75 MHz, DMSO-d₆): δ 10.1, 10.2(C-2'/ C-3'), 38.6(*β*-*C*H₂Ph), 40.7(C-1'), 59.1(d, *J*_{C-F} = 11.6 Hz, *α*-CH-NH),109.7(C-3), 115.1(d, ²*J*_{C-F} = 22.8 Hz, C-5), 117.6(d, ³*J*_{C-F} = 7.2 Hz, C-4a), 127.5 (C-4"), 128.9(C-2"/ C-6"), 129.0(d, ³*J*_{C-F} = 5.8 Hz, C-8), 129.7(C-3"/ C-5"), 135.6(d, ⁴*J*_{C-F} = 1.1 Hz, C-8a), 136.1(C-1"), 137.5(d, ²*J*_{C-F} = 14.4 Hz, C-7), 150.1(d, ¹*J*_{C-F} = 248 Hz, C-6), 152.2(C-2), 165.4(C(3)-*C*O₂H), 172.8(CH-*C*O₂H), 175.5(d, ⁴*J*_{C-F} = 2.5 Hz, C-4).

### Example 22

### (S)-7-{[Carboxy(phenyl)methyl]amino}-1-cyclopropyl-6-fluoro-8-nitro-4-oxo-1,4-dihydroquinoline-3-carboxylic acid [(S)-V-13a]

A stirred mixture of (*S*)-phenylglycine [1.36 g, 9 mmol], **VII** (1.0 g, 3 mmol) and sodium hydrogen carbonate (1.5 g, 18 mmol) in 50 % aqueous ethanol (140 ml) was heated at 72-75 °C for 70-80 h under reflux conditions. Work-up of the reaction mixture as described for **V-1a** above, gave the title compound as fluorescent yellow solid that was recrystallized from chloroform / ethanol (2:1, v/v).

Yield 0.70 g (53 %), mp 205 - 207 °C; MS(FAB): *m*/*z* ( % rel. int.): 442[100, (M+H)⁺ / calcd. for C₂₁H₁₆FN₃O₇ 441(M)]; ¹H NMR (300 MHz, DMSO-d₆): δ 0.96(m, 4H, H₂-2'/ H₂-3'), 3.68(m, 1H, H-1'), 5.67(dt, *J* = 6 Hz, 3 Hz, 1H, *α*-C*H-*Ph), 7.31(m, 5H, H-2"/ H-6", H-3"/ H-5", H-4"), 7.95(d, ³*J*_{H-F} = 13.2 Hz, 1H, H-5), 8.04(br d, *J* = 6 Hz, 1H, N-H), 8.75(s, 1H, H-2), 13.36(br s, 1H, CH-CO₂*H*), 14.48(br s, 1H, C(3)-CO₂H); ¹³C NMR (75 MHz, DMSO-d₆): δ 10.1(C-2'/ C-3'), 40.8(C-1'), 61.4(*α*-*C*H-Ph), 109.8(C-3), 115.1(d, ²*J*_{C-F} = 21.9 Hz, C-5), 117.8(d, *³J*_{C-F} *=* 6.8 Hz, C-4a), 127.2(C-2"/ C-6"), 129.0(C-4"), 129.1(d, *³J_{C-F}* = 5.5 Hz, C-8), 129.5(C-3"/ C-5"), 135.6(C-8a), 137.0(d, ²*J*_{C-F} = 15.9 Hz, C-7), 138.1(C-1"), 150.0(d, ¹*J*_{C-F}= 249 Hz, C-6), 152.2(C-2), 165.3(C(3)-*C*O₂H), 172.2(CH-*C*O₂H), 175.5(d, ⁴*J*_{C-F} = 2.3 Hz, C-4).

### Example 23

### (S)-7-(1-Carboxypropylamino)-1-cyclopropyl-6-fluoro-8-nitro-4-oxo-1,4-dihydroquinoline-3-carboxylic acid [(S)-V-14a]

This compound is prepared from interaction of **VII** and (*S*)-2-aminobutyric acid

Yield 1.05 g (87 %), mp 199- 201°C (decomposition); *Anal*.Calcd for C₁₇H₁₆FN₃O₇ (393.32): C, 51.91; H, 4.10; N, 10.68; found C, 51.64; H,4.16; N,10.39; IR (KBr): *ν* 3302, 3064, 2970, 2928, 2877, 1736, 1711, 1626, 1540, 1464, 1328, 1217, 1149, 1038 cm⁻¹; ¹H NMR (300 MHz, DMSO-d₆): δ 0.82(t, ³*J*_{H-F} = 7.3 Hz, 3H, CH₃), 0.90, 0.97(2m, 4H, H₂-2'/ H₂-3'), 1.87(m, 2H, C*H*₂Me), 3.68(m, 1H, H-1'), 4.58(m, 1H, *α-*C*H*-NH), 7.31(d, *J* = 7.4 Hz, 1H, N-H), 8.07(d, ³*J*_{H-F} = 13.7 Hz, 1H, H-5), 8.77(s, 1H, H-2), 13.38(br s, 1H, CH-CO₂*H*), 14.60(br s, 1H, C(3)-CO₂H); ¹³C NMR (75 MHz, DMSO-d₆): δ 9.3(CH₃), 10.1 (C-2'/C-3'), 26.0(*β*-CH₂), 40.8(C-1'), 58.9(d, *J*_{C-F} = 11.0 Hz, *α*-CH), 109.7 (C-3), 115.1 (d, *²J*_{C-F}= 22.8 Hz, C-5), 117.4(d, *³J_{C-F}=* 7.0 Hz, C-4a), 129.0(d, ³*J*_{C-F} = 5.5 Hz, C-8), 135.7 (C-8a), 137.9(d, ²*J*_{C-F} = 15.1 Hz, C-7), 150.3(d, ¹*J*_{C-F} = 248 Hz, C-6), 152.1(C-2), 165.5(C₃-*C*O₂H), 173.4 (*α*-*C*O₂H), 175.5(d, *⁴J_{C-F}=* 2.7 Hz, C-4).

### Example 24

### (S)-Methyl 1-(cyclopropyl-6-fluoro-7-(1-methoxycarbonyl-propylamino)-8-nitro-4-oxo-1,4-dihydro-quinoline-3-carboxylate [(S)-V-14b]

Yield g (91 %); *Anal*.Calcd for C₁₉H₂₀FN₃O₇ (421.38): C, 54.16; H, 4.78; N, 9.97; found C,53.78; H,4.89; N,9.95; IR (KBr): *ν* 3358, 3092, 3012, 2950, 1728, 1700, 1617, 1512, 1474, 1326, 1240, 1166 cm⁻¹; ¹H NMR (300 MHz, CDCl₃): δ 0.83, 1.07(2m, 4H, H₂-2'/ H₂-3'), 0.95(t, *J* = 7.1 Hz, 3H, C*H*₃CH₂), 1.88(m, 2H, β-C*H*₂Me), 3.58(m, 1H, H-1'), 3.71(s, 3H, CH-CO₂C*H*₃), 3.88(s, 3H, C(3)-CO₂CH₃), 4.59(m, 1H, α- C*H*-NH), 6.82(d, *J =* 8 Hz, 1H, N-H), 8.10(d, ³*J*_{H-F} = 13.4 Hz, 1H, H-5), 8.56(s, 1H, H-2); ¹³C NMR (75 MHz, CDCl₃): δ 9.3(*C*H₃CH₂), 10.0, 10.1(C-2'/ C-3'), 26.6(β-*C*H₂Me), 39.0(C-1'), 52.4(C(3)-CO₂*C*H₃), 52.8(CH-CO₂*C*H₃), 59.1(d, *J*_{C-F} = 11.7 Hz, α-CH-NH), 112.4(C-3), 116.8(d, *²J*_{C-F}*=* 22.7 Hz, C-5), 121.3(d, ³*J*_{C-F} = 6 Hz, C-4a), 129.7(d, ³*J*_{C-F} = 5 Hz, C-8), 134.3(C-8a), 136.3(d, ²*J*_{C-F} = 14.9 Hz, C-7), 149.8(d, ¹*J*_{C-F} = 247 Hz, C-6), 151.5(C-2), 165.3(C(3)-*C*O₂Me), 171.2(d, ⁴*J*_{C-F} = 2.1 Hz, C-4), 172.5(CH-*C*O₂Me).

### Example 25

### 7-[(1-Carboxycyclopentyl)amino]-1-cyclopropyl-6-fluoro-8-nitro-4-oxo-1,4-dihydroquinoline-3-carboxylic acid [VB-3a]

This compound is prepared from interaction of **VII** and (*S*)-1-aminocyclopentane-1-carboxylic acid

Yield 0.133 g (11 %), mp 243 - 245 °C (decomposition, changes color around 230 °C); *Anal*.Calcd for C₁₉H₁₈FN₃O₇ (419.11): C, 54.42; H, 4.33; N, 10.02; found C,54.54; H,4.38; N,9.70; IR (KBr): *ν* 3379, 3108, 2970, 1745, 1625, 1598, 1515, 1455, 1436, 1328, 1234, 1166, 1038 cm⁻¹; MS(FAB): *m* / *z* ( % rel. int.): 420 [100, (M+H)⁺ / calcd. for C₁₉H₁₈FN₃O₇ 419 (M)]; MS(EI): 399*(51, M-HF) *, 355(94), 310(83), 297(65), 282(57), 254(41), 245(23), 213(29), 200(20), 185(16), 171(14), 95(25), 67(37), 44(100);

*HRMS calcd. for C₁₉H₁₇N₃O₇ 399.106607, found 399.106410;

¹H NMR (300 MHz, DMSO-d₆): δ 0.92, 0.97(2m, 4H, H₂-2' / H₂-3'), 1.69(m, 4H, H₂-3" / H₂-4"), 1.98, 2.25(2m, 4H, H₂-2" / H₂-5"), 3.68 (m, 1H, H-1'), 7.20 ( s, 1H, *N*-H ), 8.35 (d, ³*J*_{H-F} = 11.2 Hz, 1H, H-5 ), 8.75(s, 1H, H-2 ), 12.94 (br s, 1H, C_{1"}-CO₂H), 14.60(br s, 1H, C₃-CO₂H ); ¹³C NMR (75 MHz, DMSO-d₆): δ 10.2 (C-2' / C-3'), 25.1 (C-3" / C-4"), 40.1 (C-2" / C-5"), 40.7 (C-1'), 69.9 (d, *J*_{C-F} = 3.3 Hz , C-1"), 109.7(C-3), 115.1 (d, ²*J*_{C-F} = 23.6 Hz , C-5), 117.4 (d, ³*J*_{C-F} = 7.3 Hz, C-4a), 128.9 (d, ³*J*_{C-F} = 5.8 Hz, C-8), 137.0 (d, ²*J*_{C-F}= 16.2 Hz, C-7), 135.8 (C-8a), 149.5 (d, ¹*J*_{C-F} = 247 Hz, C-6), 152.2 (C-2), 165.4 (C₃- *C*O₂H), 175.5 (d, *J*_{C-F} = 3.4 Hz, C_{1"}-*C*O₂H), 175.6 (d, ⁴*J*_{C-F} = 2.7 Hz, C-4)

### Example 26

### 10-Cyclopropyl-5-fluoro-2,7-dioxo-1,2,3,4,7,10-hexahydropyrido[2,3-f]quinoxaline-8-carboxylic acid (III-1a)

To a vigorously stirred solution of 7-[(carboxymethyl)amino]-1-cyclopropyl-6-fluoro-8-nitro-4-oxo-1,4-dihydroquinoline-3-carboxylic acid (**V-1a** / 0.37 g, 1.0 mmol) and potassium carbonate (0.96 g, 7.0 mmol) in water (20 ml), was added portionwise a solution of sodium dithionite (1.22 g, 7.0 mmol) in water (5 ml). Following each addition, the solution acquired a dark brown color which shortly faded away (1-2 min). The reaction mixture was further stirred at rt for 25 min. Thereafter, the pH of the resultant solution was adjusted to about 4 by slow addition of 3*N* HCl, the precipitated product was filtered, washed successively with water and methanol, and air-dried.

Yield 0.17 g (54 %), mp 290- 292 °C (decomposition / the crystals darken at 270 °C). MS (TOF, ES⁺): *m* / *z* 318 (M + H)⁺, HRMS calcd for C₁₅H₁₃FN₃O₄ 318.0890, found 318.0885; *m* / *z* 340 (M + Na)⁺, HRMS calcd for C₁₅H₁₂FN₃O₄Na 340.0709, found 340.0704; ¹H NMR (300 MHz, 3% NaOD in D₂O): δ 0.21, 0.69(2m, 4H, H₂-2'/ H₂-3'), 3.27(s, 2H, H₂-3), 3.98(m, 1H, H-1'), 7.00(d, *³J*_{*H-*F} = 10.8 Hz, 1H, H-6), 8.17(s, 1H, H-9); ¹³C NMR (75 MHz, 3% NaOD in D₂O): δ 9.4 (C-2'/ C-3'), 40.1(C-1'), 42.9(C-3), 102.3(d, ²*J*_{C-F} = 20.2 Hz, C-6), 114.8 (C-8), 119.9(d, ³*J*_{C-F}= 7 Hz, C-6a), 128.0(d, ³*J*_{C-F} = 3.7 Hz, C-10b), 130.0(d, ²*J*_{C-F} = 15.5 Hz, C-4a), 131.9 (C-10a), 148.9 (C-9),149.2(d, ¹*J*_{C-F} = 240 Hz, C-5), 168.1(C-2), 173.1(C(8)-CO₂⁻), 175.6 (d, ⁴*J*_{C-F} = 2.9 Hz, C-7).

### Example 27

### (S) - 10-Cyclopropyl-5-fluoro-3-methyl-2,7-dioxo-1,2,3,4,7,10-hexahydropyrido[2,3-f]quinoxaline-8-carboxylic acid [(S)-III-2a]

To a stirred solution of **V-2a** (0.38 g, 1.0 mmol) and potassium carbonate (0.96 g, 7.0 mmol) in water (20 ml), was added portionwise a solution of sodium dithionite (1.22 g, 7.0 mmol) in water (5 ml). The reaction mixture was further stirred at rt for 10 min, and worked up as described for **III-1** a above. The title product was recrystallized from chloroform/ methanol (3:1, v/v) to furnish pale yellow fine crystals.

Yield 0.21 g (64 %), mp 315 - 317 °C (decomposition / the crystals darken at 290 °C); MS (TOF ES ⁺): *m*/*z* 354 (M+ Na)⁺, HRMS calcd for C₁₆H₁₄FN₃O₄Na 354.0866, found 354.0861; ¹H NMR (300 MHz, DMSO-d₆): δ 0.97, 1.05(2m, 4H, H₂-2'/ H₂-3'), 1.32(d, *J* = 6.6 Hz, 3H, CH₃), 3.93(br q, *J* = 6.6 Hz, 1H, H-3), 4.48(m, 1H, H-1'), 7.48(br s, 1H, N(4)-H), 7.62(d, *³J*_{*H-*F} *=* 10.6 Hz, 1H, H-6), 8.65(s, 1H, H-9), 10.34(s,1H, lactam N(1)-H), 15.13(s, 1H, CO₂H); ¹³C NMR (75 MHz, DMSO-d₆): δ 10.2 (C-2'/ C-3'), 16.2 (CH₃), 39.0(C-1'), 50.4 (C-3), 105.4(d, ²*J*_{C-F} = 19.6 Hz, C-6), 107.2 (C-8), 116.1(d, ³*J*_{C-F} = 6.3 Hz, C-10b), 117.2(d, ³*J*_{C-F} = 7.1 Hz, C-6a), 129.3 (C-10a), 131.9(d, ²*J*_{C-F} = 18.2 Hz, C-4a), 149.4(d, ¹*J*_{C-F} = 242 Hz, C-5), 151.0 (C-9), 166.2(CO₂H), 166.5(C-2), 176.6(d, ⁴*J*_{C-F} = 3.1Hz, C-7).

### Example 28

### (S)- 10-Cyclopropyl-5-fluoro-3-isopropyl-2,7-dioxo-1,2,3,4,7,10-hexahydropyrido[2,3-f]quinoxaline-8-carboxylic acid [(S) - III-3a]

To a stirred solution of (±)-7-(1-carboxy-2-methylpropylamino)-1-cyclopropyl-6-fluoro-8-nitro-4-oxo-1,4-dihydroquinoline-3-carboxylic acid (**V-3a,** 0.36 g, 1 mmole) and (0.96 g, 7 mmol) of potassium carbonate in 20 ml water; an aqueous solution of sodium dithionite (0.87 g, 5 mmol) in 5 ml water, was added drop-wise. The reaction mixture was further stirred at r.t. for 15-25 minutes. Thereafter, the pH of the solution was adjusted to about 4.0. The precipitate product was filtered, washed with water and air-dried. Then further re-crystallization was carried out from acetone and ethanol (1:1), yielding faint yellow crystals.

Yield 0.33g (92%), mp 285 - 291°C (decomposition); *Anal*.Calcd for C₁₈H₁₈FN₃O₄ (359.35): C, 60.16; H, 5.05; N, 11.69; found: C, 60.22; H, 5.04, N, 11.76; IR (KBr): *ν* 3282, 2996, 1724, 1683, 1621, 1581, 1530, 1436, 1385, 1322, 1184, 1083, 1027 cm⁻¹; MS(EI): *m*/*z* (% rel.int.): 359(38), 315(100), 288(7), 272(39), 257(55), 244(40), 230(39), 216(33), 203(44), 188(52), 175(74), 163(20), 147(21), 135(11), 121(9), 108(7), 95(3); HRMS: Calcd for C₁₈H₁₈FN₃O₄ 359.12810, found 359.12940; ¹H NMR (300 MHz, DMSO-d₆): δ 0.79 , 0.89(2m, 2H, overlapped with a C*H*₃ doublet), and 1.06, 1.26(2m, 2H) (H₂-2'/ H₂-3'), 0.90(d, *J* = 6.5 Hz, 3H) and 0.94(d, *J* = 6.7 Hz, 3H) [CH(C*H*₃)₂], 1.88 (m, 1H, C*H*Me₂), 3.53(dd, *J* = 8.1, 2.9 Hz, 1H, H-3), 4.50(br m, 1H, H-1'), 7.60(d, ³*J*_{H-F} = 10.5 Hz, 1H, H-6), 7.77(br s, 1H, N(4)-*H*), 8.62(s, 1H, H-9), 10.36(s, 1H, lactam N(1)-*H*), 15.11(s, 1H, CO₂H); ¹³C NMR (75 MHz, DMSO-d₆): δ 9.0, 11.3 (C-2'/ C-3'), 18.9, 19.2[CH(*C*H₃)₂], 29.5(*C*HMe₂), 39.0(C-1'), 61.2 (C-3), 105.4(d, ²*J*_{C-F} = 19.4 Hz, C-6), 107.1(C-8), 115.8(d, ³*J*_{C-F} = 6.4 Hz, C-10b), 116.9(d, ³*J*_{C-F} = 7.1 Hz, C-6a), 129.3(C-10a), 130.7(d, ²*J*_{C-F} = 18.3 Hz, C-4a), 149.3(d, ¹*J*_{C-F} = 242 Hz, C-5), 150.9 (C-9), 164.5(C-2), 166.2(*C*O₂H), 176.6(d, ⁴*J*_{C-F} = 3.2 Hz, C-7).

### Example 29

### (S)- 10-Cyclopropyl-5-fluoro-3-isobutyl-2,7-dioxo-1,2,3,4,7,10-hexahydropyrido[2,3-f]quinoxaline-8-carboxylic acid [(S)-III-4a]

This compound was prepared by reductive cyclization (using Na₂S₂O₄) of **V-4a**.

Yield 0.17 g (95%), mp 293 - 295°C (decomposition, changes color at 270 °C); [*α*]_{D} = +28.6° (DMSO, *c*∼1); *Anal*.Calcd for C₁₉H₂₀FN₃O₄ (373.38): C, 61.12; H, 5.40; N, 11.25; found C,61.31; H,5.43; N,11.38; IR (KBr): *ν* 3311, 3260, 3064, 2945, 1728, 1685, 1617, 1575, 1532, 1438, 1370, 1328, 1166, 1115 cm⁻¹; MS (TOF, ES⁺): *m* / *z* 374(M+H)⁺, HRMS calcd for C₁₉H₂₁FN₃O₄ 374.1516, found 374.1511; *m* / *z* 396 (M + Na)⁺, HRMS calcd for C₁₉H₂₀FN₃O₄Na 396.1335, found 396.1330; ¹H NMR (300 MHz, DMSO-d₆): δ 0.84 (m, 2H, H₂-2'), 1.05, 1.22(2m, 2H, H₂-3'), 0.87, 0.89(2d, *J* = 5.8 Hz, 6H, (CH₃)₂), 1.44 (m, 2H, C₃-C*H*₂), 1.67(m, 1H, C*H*Me₂), 3.92(m, 1H, H-3), 4.48(m, 1H, H-1'), 7.61(d, ³*J*_{H-F} = 10.5 Hz, 1H, H-6), 7.65(br s, 1H, N(4)-*H*), 8.64(s, 1H, H-9), 10.42(s, 1H, lactam *N*(1)-H), 15.13(s, 1H, CO₂H); ¹³C NMR (75 MHz, DMSO-d₆): δ 9.0, 11.2 (C-2'/C-3'), 22.81, 22.83[(CH₃)₂], 24.4(*C*HMe₂), 39.0(C-1'), 39.1(*C*₃-*C*H₂), 53.6 (C-3), 105.4(d, ²*J*_{C-F} = 19.5 Hz, C-6), 107.1 (C-8), 115.7(d, ³*J*_{C-F} = 6.3 Hz, C-6a), 117.0(d, ³*J*_{C-F}= 7.1 Hz, C-10b), 129.4 (C-10a), 130.8(d, ²*J*_{C-F} = 18.2 Hz, C-4a), 149.7(d, ¹*J*_{C-F} = 242 Hz, C-5), 150.9 (C-9), 165.4(C-2), 166.2(*C*O₂H), 176.6(d, ⁴*J*_{C-F} = 3.2 Hz, C-7).

### Example 30

### (S)- 3-sec-Butyl- 10-cyclopropyl-5-fluoro-2,7-dioxo-1,2,3,4,7,10-hexahydropyrido[2,3-f]quinoxaline-8-carboxylic acid [(S)-III-5a]

This compound was prepared by reductive cyclization (using Na₂S₂O₄) of **V-5a.**

Yield 0.135 g (76%), mp 290 - 291°C (decomposition); [*α*]_{D} = +77.1° (DMSO, *c*∼1); *Anal*.Calcd for C₁₉H₂₀FN₃O₄ (373.38): C, 61.12; H, 5.40; N, 11.25; found C,61.45; H,5.48; N,11.15; MS (TOF, ES⁺): *m* / *z* 374 (M + H)⁺, HRMS calcd for C₁₉H₂₁FN₃O₄ 374.1516, found 374.1511; *m* / *z* 396 (M + Na)⁺, HRMS calcd for C₁₉H₂₀FN₃O₄Na 396.1335, found 396.1330; ¹H NMR (300 MHz, DMSO-d₆): δ 0.80(t, *J* = 7.3 Hz, 3H, C*H*₃CH₂), 0.91(d, *J* = 6.7 Hz, 3H, C*H*₃CH), 0.85(m, 2H, H₂-2'), 1.07, 1.26(2m, 2H, H₂-3'), 1.12(m, 1H, C*H*_{A}Me), 1.55(m, 1H, C*H*_{B}Me), 1.65(m, 1H, C*H*Me), 3.63(m,1H, H-3), 4.50(m,1H, H-1'), 7.61(d, ³*J*_{H-F} = 10.6 Hz, 1H, H-6), 7.79(br d, *J* = 3.0 Hz, 1H, *N*(4)-H), 8.62(s, 1H, H-9), 10.38(s, 1H, lactam *N*(1)-H), 15.13(s, 1H, C₈-CO₂H); ¹³C NMR (75 MHz, DMSO-d₆): δ 9.0, 11.3 (C-2'/C-3'), 11.1 (*C*H₃CH₂), 15.2(*C*H3CH), 25.0(*C*H₂Me), 35.6(*C*HMe), 39.0(C-1'), 59.8 (C-3), 105.4(d, ²*J*_{C-F}= 19.3 Hz, C-6), 107.1 (C-8), 115.8(d, ³*J*_{C-F} = 6.4 Hz, C-10b), 116.8(d, ³*J*_{C-F} = 7.2 Hz, C-6a), 129.4 (C-10a), 130.7(d, ²*J*_{C-F} = 18.3 Hz, C-4a), 149.4(d, ¹*J*_{C-F} = 242 Hz, C-5), 150.9 (C-9), 164.3(C-2), 166.2(C₃-*C*O₂H), 176.6(d, ⁴*J*_{C-F} = 3.2 Hz, C-7).

### Example 31

### (S)- 10-Cyclopropyl-5-fluoro-3-hydroxymethyl-2,7-dioxo-1,2,3,4,7,10-hexahydropyrido[2,3-f]quinoxaline-8-carboxylic acid [(S)-III-6a]

This compound was prepared by reductive cyclization (using Na₂S₂O₄) of **V-6a**.

Yield 0.115 g (64%), mp 299 - 301 °C (decomposition, softens at 280 °C); [*α*]_{D} = +18.1° (DMSO, *c*∼1); *Anal*.Calcd for C₁₆H₁₄FN₃O₅ (347.30): C, 55.33; H, 4.06; N, 12.10; found C,55.56; H,4.21; N,12.21; MS (TOF, ES⁺): *m* / *z* 348 (M + H)⁺, HRMS calcd for C₁₆H₁₅FN₃O₅ 348.0995, found 348.0990; *m* / *z* 370 (M + Na)⁺, HRMS calcd for C₁₆H₁₄FN₃O₅Na 370.0815, found 370.0810; ¹H NMR (300 MHz, DMSO-d₆): δ 0.84, 0.97 (2m, 2H, H₂-2'), 1.02, 1.17(2m, 2H, H₂-3'), 3.64(m, 1H, C*H*_{A}-OH), 3.70(m, H, C*H*_{B}-OH), 3.98(br m, 1H, H-3), 4.48(br m, 1H, H-1'), 5.08(t, *J* = 5.3 Hz, 1H, CH₂O-*H*), 7.44(br s, 1H, N(4)-*H*), 7.60(d, *³J_{H-F}* = 10.6 Hz, 1H, H-6), 8.62(s, 1H, H-9), 10.31 (s, 1H, lactam *N*(1)-H), 15.18(s, 1H, C₃-CO₂H); ¹³C NMR (75 MHz, DMSO-d₆): δ 9.8, 11.1 (C-2'/C-3'), 38.8(C-1'), 57.6 (C-3), 62.4(CH₂OH), 105.2(d, ²*J*_{C-F} = 19.5 Hz, C-6), 107.0 (C-8), 115.5(d, ³*J*_{C-F} = 6.3 Hz, C-10b), 116.4(d, ³*J*_{C-F} = 7.0 Hz, C-6a), 128.9 (C-10a), 131.7(d, ²*J*_{C-F} = 18.0 Hz, C-4a), 149.2(d, ¹*J*_{C-F} = 242 Hz, C-5), 151.0 (C-9), 164.9(C-2), 166.3(C₈-*C*O₂H), 176.6(d, ⁴*J*_{C-F} = 3.1Hz, C-7).

### Example 32

### (S)- 10-Cyclopropyl-5-fluoro-3-(1-hydroxyethyl)-2,7-dioxo-1,2,3,4,7,10-hexahydropyrido[2,3-f]quinoxaline-8-carboxylic acid [(S)-III-7a]

This compound was prepared by reductive cyclization (using Na₂S₂O₄) of **V-7a**.

Yield 0.125 g (69 %), mp 280 - 282°C (decomposition, darkens at 240 °C); [*α*]_{D} = +27.6°(DMSO, *c*∼1); *Anal.Calcd* for C₁₇H₁₆FN₃O₅ (361.32): C, 56.51; H, 4.46; N, 11.63; found C,56.30; H,4.26; N,11.53; IR (KBr): *ν* 3396, 3336, 3200, 3072, 2962, 2885, 1716, 1685, 1617, 1532, 1447, 1379, 1319, 1132, 1089 cm⁻¹; MS (EI): *m*/*z* (% rel.int.): 361 (4, M⁺), 343(6), 317(54), 299(37), 273(82), 257(33), 244(100), 231(27), 216(50), 188(27), 175(29), 162(21), 147(11), 107(8); HRMS: calcd for C₁₇ H₁₆ F N₃ O₅ 361.10736(M⁺), found 361.10748; ¹H NMR (300 MHz, DMSO-d₆): δ 0.74, 0.89 (2m, 2H, H₂-2'), 1.05, 1.22(2m, 2H, H₂-3'), 1.13(d, *J* = 6.3 Hz, 3H, CH₃), 3.71(m, 1H, H-3), 3.87(m, 1H, CH-OH), 4.48(m, 1H, H-1'), 5.02(d, *J* = 5.3 Hz, 1H, CH-O*H*), 7.57(br s, 1H, *N*(4)-*H*), 7.60(d, *³J*_{*H*-F} *=* 10.7 Hz, 1H, H-6), 8.62(s, 1H, H-9), 10.36(s, 1H, lactam *N*(1)-H), 15.22(s, 1H, CO₂H); ¹³C NMR (75 MHz, DMSO-d₆): δ 9.4, 11.4 (C-2'/C-3'), 20.3(CH₃), 38.8(C-1'), 61.6 (C-3), 66.6(CH-OH), 105.2(d, ²*J*_{C-F} = 19.5 Hz, C-6), 107.0 (C-8), 115.8(d, ³*J*_{C-F} = 6.4 Hz, C-10b), 116.4(d, ³*J*_{C-F} = 7.1 Hz, C-6a), 128.9 (C-10a), 131.5(d, ²*J*_{C-F} = 18.0 Hz, C-4a), 149.3(d, ¹*J*_{C-F} = 241 Hz, C-5), 151.0 (C-9), 164.9(C-2), 166.3(CO₂H), 176.6(d, ⁴*J*_{C-F} = 3.3 Hz, C-7).

### Example 33

### (S)- 10-Cyclopropyl-5-fluoro-3-(2-methylsulfanylethyl)-2,7-dioxo-1,2,3,4,7,10-hexahydropyrido[2,3-I]quinoxaline-8-carboxylic acid [(S)-III-8a]

This compound was prepared by reductive cyclization (using Na₂S₂O₄) of **V-8a**.

Yield 0.16 g (88 %), mp 255- 257°C (decomposition); [*α*]_{D} = -39.2° (DMSO, *c*∼1); *Anal*.Calcd for C₁₈H₁₈FN₃O₄S (391.42): C, 55.23; H, 4.64; N, 10.74; found C,55.37; H,4.57; N,10.48; IR (KBr): *ν* 3308, 3067, 2896, 1721, 1684, 1617, 1586, 1524, 1444, 1376, 1320, 1208 cm⁻¹; MS (TOF, ES⁺): *m*/*z* 392 (M + H)⁺, HRMS calcd for C₁₈H₁₉FN₃O₄S 392.1080, found 392.1075; *m* / *z* 414 (M + Na)⁺, HRMS calcd for C₁₈H₁₈FN₃O₄S Na 414.0899, found 414.0894; ¹H NMR (300 MHz, DMSO-d₆): δ 0.86, 1.17 (2m, 2H), and 1.01(m, 2H) (H₂-2'/ H₂-3'), 1.93(m, 2H, C₃-CH₂), 2.00(s, 3H, CH₃-*S*), 2.56(m, 2H, C*H*₂-*S*), 4.02(m, 1H, H-3), 4.46(m, 1H, H-1'), 7.59(s, 1H, N(4)-*H*), 7.61(d, ³*J*_{H-F} = 10.5 Hz, 1H, H-6), 8.63(s, 1H, H-9), 10.36(s, 1H, lactam N(1)-*H*), 15.08(br s, 1H, CO₂H); ¹³C NMR (75 MHz, DMSO-d₆): δ 9.5, 10.1 (C-2'/C-3'), 15.0( CH₃-*S*), 29.4(*S*-*C*H₂), 30.1(*C*₃-CH₂), 39.0(C-1'), 54.0 (C-3), 105.5(d, ²*J*_{C-F} = 19.5 Hz, C-6), 107.2 (C-8), 115.7(d, ³*J*_{C-F} = 5.9 Hz, C-6a), 117.3(d, ³*J*_{C-F} = 6.8 Hz, C-10b), 129.4 (C-10a), 131.1 (d, ²*J*_{C-F} = 18.2 Hz, C-4a), 149.6(d, ¹*J*_{C-F} = 242.0 Hz, C-5), 151.0 (C-9), 165.3(C-2), 166.3(*C*O₂H), 176.6(d, ⁴*J*_{C-F} = 3.2Hz, C-7).

### Example 34

### (S)-3-(4-Acetylaminobutyl)-10-cyclopropyl-5-fluoro-2,7-dioxo-1,2,3,4,7,10-hexahydropyrido[2,3-f]quinoxaline-8-carboxylic acid [(S)-III-9a]

This compound was prepared by reductive cyclization (using Na₂S₂O₄) of **V-9a**.

Yield 0.125 g (58%), mp 215 - 216 °C (decomposition); MS (TOF, ES⁺): *m* / *z* 431 (M + H)⁺, HRMS calcd for C₂₁H₂₄FN₄O₅ 431.1731, found 431.1725; *m*/*z* 453(M + Na)⁺, HRMS calcd for C₂₁H₂₃FN₄O₅Na 453.1550, found 453.1545; ¹H NMR (300 MHz, DMSO-d₆): δ 0.79, 0.88(2m, 4H, H₂-2'), 0.93, 1.17(2m, 4H, H₂-3'), 1.22, 1.39(m, 2H, δ-CH₂), 1.36(m, 2H, γ-CH₂), 1.61(m, 2H, β-CH₂), 1.72(s, 3H, CH₃), 2.96(m, 2H, ε-CH₂), 3.82(m, 1H, H-3), 4.42(m, 1H, H-1'), 7.37 (br s, 1H, *N*(4)-H), 7.59(d, ³*J*_{H-F} = 10.8 Hz, 1H, H-6), 7.78 (br s, 1H, *H-N*-COMe), 8.62(s, 1H, H-9), 10.31 (br s, 1H, lactam *N*(1)-H), 14.12(br s, 1H, CO₂H).

### Example 35

### (S)- 3-Carboxymethyl-10-cyclopropyl-5-fluoro-2,7-dioxo-1,2,3,4,7,10-hexahydropyrido[2,3-f]quinoxaline-8-carboxylic acid [(S)-III-10a]

This compound was prepared by reductive cyclization (using Na₂S₂O₄) of **V-10a.**

Yield (65%), mp 293 - 295 °C (decomposition, shrinkage at 270 °C); [α]_{D} = +70.6° (DMSO, *c*~1); *Anal*.Calcd for C₁₇H₁₄FN₃O₆ (375.31): C, 54.40; H, 3.76; N, 11.20; found C,54.51; H,3.79; N,11.08; IR (KBr): ν 3216, 3068, 1728, 1684, 1623, 1524, 1443, 1394, 1295, 1158 cm⁻¹; MS (TOF, ES⁺): *m*/*z* 376 (M + H)⁺, HRMS calcd for C₁₇H₁₅FN₃O₆ 376.0945, found 376.0939; *m*/*z* 398 (M + Na)⁺, HRMS calcd for C₁₇H₁₄FN₃O₆Na 398.0764, found 398.0759; ¹H NMR (300 MHz, CDCl₃): δ 0.98, 1.07(2m, 4H, H₂-2'/ H₂-3'), 2.74(m, 3.8 Hz, 2H, α-C*H*₂-CO₂H), 4.22(m, 1H, H-3), 4.50(m, 1H, H-1'), 7.48 (br s, 1H, H-4), 7.63 (d, ³*J*_{H-F} = 10.4 Hz, 1H, H-6), 8.65 (s, 1H, H-9), 10.40 (s, 1H, H-1), 12.38 (br s, 1H, α CO₂*H*), 15.11 (s, 1H, C₈-CO₂H); ¹³C NMR (75 MHz, DMSO-d₆): δ 10.0, 10.6 (C-2'/C-3'), 35.3(α-CH₂), 39.1(C-1'), 51.7 (C-3), 105.4(d, ²*J*_{C-F} = 19.7 Hz, C-6), 107.2 (C-8), 116.0(d, ³*J*_{C-F} = 6.0 Hz, C-6a), 117.3(d, ³*J*_{C-F} = 7.2 Hz, C-10b), 129.3 (C-10a), 131.7(d, ²*J*_{C-F} = 18.0 Hz, C-4a), 149.3(d, ¹*J*_{C-F} = 243 Hz, C-5), 151.1 (C-9), 164.9(C-2), 166.2(C₈-*C*O₂H), 171.9(α-CO₂H), 176.6(d, ⁴*J*_{C-F} = 3.1Hz, C-7).

### Example 36

### (S)-3-(2-Carboxy-ethyl)-10-cyclopropyl-5-fluoro-2,7-dioxo-1,2,3,4,7,10-hexahydropyrido[2,3-f]quinoxaline-8-carboxylic acid [(S)-III-11a]

This compound was prepared by reductive cyclization (using Na₂S₂O₄) of **V-11a.**

Yield (66 %), [α]_{D} = +16.5° (5% aq. K₂CO₃, *c*~1); *Anal*. Calcd for C₁₈H₁₆FN₃O₆ (389.33): C, 55.53; H, 4.14; N, 10.79; found C,55.32; H,3.94; N,10.52; MS (TOF, ES⁺): *m*/*z* 390 (M + H)⁺, HRMS calcd for C₁₈H₁₇FN₃O₆ 390.1101, found 390.1096; *m*/*z* 412 (M + Na)⁺, HRMS calcd for C₁₈H₁₆FN₃O₆Na 412.0921, found 412.0915; ¹H NMR (300 MHz, DMSO-d₆): δ 0.86, 0.96 (2m, 2H, H₂-2'), 1.02, 1.17(2m, 2H, H₂-3'), 1.87(m, 2H, C₃-CH₂), 2.36(m, 2H, C*H*₂CO₂H), 3.94(m, 1H, H-3), 4.48(m, 1H, H-1'), 7.59(s, 1H, *N*(4)-*H*), 7.61(d, ³*J*_{H-F} = 10.8 Hz,1H, H-6), 8.64(s, 1H, H-9), 10.40(s, 1H, lactam *N*(1)-*H*), 12.16(br s, 1H, CH₂-CO₂*H*), 15.09(br s, 1H, C₈-CO₂H); ¹³C NMR (75 MHz, DMSO-d₆): δ 9.5, 10.8 (C-2'/ C-3'), 25.5(C₃-*C*H₂), 29.7(*C*H₂-CO₂H), 39.0(C-1'), 54.2 (C-3), 105.4(d, ²*J*_{C-F} = 19.4 Hz, C-6), 107.2 (C-8), 115.7(d, ³*J*_{C-F} = 6.2 Hz, C-6a), 117.2(d, ³*J*_{C-F} = 7.1 Hz, C-10b), 129.4 (C-10a), 131.0(d, ²*J*_{C-F} = 18.3 Hz, C-4a), 149.6(d, ¹*J*_{C-F} = 242 Hz, C-5), 151.0 (C-9), 165.2(C-2) , 166.2(C₈-*C*O₂H), 174.3(CH₂-*C*O₂H), 176.6(d, ⁴*J*_{C-F} = 3.1Hz, C-7).

### Example 37

### (S)-3-Benzyl-10-cyclopropyl-5-fluoro-2,7-dioxo-1,2,3,4,7,10-hexahydropyrido[2,3-f]quinoxaline-8-carboxylic acid [(S)-III-12a]

To a stirred solution of **V-12a** (0.91 g, 2.0 mmol) and potassium carbonate (1.9 g, 7.0 mmol) in water (40 ml), was added portionwise a solution of sodium dithionite (2.26 g, 13.0 mmol) in water (10 ml). The reaction mixture was further stirred at rt for 10 min and worked up as described for **III-1** a above. The title product was recrystallized from methanol, to give off-white crystals.

Yield (68 %), mp 262 - 264 °C (decomposition / the crystals change color at 255 °C); *Anal*.Calcd for C₂₂H₁₈FN₃O₄ (407.39): C, 64.86; H, 4.45; N, 10.31; found C,64.66; H,4.26; N,10.12; MS(EI): *m*/*z* (% rel.int.): 407 (22, M⁺), 365(2), 363(8), 316(100), 272(33), 257(24), 244(6), 231(6), 202(5), 188(8), 175(5), 162(3), 92(2), 91(16); HRMS: Calcd for C₂₂H₁₈FN₃O₄ 407.12810, found 407.12769; ¹H NMR (300 MHz, DMSO-d₆): δ 0.88, 0.94(2m, 2H) and 1.04, 1.22(2m, 2H) (H₂-2', H₂-3), 2.95(m, 2H, C*H*₂Ph), 4.19(br m, 1H, H-3), 4.50(br m, 1H, H-1'), 7.20(m, 5H, H-4", H-3"/ H-5", H-2"/ H-6"), 7.53(br s, 1H, N(4)-H), 7.64(d, *³J*_{*H-*F} = 10.4 Hz, H-6), 8.64(s, 1H, H-9), 10.36(s, 1H, lactam N(1)-H), 15.13(s, 1H, CO₂H); ¹³C NMR (75 MHz, DMSO-d₆): δ 9.5, 11.1(C-2'/ C-3'), 36.7(*C*H₂Ph), 39.0(C-1'), 56.5(C-3), 105.5(d, ²*J*_{C-F} = 19.4 Hz, C-6), 107.1(C-8), 115.6(d, ³*J*_{C-F} = 6 Hz, C-6a), 117.1(d, ³*J*_{C-F} = 7.1 Hz, C-10b), 127.1(C-4"), 128.8(C-2"/ C-6"), 129.2(C-10a), 130.0(C-3"/ C-5"), 130.7(d, ²*J*_{C-F}= 18.2 Hz, C-4a), 137.0(C-1"), 149.5(d, ¹*J*_{C-F} = 250 Hz, C-5), 151.0(C-9), 164.9(C-2), 166.2(CO₂H), 176.6(d, ⁴*J*_{C-F} = 2.9 Hz, C-7).

### Example 38

### (S)-10-Cyclopropyl-5-fluoro-2,7-dioxo-3-phenyl-1,2,3,4,7,10-hexahydropyrido[2,3-f]quinoxaline-8-carboxylic acid [(S)-III-13a]

To a stirred solution of **V-13a** (0.22 g, 0.50 mmol) and potassium carbonate (0.48 g, 3.5 mmol) in water (10 ml), was added portionwise a solution of sodium dithionite (0.61 g, 3.5 mmol) in water (3 ml). The reaction mixture was further stirred at rt for 10 min and worked up as described for **V-13a** above. The title product was recrystallized from isopropanol, to give off-white crystals.

Yield 0.13 g (66 %), mp 000 - 000 °C (decomposition); *Anal*.Calcd for C₂₁H₁₆FN₃O₄ (393.37): C, 64.21; H, 4.10; N, 10.68; found C,64.02; H,4.15; N,10.54; ¹H NMR (300 MHz, DMSO-d₆): δ 0.90, 1.08(2m, 4H, H₂-2'/ H₂-3'), 4.38(m, 1H, H-1'), 5.17(br s, 1H, H-3), 7.31(br m, 5H, H-2"/ H-6", H-3"/ H-5", H-4"), 7.67(d, *³J*_{*H*-F} = 10.3 Hz, H-6), 8.34(br s, 1H, N(4)-H), 8.58(s, 1H, H-9), 10.59(br s, 1H, lactam N(1)-H), 15.08(br s, 1H, CO₂H); ¹³C NMR (75 MHz, DMSO-d₆): δ 8.6, 11.0 (C-2'/ C-3'), 38.8(C-1'), 58.0 (C-3), 105.6(d, ²*J*_{C-F} = 18.8 Hz, C-6), 107.3 (C-8), 115.5(d, ³*J*_{C-F} = 6.2 Hz, C-10b), 117.5(d, ³*J*_{C-F} = 7.0 Hz, C-6a), 126.2 (C-2"/ C-6"), 128.5 (C-4'), 129.1 (C-3"/ C-5"), 129.3(C-10a), 131.1(d, ²*J*_{C-F} = 18.2 Hz, **C-4a**), 137.6 (C-1"), 149.4(d, ¹*J*_{C-F} = 244 Hz, C-5), 151.1 (C-9), 164.0 (C-2), 166.2(CO₂H), 176.6(d, ⁴*J*_{C-F} = 3 Hz, C-7).

### Example 39

### (S)-10-Cyclopropyl-3-ethyl-5-fluoro-2,7-dioxo-1,2,3,4,7,10-hexahydropyrido [2,3-f]quinoxaline-8-carboxylic acid [(S)-III-14a]

This compound was prepared by reductive cyclization (using Na₂S₂O₄) of **V-14a.** Yield 0.14 g (91%), mp 270- 272 °C (decomposition, changes color at 260 °C); IR (KBr): *v* 3311, 3072, 2970, 2868, 1728, 1685, 1617, 1532, 1438, 1379, 1310, 1081 cm⁻¹; MS (TOF, ES⁺): *m*/*z* 346 (M + H)⁺, HRMS calcd for C₁₇H₁₇FN₃O₄ 346.1203, found 346.1198; *m*/*z* 368 (M + Na)⁺, HRMS calcd for C₁₇H₁₆FN₃O₄Na 368.1022, found 368.1017; ¹H NMR (300 MHz, DMSO-d₆): δ 0.91 (t, , *J=* 7.2 Hz, 3H, CH₃) 0.85, 0.96(2m, 2H, H₂-2'), 1.02, 1.19(2m, 2H, H₂-3'), 1.65(m, 2H, CH₂Me), 3.82(m, 1H, H-3), 4.46(m, 1H, H-1'), 7.52(br s, 1H, *N*(4)-*H*), 7.55(d, ³*J*_{H-F} = 10.2 Hz, 1H, H-6), 8.61(s,1H, H-9), 10.28(s, 1H, lactam *N*(1)-*H*), 15.08(br s, 1H, CO₂H); ¹³C NMR (75 MHz, DMSO-d₆): δ 9.4, 10.9 (C-2'/C-3'), 10.0 (CH₃), 24.0(C₃-*C*H₂), 39.0(C-1'), 56.2 (C-3), 105.3(d, ²*J*_{C-F} = 19.7 Hz, C-6), 107.1 (C-8), 115.5(d, ³*J*_{C-F} = 5.4 Hz, C-10b), 116.9(d, ³*J*_{C-F} = 7.1 Hz, C-6a), 129.2 (C-10a), 131.2(d, ²*J*_{C-F} = 18.3 Hz, C-4a), 149.5(d, ¹*J*_{C-F} = 242.2 Hz, C-5), 150.9 (C-9), 165.6(C₈-*C*O₂H), 166.3(C-2), 176.6(d, ⁴*J*_{C-F} = 3.2 Hz, C-7).

### Example 40

### [(S)-IIIB-3a] 1-Aminocyclopentane acid cyclic derivative

This compound was prepared by reductive cyclization (using Na₂S₂O₄) of **VB-1**.

Yield 0.15 g (83%), mp > 300 °C (darkens at 290 °C); *Anal*.Calcd for C₁₉H₁₈FN₃O₄ (371.36): C, 61.45; H, 4.89; N, 11.32; found C,61.71; H,4.77; N,11.10; IR (KBr): ν 3302, 3200, 3064, 2945, 1719, 1677, 1617, 1574, 1515, 1447, 1370, 1328, 1192 cm

MS (EI): *m*/*z* (% rel.int.): 371 (49, M⁺), 327(100), 298(18), 270(56), 242(11), 229(12), 215(9), 201(10), 188(23), 160(9), 147(8), 134(9); HRMS: calcd for C₁₉ H₁₈ FN₃O₄ 371.12810 (M⁺), found 371.12769; ¹HNMR (300 MHz, 3% NaOD in D₂O): δ 0.30, 0.74(2m, 4H, H₂-2'/ H₂-3'), 1.34, 1.74(2m, 4H, H₂-2"/ H₂-5"), 1.43(m, 4H, H₂-3"/ H₂-4"), 4.15(m, 1H, H-1'), 7.17(d, ³*J*_{H-F} = 11.1 Hz, H-6), 8.30(s, 1H, H-9); ¹³C NMR (75 MHz, 3% -NaOD in D₂O): δ 9.2(C-2', C-3'), 24.2(C-3"/ C-4"), 35.1(C-2"/ C-5"), 40.2 C-1'), 62.4(C-3), 101.7(d, ²*J*_{C-F} = 20.3 Hz, C-6), 115.2(C-8), 120.1(d, ³*J*_{C-F} = 6.9 Hz, C-6a), 128.9(d, ³*J*_{C-F} = 3.6 Hz, C-10b), 129.4(d, ²*J*_{C-F} = 15.7 Hz, C-4a), 132.0(C-10a), 148.8(C-9), 149.9(d, ¹*J*_{C-F} = 240 Hz, C-5), 172.4(C-2), 173.2(CO₂⁻), 175.9(d, ⁴*J*_{C-F} = 3.4 Hz, C-7).

### Example 41

### (S)-7-(1-Carboxyethylamino)-1-cyclopropyl-8-fluoro-6-nitro-4-oxo-1,4-dihydroquinoline-3-carboxylic acid [(S)-VI-2a]

This compound is prepared from interaction of **VIIA** with (*S*)-alanine

Yield (65 %); *Anal*.Calcd for C₁₆H₁₄FN₃O₇ (379.30): C, 50.67; H, 3.72; N, 11.08; found C,50.53; H,3.61; N,10.98; MS(EI): *m*/*z* (% rel. int.): 379(2), 363(2), 351(3), 343(6), 335(49), 334(16), 308(12), 290(19), 279(18), 263(87), 256(16), 242(28), 217(27), 216(20), 189(17), 148(12), 107(6), 44(100); HRMS: Calcd for C₁₆H₁₄FN₃O₇ 379.08153, found 379.08183; ¹H NMR (300 MHz, 3% NaOD in D₂O): δ 0.77, 0.95(2m, 4H, H₂-2'/H₂-3'), 1.26(d, *J* = 6.6 Hz, 3H, CH₃), 3.52(br m, 1H, H-1'), 4.14( overlapped dq, *J*_{H-F} = 8.1 Hz, 1H, C*H*Me), 8.21(s, 1H, H-2), 8.28(s, 1H, H-5). ¹³C NMR (75 MHz, 3% NaOD in D₂O): δ 8.1(d, *J*_{C-F} = 8.4 Hz, C-2'), 8.3(d, *J*_{C-F} = 8.4 Hz, C-3'), 19.6(d, *J_{C-F}* = 3.1 Hz, CH₃), 38.9(d, *J*_{C-F} = 13.1 Hz, C-1'), 56.0(d,*J*_{C-F} = 13.3 Hz, α-*C*HCH₃₎, 116.1(C-3), 116.6(C-4a), 121.3(d, ⁴*J*_{C-F} = 1.7 Hz, C-5), 131.5(d, ³*J*_{C-F} = 6.4 Hz, C-6), 134.3(d, ²*J*_{C-F} = 5.7 Hz, C-8a), 136.0(d, ²*J*_{C-F} = 12.8 Hz, C-7), 139.4(d, ¹*J*_{C-F} = 246 Hz, C-8), 151.3(C-2), 171.0(C₃-*C*O₂⁻), 175.2(d, ⁴*J*_{C-F} = 1.6 Hz, C-4), 179.8(d, *J*_{C-F} = 1.4 Hz, CH-*C*O₂⁻).

### Example 42

### 6-Cyclopropyl-5-fluoro-2,9-dioxo-1,2,3,4,6,9-hexahydro-pyrido[2,3-g]quinoxaline-8-carboxylic acid [IV-1a]

This compound was prepared by reductive cyclocondensation (using Na₂S₂O₄) of **VI-1a**.

Yield (72%); *Anal.*Calcd for C₁₅H₁₂FN₃O₄ (317.27): C, 56.78; H, 3.81; N, 13.24; found C,56.52; H,3.75; N,13.01; MS(EI): *m*/*z* (% rel. int.): 317(28), 287(5), 273(100), 252(17), 244(31), 224(10), 216(11), 204(12), 187(6), 175(20), 139(6), 135(6), 97(11), 83(14); HRMS: Calcd for C₁₅H₁₂FN₃O₄ 317.08115, found 317.08034; ¹H NMR (300 MHz, 3% NaOD in D₂O): δ 0.65(m, 2H, H₂-2'), 0.80(m, 2H, H₂-3'), 3.32(br m, 1H, H-1'), 3.57(br s, 2H, H₂-3), 6.88(s, 1H, H-10), 7.96 (s, 1H, H-7); ¹³C NMR (75 MHz, 3% NaOD in D₂O): δ 7.8, 7.9(C-2'/ C-3'), 38.4(d, *J*_{C-F} = 11.6 Hz, C-1'), 43.2(C-3), 110.0(C-10), 114.6(C-8), 118.6 (C-9a), 126.2(d, ²*J*_{C-F} = 6.0 Hz, C-5a), 129.6(d, ²*J*_{C-F} = 12.5 Hz, C-4a), 134.9(d, ³*J*_{C-F} = 1.8 Hz, C-10a), 137.3(d, ¹*J*_{C-F} = 239 Hz, C-5), 146.3(C-7), 171.5(C-2), 172.9(CO₂⁻), 175.0(d, ⁴*J*_{C-F} = 1.6 Hz, C-9).

### Example 43

### 6-Cyclopropyl-5-fluoro-3-methyl-2,9-dioxo-1,2,3,4,6,9-hexahydro-pyrido[2,3-g]quinoxaline-8-carboxylic acid [IV-2a]

This compound was prepared by reductive cyclocondensation (using Na₂S₂O₄) of **VI-2a**.

Yield (65%); *Anal*.Calcd for C₁₆H₁₄FN₃O₄ (331.30): C, 58.01; H, 4.26; N, 12.68; found C,57.92; H,4.15; N,12.43; MS(EI): *m*/*z* (% rel. int.): 331(24), 313(2), 287(100), 272(27), 252(17), 244(34), 216(10), 203(14), 188(6), 175(14), 147(6), 135(4), 122(6), 114(8), 107(6), 91(6), 83(4) ; HRMS: Calcd for C₁₆H₁₄FN₃O₄ 331.09680, found 331.09696; ¹H NMR (300 MHz, 3% NaOD in D₂O): δ 0.62, 0.72(2m, 2H, H₂-2'), 0.82(m, 2H, H₂-3'), 1.06(d, *J* = 6.6 Hz, 3H, CH₃), 3.36(m, 1H, H-1'), 3.73(q, *J =* 6.6 Hz, 1H, H-3), 6.98(s, 1H, H-10), 7.99 (s, 1H, H-7); ¹³C NMR (75 MHz, 3% NaOD in D₂O): δ 7.8(d, *J*_{C-F} = 8.4 Hz, C-2'), 7.9(d, *J*_{C-F} = 9.2 Hz, C-3'), 19.8(CH₃), 38.5(d, *J*_{C-F} = 11.7 Hz, C-1'), 49.4(C-3), 109.8(C-10), 114.7(C-8), 119.1 (C-9a), 126.2(d, ²*J*_{C-F} = 5.0 Hz, C-5a), 129.4(d, ²*J*_{C-F} = 13.1 Hz, C-4a), 135.1(d, ³*J*_{C-F} = 3.2 Hz, C-10a), 137.8(d, *J*_{C-F} = 239 Hz, C-5), 146.4(C-7), 172.9(CO₂⁻), 175.1(d, ⁴*J*_{C-F} = 2.3 Hz, C-9), 175.3(C-2).

### Example 44

### (S)- 6-Cyclopropyl-5-fluoro-3-(2-methylsulfanylethyl)-2,9-dioxo-1,2,3,4,6,9-hexahydropyrido[2,3-g]quinoxaline-8-carboxylic acid [(S)-IV-8a]

This compound was prepared by reductive cyclocondensation (using Na₂S₂O₄) of **VI-8a.**

Yield (72 %); *Anal.Calcd* for C₁₈H₁₈FN₃O₄S (391.42): C, 55.23; H, 4.64; N, 10.74; found C,55.02; H,4.35; N,10.52; MS(EI): *m*/*z* (% rel. int.): 391(100), 357(1), 347(67), 343(18), 330(9), 318(11), 299(56), 286(40), 272(33), 244(12), 231(10), 203(12), 174(9), 148(5); HRMS: Calcd for C₁₈H₁₈FN₃O₄S 391.10017, found 391.10180;

¹H NMR (300 MHz, DMSO-d₆): δ 1.12 (m, 4H, H₂-2'/ H₂-3'), 1.98 (s, 3H, *S*-CH₃), 2.02 (m, 2H, C₃-C*H*₂), 2.53 (m, 2H, *S*-CH₂), 4.01 (m, 1H, H-1'), 4.17 (m, 1H, H-3), 7.22 (br s, 1H, *N*₄-H), 7.36 (s, 1H, H-10), 8.42 (s, 1H, H-7), 10.87 (s, 1H, *N*₁-H), 15.35 (br s, 1H, CO₂H); ¹³C NMR (75 MHz, DMSO-d₆): δ 8.9 (d, *J*_{C-F} = 6.8 Hz, C-2'/C-3'), 15.0 (*S*-CH₃), 29.1 (CH₂- S), 32.9 (C₃-*C*H₂), 40.5 (C-1'), 54.1 (C-3), 105.0 (C-10), 106.2 (C-8), 116.4 (C-9a), 126.9 (d, ²*J*_{C-F} = 6.6 Hz, C-5a), 127.6 (d, ³*J*_{C-F} = 4.5 Hz, C-10a), 129.7 (d, ²*J*_{C-F} = 16.1 Hz, C-4a), 138.2 (d, ¹*J*_{C-F} = 241 Hz, C-5), 148.2 (C-7), 166.7 (CO₂H), 166.8 (C-2), 176.2 (d, ⁴*J*_{C-F} = 2.6 Hz, C-9).

### Example 45

### (S)-3-Benzyl-6-cyclopropyl-5-fluoro-2,9-dioxo-1,2,3,4,6,9-hexahydro-pyrido[2,3-g]quinoxaline-8-carboxylic acid [(S)-IV-12a]

This compound was prepared by reductive cyclocondensation (using Na₂S₂O₄) of **VI-12a.**

Yield (68%); *Anal*.Calcd for C₂₂H₁₈FN₃O₄ (407.39): C, 64.86; H, 4.45; N, 10.31; found C,64.58; H,4.32; N,10.24; MS(EI): *m*/*z* (% rel. int.): 407(20), 363(27), 344(20), 316(74), 272(100), 254(28), 231(11), 203(11), 175(5), 148(2), 92(3), 91(28); HRMS: Calcd for C₂₂H₁₈FN₃O₄ 407.12810, found 407.12839; ¹H NMR (300 MHz, 3% NaOD in D₂O): δ 0.84, 0.98(2m, 4H, H₂-2'/ H₂-3'), 2.59, 2.75(2m, 2H, diastereotopic C*H*₂Ph), 3.64(m, 1H, H-1'), 3.92(m, 1H, H-3), 6.96(m, 3H, H-3"/ H-5", H-4"), 7.02(m, 2H, H-2"/ H-6"), 7.06(s, 1H, H-10), 8.16(s, 1H, H-7); ¹³C NMR (75 MHz, 3% -NaOD in D₂O): δ 8.0(d, *J*_{C-F} = 8.8 Hz, C-2'), 8.1(d, *J*_{C-F} = 8.8 Hz, C-3'), 38.5(d, *J*_{C-F} = 11.7 Hz, C-1'), 39.4(*C*H₂Ph), 55.3(C-3), 109.8(C-10), 115.4(C-8), 119.6(C-9a), 126.4(d, ²*J*_{C-F} = 5.8 Hz, C-5a), 126.6(C-4"), 128.2(C-2"/ C-6"), 129.5(d, ²*J*_{C-F} = 12.4 Hz, C-4a), 129.7(C-3"/ C-5"), 135.7(C-10a), 136.9(C-1"), 137.9(d, ¹*J*_{C-F} = 239 Hz, C-5), 146.3(C-7), 173.2(C-2), 174.1(CO₂⁻), 175.2(C-9).

### Example 46

### (S)-6-Cyclopropyl-5-fluoro-2,9-dioxo-3-phenyl-1,2,3,4,6,9-hexahydropyrido[2,3-g]quinoxaline-8-carboxylic acid [(S)-IV-13a]

This compound was prepared by reductive cyclocondensation (using Na₂S₂O₄) of **VI-13a.**

Yield (45 %); *Anal*.Calcd for C₂₁H₁₆FN₃O₄ (393.37): C, 64.12; H, 4.10; N, 10.68; found C,63.88; H,3.96; N,10.42; ¹H NMR (300 MHz, 3% NaOD in D₂O): δ 0.42, 0.75(2m, 4H, H₂-2'/ H₂-3'), 3.13(m, 1H, H-1'), 4.54(m, 1H, H-3), 6.77(m, 3H, H-3"/ H-5", H-4"), 6.84(H-2"/ H-6"), 7.03(s, 1H, H-10), 7.89(s, 1H, H-7); ¹³C NMR (75 MHz, 3% -NaOD in D₂O): δ 7.8(d, *J*_{C-F} = 7.2 Hz, C-2'), 7.9(d, *J*_{C-F} = 7.7 Hz, C-3'), 38.4(d, *J*_{C-F} = 11.3 Hz, C-1'), 57.8(C-3), 110.3(C-10), 114.8(C-8), 119.1 (C-9a), 126.3(d, ²*J*_{C-F} = 5.0 Hz, C-5a), 126.5(C-2"/ C-6"), 127.8(C-4"), 128.5(d, ²*J*_{C-F} = 12.6 Hz, C-4a), 128.6(C-3"/ C-5"), 134.9(C-10a), 137.4(d, ¹*J*_{C-F} = 240 Hz, C-5), 141.5(C-1"), 146.5(C-7), 171.9(C-2), 172.9(CO₂⁻), 175.2(d, ⁴*J*_{C-F} = 2.4 Hz, C-9).

### Example 47

### (S)-6-Cyclopropyl-3-ethyl-5-fluoro-2,9-dioxo-1,2,3,4,6,9-hexahydropyrido [2,3-g]quinoxaline-8-carboxylic acid [(S)-IV-14a]

This compound was prepared by reductive cyclocondensation (using Na₂S₂O₄) of **VI-14a.**

Yield (63 %); *Anal*.Calcd for C₁₇H₁₆FN₃O₄ (345.33): C, 59.13; H, 4.67; N, 12.17; found C,59.20; H,4.72; N,12.03; MS(EI): *m*/*z* (% rel. int.): 345(30), 316(4), 301(100), 272(45), 252(14), 244(18), 233(15), 216(6), 203(17), 187(5), 175(14), 164(4), 148(5), 99(3); HRMS: Calcd for C₁₇H₁₆FN₃O₄ 345.11245, found 345.11190; ¹H NMR (300 MHz, DMSO-d₆): δ 0.85 (t, *J* = 7.2 Hz, 3H, CH₃), 1.14 (m, 4H, H₂-2'/H₂-3'), 1.77 (m, 2H, C₃-C*H*₂), 4.06 (m, 1H, H-1'), 4.10 (m, 1H, H-3), 7.27 (br s, 1H, *N*(4)-H), 7.39 (s, 1H, H-10), 8.42 (s, 1H, H-7), 10.91 (s, 1H, *N*-H), 15.38 (br s, 1H, CO₂H); ¹³C NMR (75 MHz, DMSO-d₆): δ 8.9 (d, *J*_{C-F} = 7.7 Hz, C-2'/ C-3'), 9.1 (CH₃), 26.8 (C₃-*C*H₂), 40.4 (C-1'), 55.9 (C-3), 104.8 (C-10), 106.2 (C-8), 116.1 (C-9a), 126.9 (d, ²*J*_{C-F} = 6.4 Hz, C-5a), 127.6 (d, ³*J*_{C-F} = 4.2 Hz, C-10a), 130.0(d, ²*J*_{C-F} = 16.1 Hz, C-4a), 137.9 (d, ¹*J*_{C-F} = 240 Hz, C-5), 148.1 (C-7), 165.5 (CO₂H), 166.8 (C-2), 176.2 (d, ⁴*J*_{C-F} = 2.4 Hz, C-9).

Other compounds were prepared by methods analogous to these methods using analogous starting materials and reagents.

### Example 48

### Antibacterial Data

The *MICs* were determined by the conventional broth dilution method using two serial dilution technique. The standardization of bacterial test suspension was carried out according to *Mcfarland standard method* as described by the National Committee for Clinical Laboratories Standard (NCCLS) (1993). Stock solutions of the test compounds were prepared using *DMSO* to obtain concentration of 200 µg/cm³. Serial dilutions were prepared to obtain test concentrations ranging from 100 µg/cm³ - 0.098 µg/cm³. Each tube was then inoculated with 0.1 cm³ of the cultured bacteria (containing approximately 1 to 2X10⁸ CFU / cm³), mixed and incubated at 37 °C for 24 h. Growth inhibition with concentrations at 10 µg / cm³ or lower were carried out in duplicates. All test tubes showing positive/negative growth were confirmed by the agar plate method. The results were recorded according to presence and absence of growth. The *MICs* were calculated as the average concentration of the test agent in the broth tubes showing consecutive positive and negative growth. The *in vitro* antibacterial activity of the model compounds are given in Table 1:

**Table 1. In vitro antibacterial activity (MIC values, µg/cm³) of model compounds of the formulae III, V, IX and XI as compared with the reference ciprofloxacin.**

| **Compound number** | **S. *aureus* ATCC6538a** **MIC (µg / ml)** | **E. *coli* ATCC 8739** **MIC (µg / ml)** |
|---|---|---|
| **V-2a** | 9.5 | 19.5 |
| **V-3a** | 0.439 | 22.5 |
| **V-4a** | 10.7 | 10.7 |
| **V-5a** | 39.5 | 19.7 |
| **V-6a** | 9.37 | 18.75 |
| **V-7a** | 19.68 | 19.68 |
| **V-8a** | 21.56 | 21.56 |
| **V-9a** | 11.25 | 1.4 |
| **V-11a** | 10.3 | 20.6 |
| **V-14a** | 24.37 | 24.37 |
| **XI-1a** / Sarcosine open | 12 | 12 |
| **VB-3a** | 19.95 | 9.95 |
| **III-2a** | 5.8 | 11.5 |
| **III-4a** | 9.84 | 19.68 |
| **III-8a** | 11.25 | 22.5 |
| **III-9a** | 52.5 | 26.25 |
| **III-10a** | 18.75 | 18.75 |
| **III-14a** | 20.6 | 10.3 |
| **IX-1a** / Sarcosine cyclized | 10.5 | 0.65 |
| **IIIB-3a** | 23.4 | 23.4 |
| Reference / **Ciprofloxacin** | 1.23 | 0.31 |

## Claims

1. A compound of the general formula 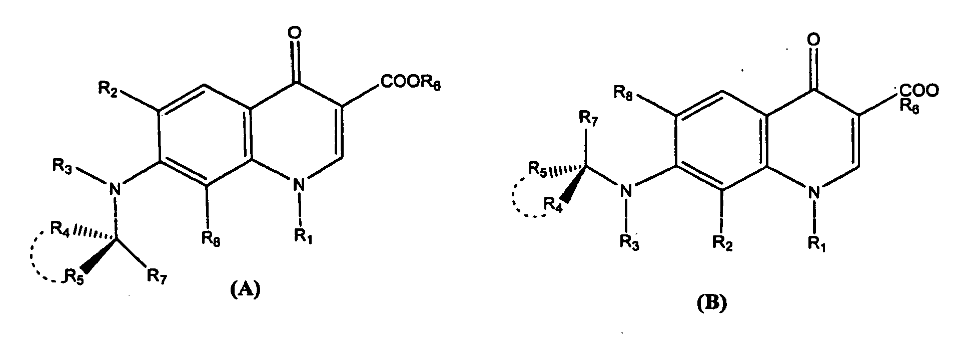 or a physiologically tolerated salt or ester thereof,
in which
R₁ represents an optionally substituted C₁₋₁₂ alkyl, C₂₋₁₂ alkenyl, C₃₋₁₂ cycloalkyl,
C₁-₁₂ alkoxy, amino, C₁₋₁₂ alkylamino, di-C₁₋₁₂ alkylamino, C₆₋₂₄ aryl orC₇₋₃₀ aralkyl group; R₂ represents a halogen atom, a hydroxyl group or an optionally substituted C₁₋₁₂ alkoxy group;
R₃ represents a hydrogen atom or an optionally substituted C₁₋₁₂ alkyl group;
R₄ and R₅ each independently represent a hydrogen atom or an optionally substituted C₁₋₁₂ alkyl, C₆₋₂₄ aryl, C₇₋₃₀ aralkyl, 3- to 18-membered heterocyclyl or 3- to 18-membered heterocyclyl-C₁₋₆ alkyl group, or
R₄ and R₅ together represent an optionally substituted alkylene group;
R₆ represents a hydrogen atom or an optionally substituted C₁₋₁₂ alkyl group;
R₇ represents a group -COOR₆, where R₆ is as defined above, and
R₈ represents a nitro group, or R₇ and R₈ together represent a group -C(O)-NR₉-, where R₉ is a hydrogen atom or an optionally substituted C₁₋₁₂ alkyl group;
optional substituents being selected from halogen atoms, nitro, cyano, hydroxyl, C₃₋₈ cycloalkyl, C₁₋₁₂ alkyl, C₁₋₁₂ haloalkyl, C₁₋₁₂ alkoxy, C₁₋₁₂ haloalkoxy, amino, C₁₋₁₂ alkylamino, di-(C₁₋₁₂ alkyl)amino, formyl, C₁₋₁₂ alkoxycarbonyl, carboxyl; C₁₋₁₂ alkanoyl, C₁₋₁₂ alkylthio, C₁₋₁₂ alkylsulphinyl, C₁₋₁₂ alkylsulphonyl, C₁₋₁₂ alkylsulphonato, carbamoyl, C₁₋₁₂ alkylamido, C₆₋₁₀ aryl and C₇₋₁₁ aralkyl groups.

2. A compound according to claim 1 in which R₁ represents a C₁₋₆ alkyl, C₂₋₆ alkenyl, C₃₋₆ cycloalkyl, C₁₋₆ alkoxy, C₁₋₆ alkylamino or C₆₋₁₄ aryl group, each group being optionally substituted by one or more substituents selected from the group consisting of halogen atoms, nitro, cyano, hydroxyl, C₁₋₄ alkyl, C₁₋₄ haloalkyl, C₁₋₄ alkoxy and C₁₋₄ haloalkoxy groups.

3. A compound according to claim 1 or claim 2 in which R₂ represents a halogen atom, a hydroxyl group or a C₁₋₄ alkoxy group.

4. A compound according to any one of the preceding claims in which R₃ represents a hydrogen atom or a C₁₋₄ alkyl group.

5. A compound according to any one of the preceding claims in which R₄ and R₅ each independently represent a hydrogen atom or a C₁₋₆ alkyl, C₆₋₁₄ aryl, C₇₋₁₈ aralkyl, 3- to 10-membered heterocyclyl or 3- to 10-membered heterocyclyl-C₁₋₆ alkyl group, each group being optionally substituted by one or more substituents selected from the group consisting of halogen atoms, hydroxyl, C₁₋₄ haloalkyl, C₁₋₄ alkoxy, C₁₋₄ haloalkoxy, C₁₋₄ alkylthio, C₁₋₄ haloalkylthio, carboxyl, amino, carbamoyl, C₁₋₄ alkylamido and -NH-CO-R' groups, where R' is a hydrogen, C₁₋₄ alkyl or phenyl group, or R₄ and R₅ together represent a group -(CH₂)ₙ- where n is an integer from 2 to 5.

6. A compound according to any one of the preceding claims in which R₆ represents a hydrogen atom or a C₁₋₄ alkyl group.

7. A compound according to any one of the preceding claims in which R₇ represents a group -COOR₆, where R₆ is a hydrogen atom or a C₁₋₄ alkyl group, and R₈ represents a nitro group, or R₇ and R₈ together represent a group -C(O)-NH-.

8. A compound according to any one of the preceding claim, in which the compound is of the formula I or II, 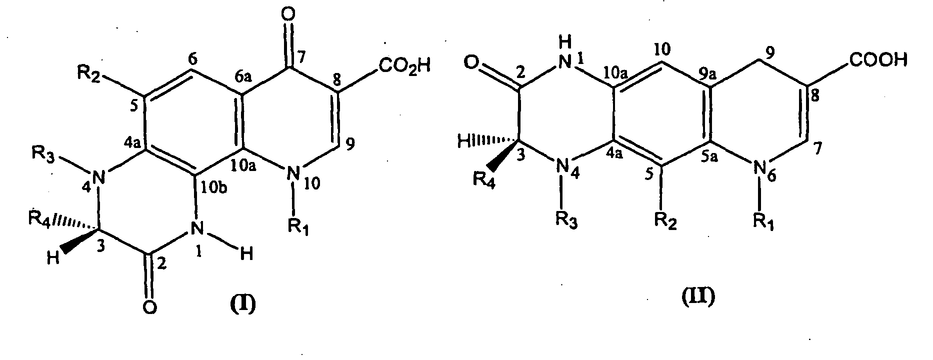 or a physiologically tolerated salt or ester thereof, in which
R₁ = *c*-C₃H₅, Et, 2,4-(F)₂C₆H₃;
R₂ = F, Cl, OH or OMe;
R₃ = H, Me;
R₄ = H, C₁-C₄-alkyl which is optionally substituted by hydroxyl, C₁-C₄-alkoxy or
C₁-C₄-alkylthio, -CH₂CO₂H, -CH₂CONH₂, -CH₂CH₂CO₂H, -CH₂CH₂CONH₂,
-(CH₂)₄NHCOR' [R' = H, Me, Ph], Ph, *p*-(X)C₆H₄ [X = F, Cl, OH, OMe], PhCH₂, *p*-(OH)C₆H₄CH₂-, 2,4-(OH)₂C₆H₃CH₂-, 3-(indolyl)CH₂ or 4-(imidazolyl)CH₂-, where Me is methyl and Ph is phenyl.

9. A compound according to any one of claims 1 to 7, in which the compound is of the formulae IA or IIA, 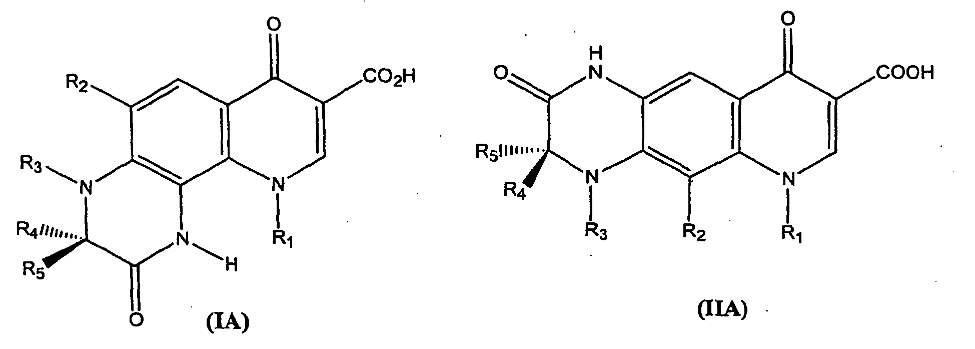 or a physiologically tolerated salt or ester thereof, in which
R₁ = *c*-C₃H₅, Et, 2,4-(F)₂C₆H₃;
R₂ = F, Cl, OH or OMe;
R₃ = H, Me;
R₄ = R₅ = Me, Ph, 2-Py, 2-thienyl or 2-furyl, where Me is methyl, Ph is phenyl and Py is pyridyl.

10. A compound according to any one of claims 1 to 7, in which the compound is of the formulae IB or IIB: 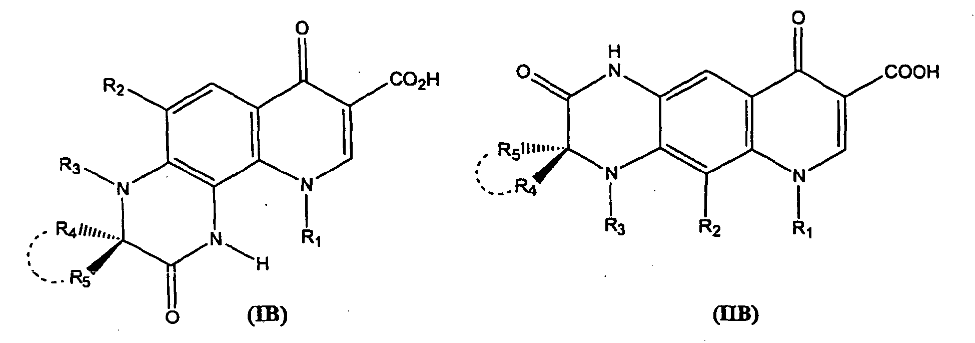 or a physiologically tolerated salt or ester thereof, in which
R₁ = *c*-C₃H₅, Et, 2,4-(F)₂C₆H₃;
R₂ = F, Cl, OH or OMe;
R₃ = H, Me;
R₄ and R₅ = [CH₂]ₙ, where n = 2, 3, 4 or 5.

11. A compound according to any one of claims 1 to 7, in which the compound is of the formulae III or IV: 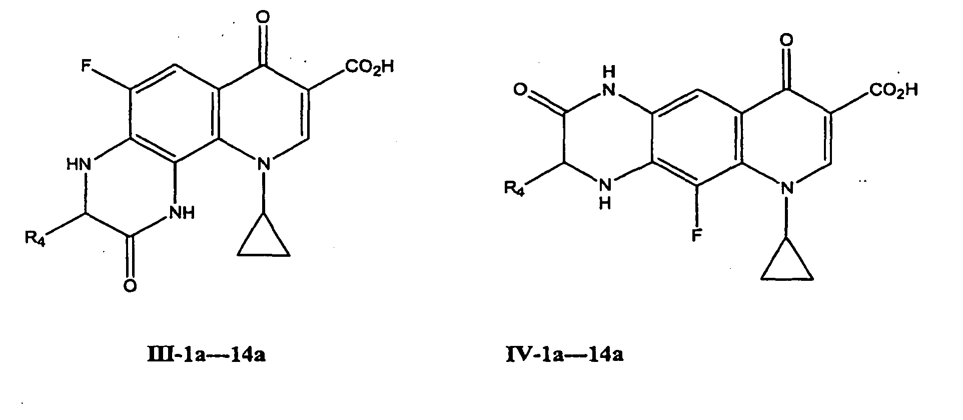 or a physiologically tolerated salt or ester thereof, in which R₄ is as set out in the table below.
| **Entry** **III**/**IV** | α**-Amino** **acid (L-)** | **R₄** |
|---|---|---|
| **1** | Gly | H |
| **2** | Ala | Me |
| **3** | Val | (Me)₂CH |
| **4** | Leu | (Me)₂CH₂CH |
| **5** | Ile | Et(Me)CH |
| **6** | ser | CH₂OH |
| **7** | Thr | MeCHOH |
| **8** | Met | MeSCH₂CH₂ |
| **9** | N-Ac Lys | Ac-NH(CH₂)₄ |
| **10** | Asp | CH₂CO₂H |
| **11** | Glu | CH₂CH₂CO₂H |
| **12** | Phe | CH₂Ph |
| **13** | Phenyl glycine* | Ph* |
| **14** | Amino butyric acid* | Et* |
| | | |
|---|---|---|
| * non-proteinic | | |

12. A compound according to any one of claims 1 to 7, in which the compound is of the formulae IIIA or IVA, 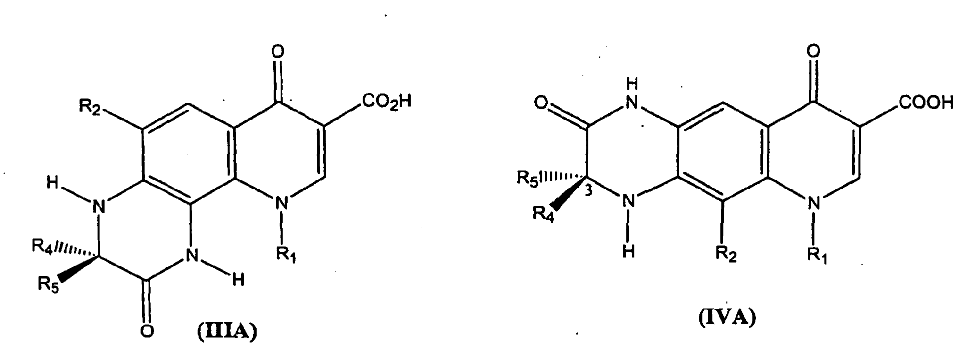 or a physiologically tolerated salt or ester thereof, in which
R₁ = *c*-C₃H₅ or Et,
R₂ = F, Cl, OH or OMe,
R₄ = R₅: Me or Ph.

13. A compound according to any one of claims 1 to 7, in which the compound is of the formula IIIB or IVB: 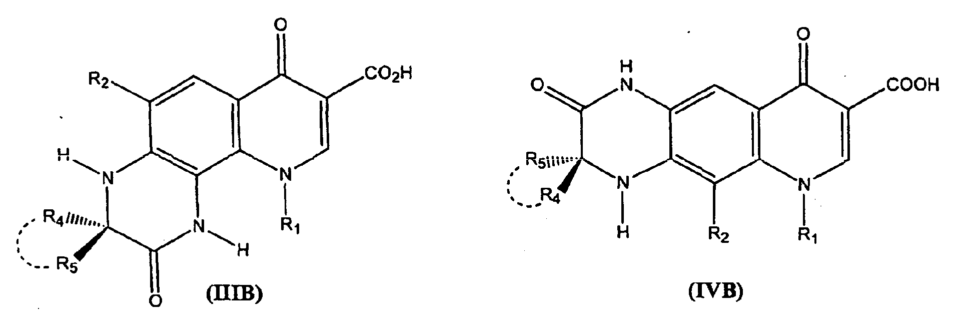 or a physiologically tolerated salt or ester thereof, in which
R₁ = *c*-C₃H₅ or Et,
R₂ = F, Cl, OH or OMe,
R₄ and R₅ = [CH₂]₂; [CH₂]₃ or [CH₂]₄.

14. A compound according to any one of claims 1 to 7, in which the compound is of the formula IX or X 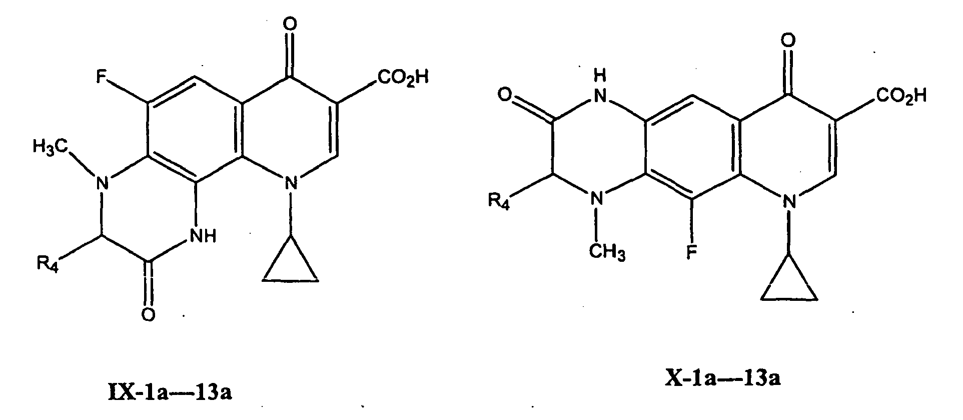 or a physiologically tolerated salt or ester thereof, in which R₄ is as set out in the table below:-
| **Entry** **IX**/**X** | **N-Me** α**-Amino** **acid (L-)** | **R₄** |
|---|---|---|
| **1** | N-Me Gly (Sarcosine) | H |
| **2** | N-Me Ala | Me |
| **3** | N-Me Val | (Me)₂CH |
| **4** | N-Me Leu | (MehCH₂CH |
| **5** | N-Me Ile | Et(Me)CH |
| **6** | N-Me Ser | CH₂OH |
| **7** | N-Me Thr | MeCHOH |
| **8** | N-Me Met | MeSCH₂CH₂ |
| **9** | N-Me Asp | CH₂CO₂H |
| **10** | N-Me Glu | CH₂CH₂CO₂H |
| **11** | N-Me Phe | CH₂Ph |
| **12** | N-Me Phenyl glycine* | Ph* |
| **13** | N-Me Amino butyric acid* | Et* |
| | | |
|---|---|---|
| * non-proteinic | | |

15. A process for the preparation of a compound of the general formula A or B as defined in any one of the preceding claims which comprises reacting a compound of the general formula 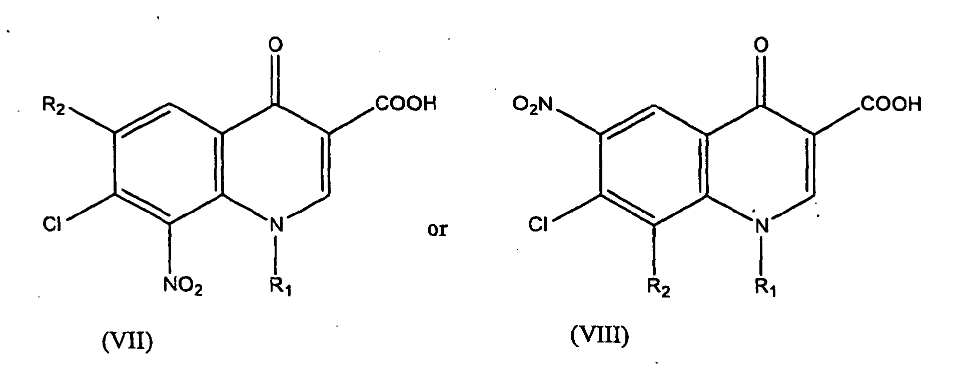 in which R₁ and R₂ are as defined in any one of the preceding claims, with a compound of the general formula 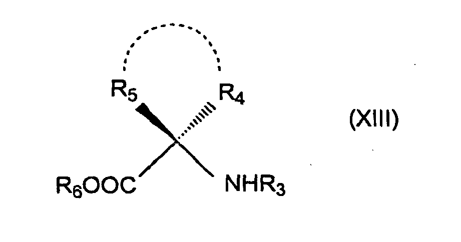 in which R₃, R₄, R₅ and R₆ are as defined in any one of the preceding claims, to form a compound of general formula A or B in which R₆ is a hydrogen atom, R₇ represents a group - COOR₆ and R₈ represents a nitro group;
if desired, subjecting the resultant compound to reductive cyclization to form a compound of general formula A or B in which R₇ and R₈ together represent a group - C(O)-NR₉-, where R₉ is as defined in any one of the preceding claims;
if desired, reacting the resultant compound with an alkylating agent to form a compound of general formula A or B in which R₆ is an alkyl group;
and/or if desired, converting the resultant compound to a physiologically tolerated salt of ester thereof.

16. A process for the preparation of a compound of the formula III or IV as defined in claim 11, which comprises the step of reductive cyclization of a respective compound of the formulae V or VI 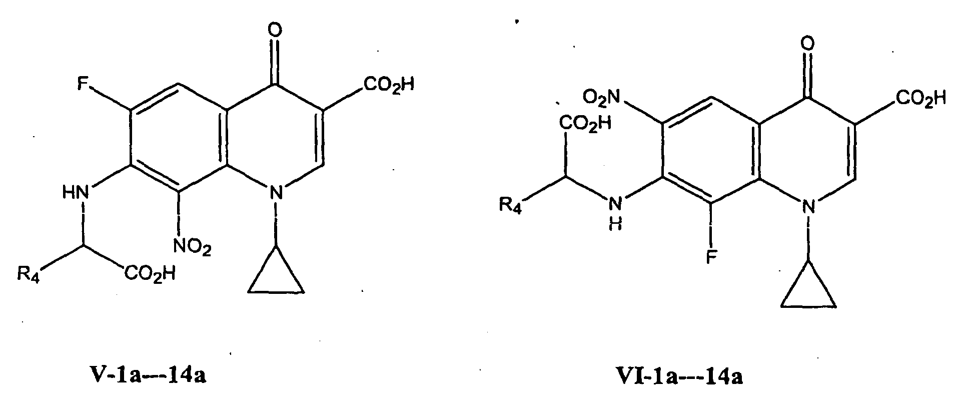 in which R₄ is as defined in claim 11, using sodium dithionite in aqueous potassium carbonate solution at room temperature.

17. A process for the preparation of a compound of the formula V or VI as defined in claim 16, which comprises the step of reacting a compound of the formula VII or VIII respectively, 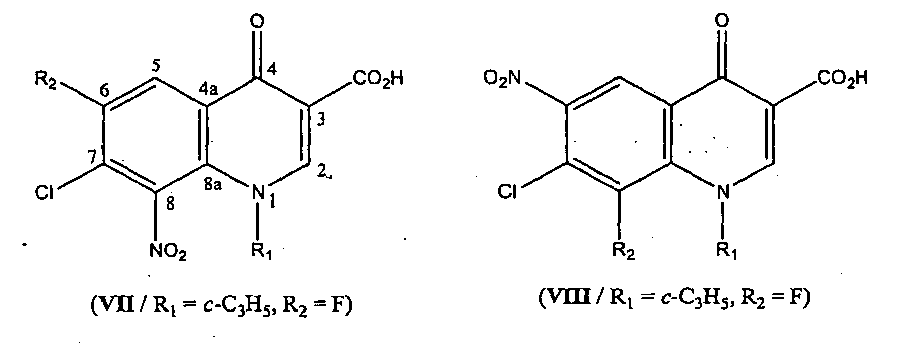 in which R₁ and R₂ are as defined in claim 11, with a particular primary α-amino acid in aqueous ethanolic potassium hydrogen carbonate solution at 60-90 °C for 1-5 days.

18. A process for the preparation of a compound of the formula IIIA or IVA as defined in claim 12, which comprises the step of reductive cyclization of a respective compound of the formulae VA or VIA 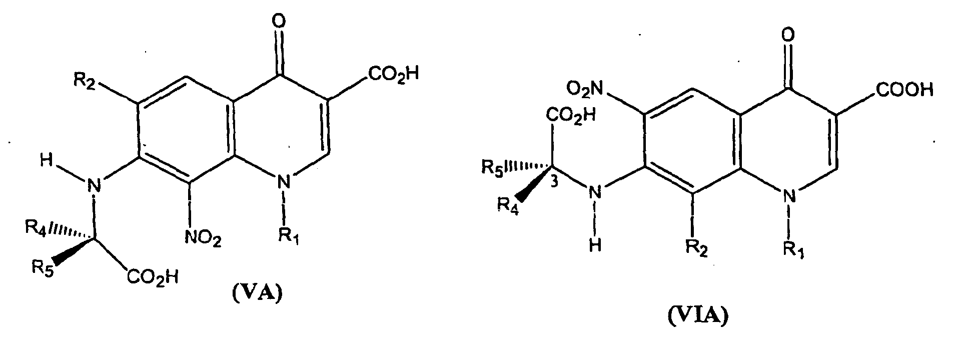 in which R₁, R₂, R₄ and R₅ are as defined in claim 12, using sodium dithionite in aqueous potassium carbonate solution at room temperature.

19. A process for the preparation of a compound of the formula VA or VIA as defined in claim 18, which comprises the step of reacting a compound of the formula VII or VIII as defined in claim 17 respectively, with a particular primary α-amino acid in aqueous ethanolic potassium hydrogen carbonate solution at 60-90 °C for 1-5 days.

20. A process for the preparation of a compound of the formula IIIB or IVB as defined in claim 13, which comprises the step of reductive cyclization of a respective compound of the formulae VB or VIB 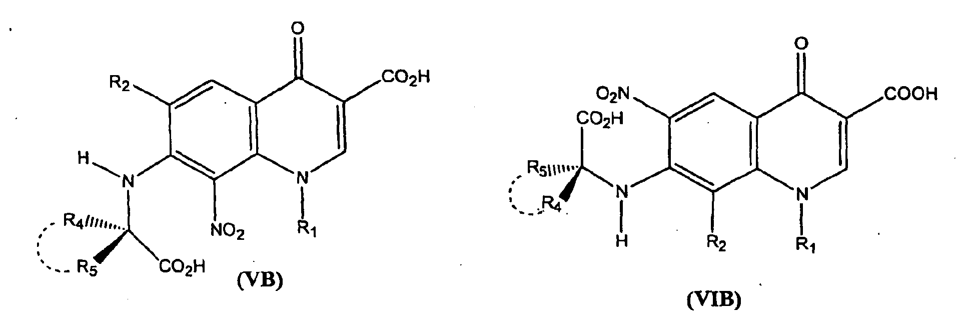 in which R₁, R₂, R4 and R₅ are as defined in claim 13, using sodium dithionite in aqueous potassium carbonate solution at room temperature.

21. A process for the preparation of a compound of the formula VB or VIB as defined in claim 20, which comprises the step of reacting a compound of the formula VII or VIII as defined in claim 17 respectively, with a particular primary α-amino acid in aqueous ethanolic potassium hydrogen carbonate solution at 60-90 °C for 1-5 days.

22. A process for the preparation of a compound of the formula IX or X as defined in claim 14, which comprises the step of reductive cyclization of a respective compound of the formula XI or XII 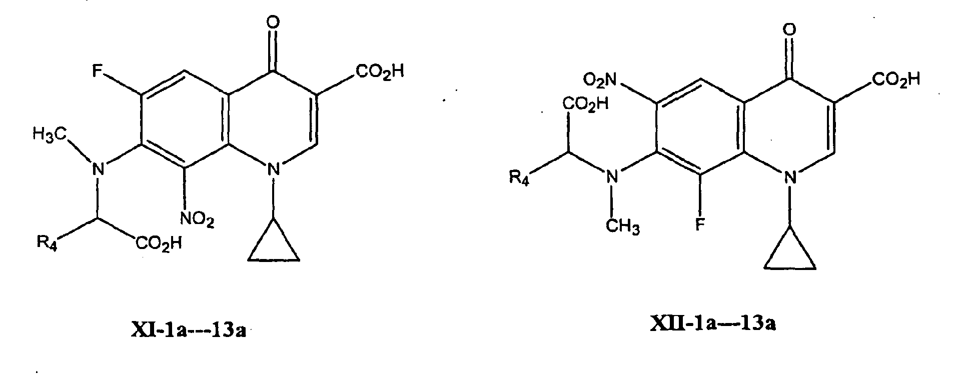 in which R₄ is as defined in claim 14, using sodium dithionite in aqueous potassium carbonate solution at room temperature.

23. A process for the preparation of a compound of the formula XI or XII as defined in claim 22, which comprises the step of reacting a compound of the formulae VII or VIII respectively, with a particular N-methyl α-amino acid in aqueous ethanolic potassium hydrogen carbonate solution at 60-90 °C for 1-5 days.

24. A process for the preparation of a methyl carboxylate ester (as a prodrug of its carboxy-compound) of the formulae I or II as defined in claim 8, IA or IIA as defined in claim 9, IB or IIB as defined in claim 10, 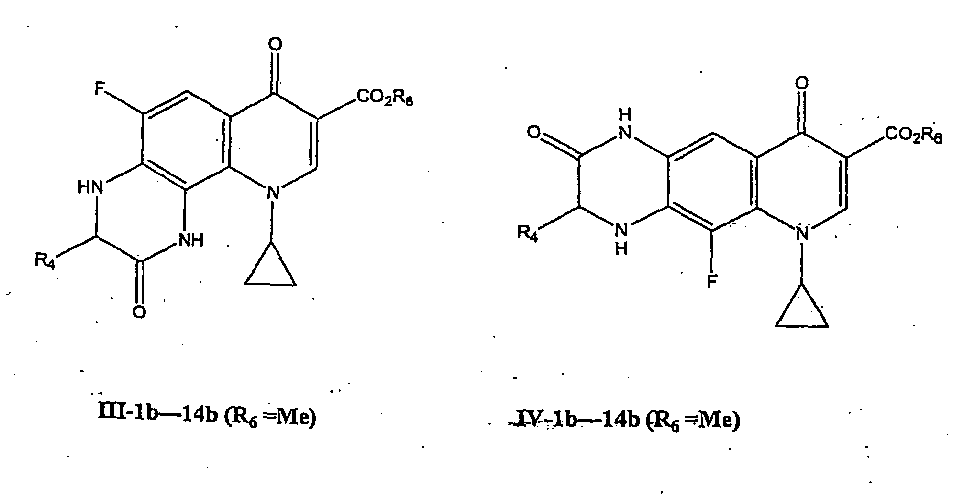 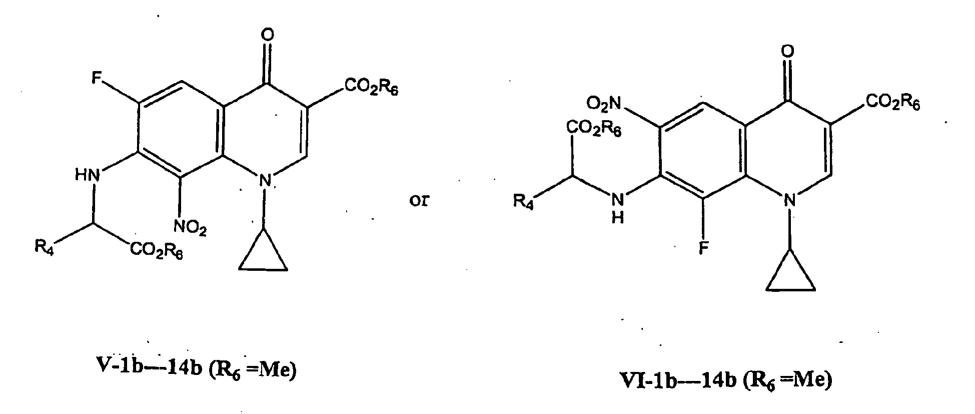 where
| **Entry** **III/IV/V/VI** | **α-Amino** **acid (L-)** | **R₄** |
|---|---|---|
| 1 | Gly | H |
| 2 | Ala | Me |
| 3 | Val | (Me)₂CH |
| 4 | Leu | (Me)₂CH₂CH |
| 5 | Ile | Et(Me)CH |
| 6 | ser | CH₂OH |
| 7 | Thr | MeCHOH |
| 8 | Met | MeSCH₂CH₂ |
| 9 | N-Ac Lys | Ac-NH(CH₂)₄ |
| 10 | Asp | CH₂CO₂H |
| 11 | Glu | CH₂CH₂CO₂H |
| 12 | Phe | CH₂Ph |
| 13 | phenyl glycine* | Ph* |
| 14 | Amino butyric acid* | Et* |
| | | |
|---|---|---|
| * non-proteinic | | |

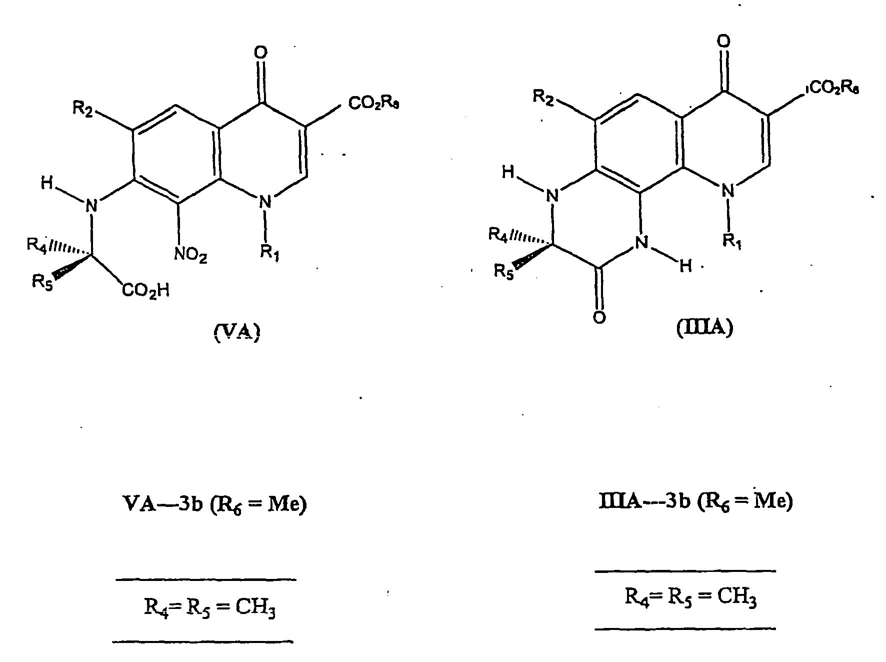

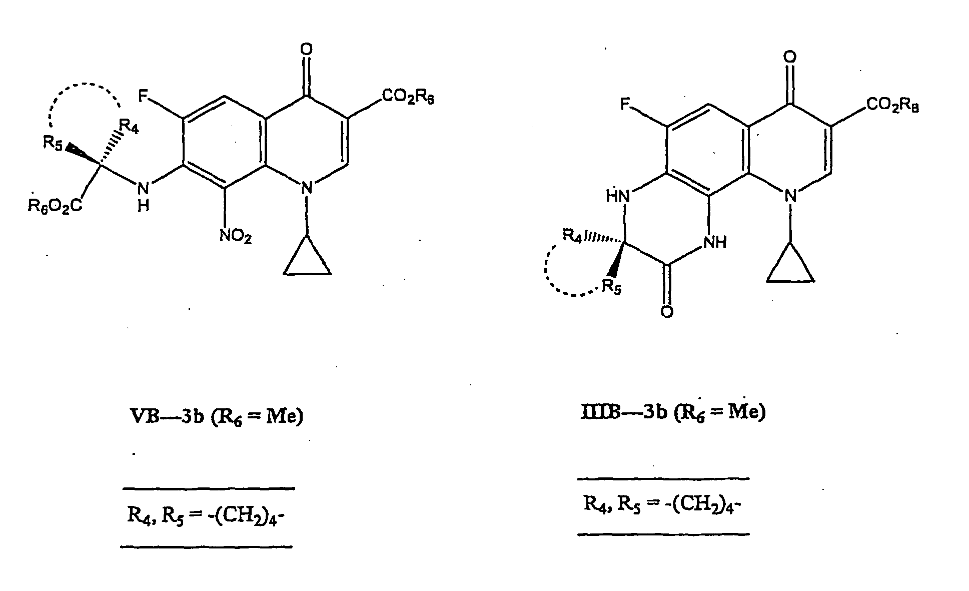

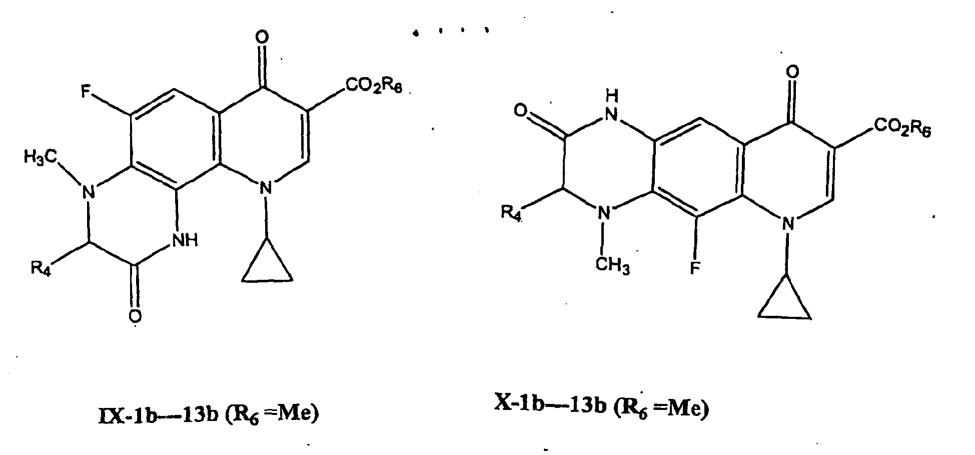

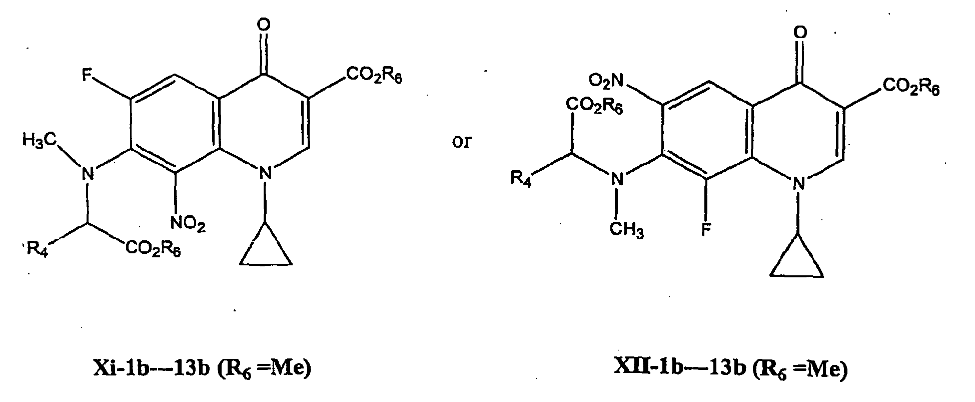
where
| **Entry** **IX/X/XI/XII** | **N-Me α-Amino** **acid (L-)** | **R₄** |
|---|---|---|
| **1** | N-Me Gly (Sarcosine) | H |
| **2** | N-Me Ala | Me |
| **3** | N-Me Val | (Me)₂CH |
| **4** | N-Me Leu | (Me)₂CH₂CH |
| **5** | N-Me Ile | Et(Me)CH |
| **6** | N-Me Ser | CH₂OH |
| **7** | N-Me Thr | MeCHOH |
| **8** | N-Me Met | MeSCH₂CH₂ |
| **9** | N-Me Asp | CH₂CO₂H |
| **10** | N-Me Glu | CH₂CH₂CO₂H |
| **11** | N-Me Phe | CH₂Ph |
| **12** | N-Me Phenyl glycine* | Ph* |
| **13** | N-Me Amino butyric acid* | Et* |
| | | |
|---|---|---|
| * non-proteinic | | |
which comprises the step of reacting the respective carboxy compound with diazomethane etherate at 0-10 °C for 20-60 minutes.

25. A pharmaceutical composition comprising a compound of the general formula A or B or a physiologically tolerated salt or ester thereof as defined in claim 1 and a carrier.

26. A process for the preparation of a pharmaceutical composition according to claim 25 which comprises bringing a compound of the general formula A or B or a physiologically tolerated salt or ester thereof as defined in claim 1 into association with a carrier.

27. A compound of the general formula A or B or a physiologically tolerated salt or ester thereof as defined in claim 1 for use in medicine.

28. A compound according to claim 27 for use as an antibacterial agent.

29. A compound of the formula III, IIIB, V or VB as defined in claim 11, claim 13, claim 16 or claim 20 respectively for use as an antibacterial agent, preferably against Gram negative bacteria (E-*coli*) and Gram positive bacteria (S. *aureus*).

30. A compound of the formulae IX or XI as defined in claim 14 or claim 22 respectively for use as an antibacterial agent, preferably against Gram negative bacteria (E-*coli*) and Gram positive bacteria (S. *aureus*).

31. Use of a compound of the general formula A or B or a physiologically tolerated salt or ester thereof as defined in claim 1 for the manufacture of a medicament for use as an antibacterial agent.

32. A process according to claim 15 wherein the optional alkylating step is carried out using diazomethane etherate at a temperature of 0 to 20°C, preferably 0 to 10°C, for 10 to 90, preferably 20 to 60, minutes.

## Patentansprüche

1. Verbindung mit der allgemeinen Formel: 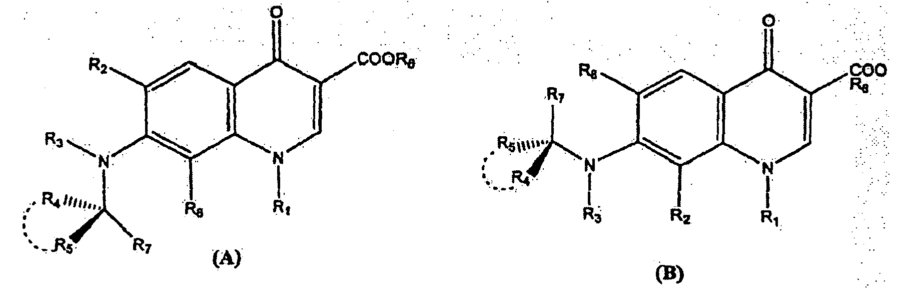 oder ein physiologisch toleriertes Salz oder ein physiologisch tolerierter Ether davon,
wobei
R₁ eine wahlweise substituierte C₁₋₁₂-Alkyl-, C₂₋₁₂-Alkenyl-, C₃₋₁₂-Cycloalkyl-, C₁-₁₂-alkoxy-, Amino-, C₁₋₁₂-Alkylamino-, Di-C₁₋₁₂-akylamino-, C₆₋₂₄-Aryl- oder Z₇₋₃₀-Aralkylgruppe darstellt;
R₂ ein Halogenatom, eine Hydroxylgruppe oder eine wahlweise substituierte C₁₋₁₂-Alkoxygruppe darstellt;
R₃ ein Wasserstoffatom oder eine wahlweise substituierte C₁₋₁₂-Akylgruppe darstellt;
R₄ und R₅ jeweils unabhängig voneinander ein Wasserstoffatom oder eine wahlweise substituierte C₁₋₁₂-Alkyl-, C₆₋₂₄-Aryl-, C₇₋₃₀-Aralkyl-, eine 3- bis 18-gliedrige Heterocyclyl- oder 3- bis 18-gliedrige Heterocyclyl-C₁₋₆-Alkylgruppe darstellen, oder
R₄ und R₅ zusammen eine wahlweise substituierte Alkylengruppe darstellen;
R₆ ein Wasserstoffatom oder eine wahlweise substituierte C₁₋₁₂-Alkylgruppe darstellt;
R₇ eine Gruppe -COOR₆ darstellt, wobei R₆ der oben gegebenen Definition entspricht, und
R₈ eine Nitrogruppe darstellt, oder R₇ und R₈ zusammen eine Gruppe -C(O)-NR₉-darstellen, wobei R₉ ein Wasserstoffatom oder eine wahlweise substituierte C₁₋₁₂-Alkylgruppe ist;
wobei optionale Substituenten unter Halogenatomen, Nitro-, Cyano-, Hydroxyl-, C₃₋₈-Cycloalkyl-, C₁₋₁₂-Alkyl-, C₁₋₁₂-Halogenalkyl-, C₁₋₁₂-Alkoxy-, C₁₋₁₂-Halogenalkoxy-, Amino-, C₁₋₁₂-Alkylamino-, Di-(C₁₋₁₂-alkyl)amino-, Formyl-, C₁₋₁₂-Alkoxycarbonyl-, Carboxyl-, C₁₋₁₂-Alkanoyl-, C₁₋₁₂-Alkylthio-, C₁₋₁₂-Alkylsulphinyl-, C₁₋₁₂-Alkylsulphonyl-, C₁₋₁₂-Alkylsulphonato-, Carbamoyl-, C₁₋₁₂-Alkylamido-, C₆₋₁₀-Aryl- und C₇₋₁₁-Aralkylgruppen ausgewählt sind.

2. Verbindung nach Anspruch 1, wobei R₁ eine C₁₋₆-Alkyl-, C₂₋₆-Alkenyl-, C₃₋₆-Cycloalkyl-, C₁₋₆-Alkoxy-, C₁₋₆-Alkylamino- oder C₆₋₁₄-Arylgruppe darstellt, wobei jede Gruppe wahlweise durch einen oder mehrere Substituenten substituiert ist, die aus der Gruppe ausgewählt sind, die aus Halogenatomen, Nitro-, Cyano-, Hydroxyl-, C₁₋₄-Alkyl-, C₁₋₄-Halogenalkyl-, C₁₋₄-Alkoxy- und C₁₋₄-Halogenalkoxygruppen besteht.

3. Verbindung nach Anspruch 1 oder Anspruch 2, wobei R₂ ein Halogenatom, eine Hydroxylgruppe oder eine C₁₋₄-Alkoxygruppe darstellt.

4. Verbindung nach einem der vorstehenden Ansprüche, wobei R₃ ein Wasserstoffatom oder eine C₁₋₄-Alkylgruppe darstellt.

5. Verbindung nach einem der vorstehenden Ansprüche, wobei R₄ und R₅ jeweils unabhängig voneinander ein Wasserstoffatom oder eine C₁₋₆-Alkyl-, C₆₋₁₄-Aryl-, C₇₋₁₈-Aralkyl-, eine 3- bis 10-gliedrige Heterocyclyl- oder 3- bis 10-gliedrige Heterocyclyl-C₁₋₆-Alkylgruppe darstellen, wobei jede Gruppe wahlweise durch einen oder mehrere Substituenten substituiert ist, die aus der Gruppe ausgewählt sind, die aus Halogenatomen, Hydroxyl-, C₁₋₄-Halogenalkyl-, C₁₋₄-Alkoxy-, C₁₋₄-Halogenalkoxy-, C₁₋₄-Alkylthio-, C₁₋₄-Halogenalkylthio-, Carboxyl-, Amino-, Carbamoyl-, C₁₋₄-Alkylamido- und NH-CO-R'-Gruppen besteht, wobei R' Wasserstoff, eine C₁₋₄-Alkyl- oder Phenylgruppe ist, oder wobei R₄ und R₅ zusammen eine Gruppe -(CH₂)ₙ- darstellen, wobei n eine ganze Zahl von 2 bis 5 ist.

6. Verbindung nach einem der vorstehenden Ansprüche, wobei R₆ ein Wasserstoffatom oder eine C₁₋₄-Alkylgruppe darstellt.

7. Verbindung nach einem der vorstehenden Ansprüche, wobei R₇ eine Gruppe - COOR₆ darstellt, wobei R₆ ein Halogenatom oder eine C₁₋₄-Alkylgruppe ist, und R₈ eine Nitrogruppe darstellt, oder wobei R₇ und R₈ zusammen eine Gruppe -C(O)-NH-darstellen.

8. Verbindung nach einem der vorstehenden Ansprüche, wobei die Verbindung die Formel I oder II, 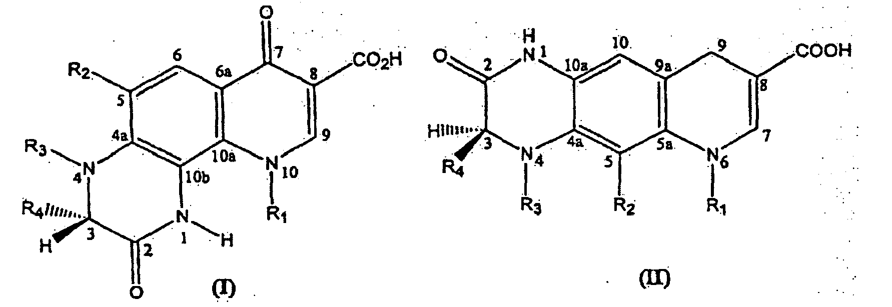 oder ein physiologisch toleriertes Salz oder einen physiologisch tolerierten Ester davon aufweist, wobei
R₁ = c-C₃H₅, Et, 2,4-(F)₂C₆H₃ ist;
R₂ = F, Cl, OH oder OMe ist;
R₃ = H, Me ist;
R₄ = H, eine wahlweise durch Hydroxyl substituierte C₁₋₄-Alkylgruppe, eine C₁₄-Alkoxy- oder C₁₋₄-Alkylthiogruppe, -CH₂CO₂H, -CH₂CONH₂, -CH₂CH₂CO₂H, -CH₂CH₂CONH₂, - (CH₂)₄NHCOR' [R' = H, Me, Ph], Ph, p-(X)C₆H₄ [X = F, Cl, OH. OMe], PhCH₂, p-(OH)C₆H₄CH₂-, 2,4-(OH)₂C₆H₃CH₂-, 3-(Indolyl)CH₂ oder 4-(Imidazolyl)CH₂- ist, wobei Me Methyl und Ph Phenyl ist.

9. Verbindung nach einem der Ansprüche 1 bis 7, wobei die Verbindung die Formel IA oder IIA, 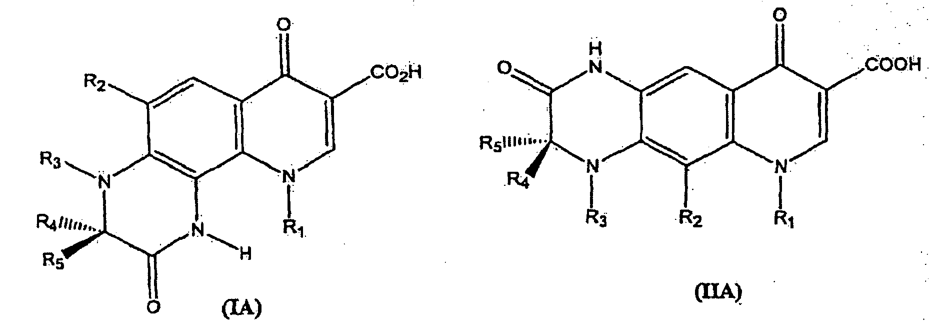 oder ein physiologisch toleriertes Salz oder einen physiologisch tolerierten Ester davon aufweist, wobei
R₁ = c-C₃H₅, Et, 2,4-(F)₂C₆H₃ ist;
R₂ = F, Cl, OH oder OMe ist;
R₃ = H, Me ist;
R₄ = R₅ = Me, Ph, 2-Py, 2-Thienyl oder 2-Furyl sind, wobei Me Methyl, Ph Phenyl und Py Pyridyl ist.

10. Verbindung nach einem der Ansprüche 1 bis 7, wobei die Verbindung die Formel IB oder IIB, 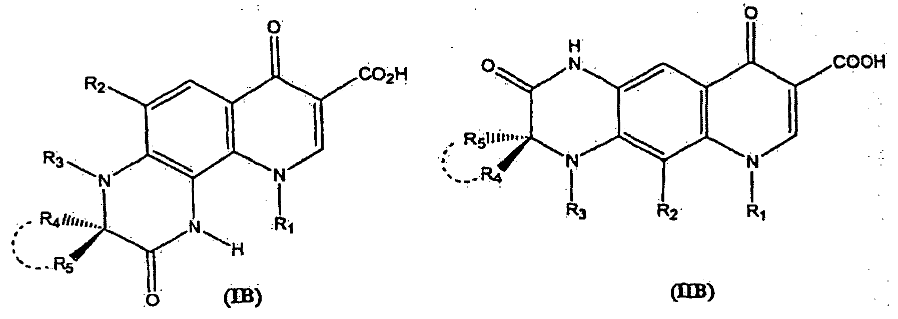 oder ein physiologisch toleriertes Salz oder einen physiologisch tolerierten Ester davon aufweist, wobei
R₁ = _{C}-C₃H₅, Et, 2,4-(F)₂C₆H₃ ist;
R₂ = F, Cl, OH oder OMe ist;
R₃ = H, Me ist;
R₄ und R₅ = [CH₂]ₙ sind, wobei n = 2, 3, 4 oder 5 ist.

11. Verbindung nach einem der Ansprüche 1 bis 7, wobei die Verbindung die Formel III oder IV, 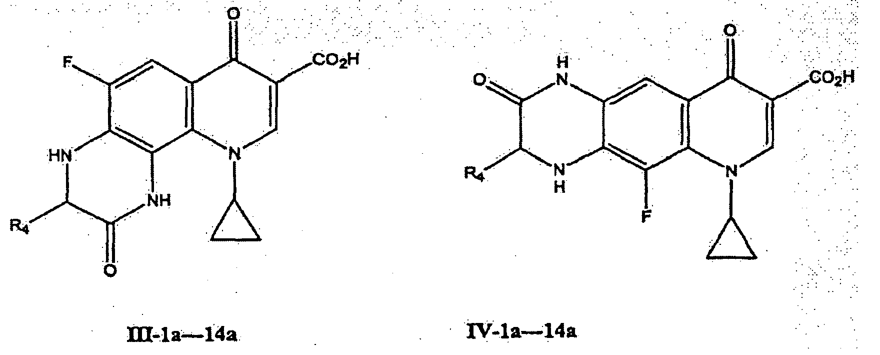 oder ein physiologisch toleriertes Salz oder einen physiologisch tolerierten Ester davon aufweist, wobei R₄ in der untenstehenden Tabelle angegeben ist.
| Eintrag III/IV | α-Aminosäure (L-) | R₄ |
|---|---|---|
| 1 | Gly | H |
| 2 | Ala | Me |
| 3 | Val | (Me)₂CH |
| 4 | Leu | (Me)₂CH₂CH |
| 5 | Ile | Et(Me)CH |
| 6 | Ser | CH₂OH |
| 7 | Thr | MeCHOH |
| 8 | Met | MeSCH₂CH₂ |
| 9 | N-Ac Lys | Ac-NH(CH₂)₄ |
| 10 | Asp | CH₂CO₂H |
| 11 | Glu | CH₂CH₂CO₂H |
| 12 | Phe | CH₂Ph |
| 13 | Phenylglycin* | Ph* |
| 14 | Aminobuttersäure* | Et* |
| | | |
|---|---|---|
| * nicht proteinhaltig | | |

12. Verbindung nach einem der Ansprüche 1 bis 7, wobei die Verbindung die Formel IIIA oder IVA, 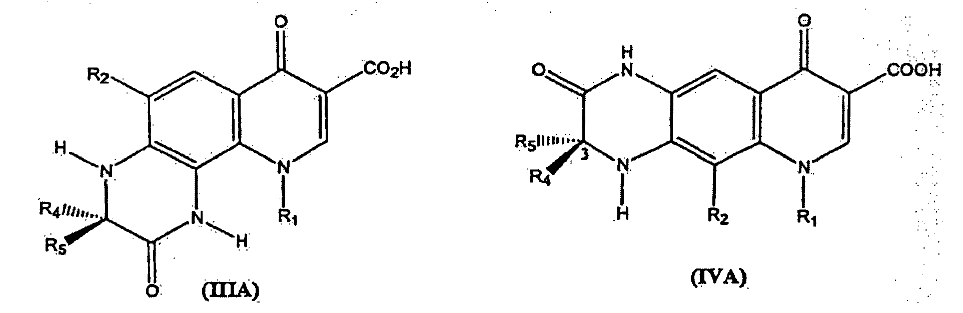 oder ein physiologisch toleriertes Salz oder einen physiologisch tolerierten Ester davon aufweist, wobei
R₁ = c-C₃H₅ oder Et ist;
R₂ = F, Cl, OH oder OMe ist;
R₄ = R₅: Me oder Ph sind.

13. Verbindung nach einem der Ansprüche 1 bis 7, wobei die Verbindung die Formel IIIB oder IVB, 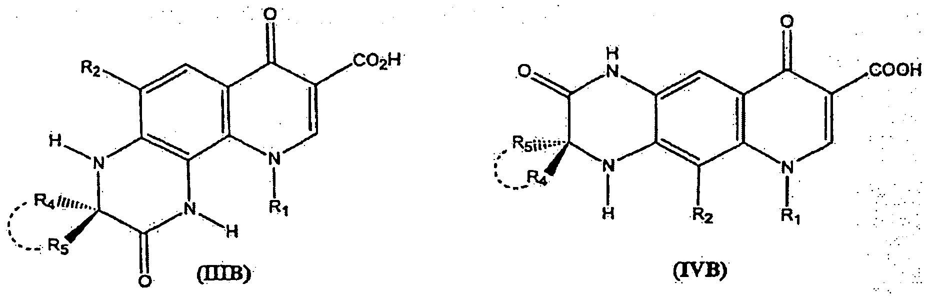 oder ein physiologisch toleriertes Salz oder einen physiologisch tolerierten Ester davon aufweist, wobei
R₁ = c-C₃H₅ oder Et ist;
R₂ = F, Cl, OH oder OMe ist;
R₄ und R₅ = [CH₂]₂; [CH₂]₃ oder [CH₂]₄ sind.

14. Verbindung nach einem der Ansprüche 1 bis 7, wobei die Verbindung die Formel IX oder X, 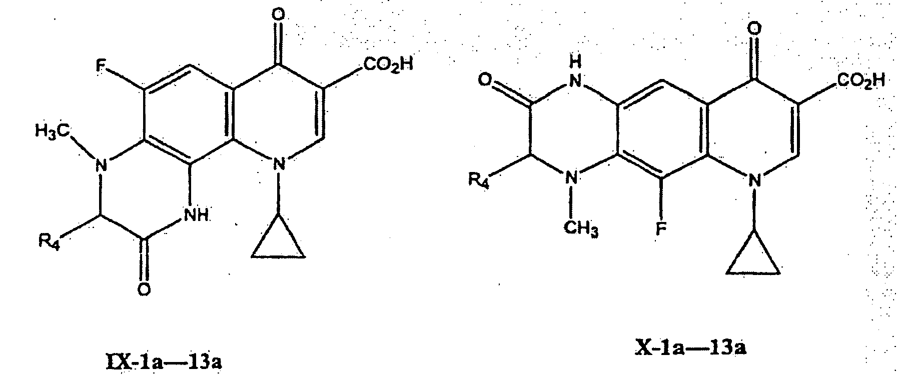 oder ein physiologisch toleriertes Salz oder einen physiologisch tolerierten Ester davon aufweist, wobei R₄ in der untenstehenden Tabelle angegeben ist.
| Eintrag IX/X | N-Me-α-Aminosäure (L-) | R₄ |
|---|---|---|
| 1 | N-Me Gly (Sarcosin) | H |
| 2 | N-Me Ala | Me |
| 3 | N-Me Val | (Me)₂CH |
| 4 | N-Me Leu | (Me)₂CH₂CH |
| 5 | N-Me Ile | Et(Me)CH |
| 6 | N-Me Ser | CH₂OH |
| 7 | N-Me Thr | MeCHOH |
| 8 | N-Me Met | MeSCH₂CH₂ |
| 9 | N-Me Asp | CH₂CO₂H |
| 10 | N-Me Glu | CH₂CH₂CO₂H |
| 11 | N-Me Phe | CH₂Ph |
| 12 | N-Me Phenylglycin* | Ph* |
| 13 | N-Me Aminobuttersäure* | Et* |
| | | |
|---|---|---|
| * nicht proteinhaltig | | |

15. Verfahren zur Herstellung einer Verbindung mit der allgemeinen Formel A oder B, wie in einem der vorstehenden Ansprüche definiert, wobei das Verfahren aufweist: Reaktion einer Verbindung mit der allgemeinen Formel 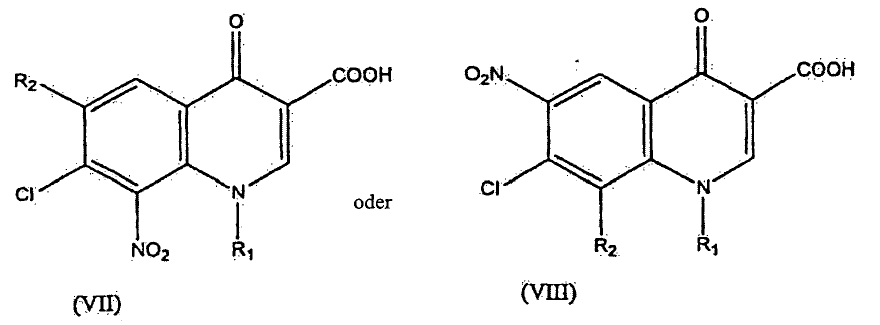 wobei R₁ und R₂ den Definitionen in einem der vorstehenden Ansprüche entsprechen, mit einer Verbindung mit der allgemeinen Formel 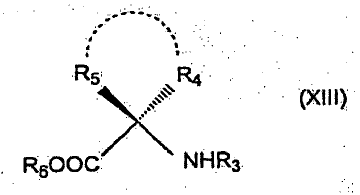 wobei R₃, R₄, R₅ und R₆ den Definitionen in einem der vorstehenden Ansprüche entsprechen, um eine Verbindung mit der allgemeinen Formel A oder B zu bilden, in der R₆ ein Wasserstoffatom ist, R₇ eine Gruppe -COOR₆ darstellt und R₈ eine Nitrogruppe darstellt;
nach Wunsch Ausführen einer reduktiven Cyclisierung der resultierenden Verbindung, um eine Verbindung mit der allgemeinen Formel A oder B zu bilden, in der R₇ und R₈ zusammen eine Gruppe -C(O)-NR₉- bilden, wobei R₉ der Definition in einem der vorstehenden Ansprüche entspricht;
nach Wunsch Reaktion der resultierenden Verbindung mit einem Alkylierungsmittel, um eine Verbindung mit der allgemeinen Formel A oder B zu bilden, in der R₆ eine Alkylgruppe ist;
und/oder nach Wunsch Umwandeln der resultierenden Verbindung in ein physiologisch toleriertes Salz oder einen physiologisch tolerierten Ester der Verbindung.

16. Verfahren zur Herstellung einer Verbindung mit der Formel III oder IV gemäß Anspruch 11, das den Schritt zur reduktiven Cyclisierung einer entsprechenden Verbindung gemäß Formel V oder VI, 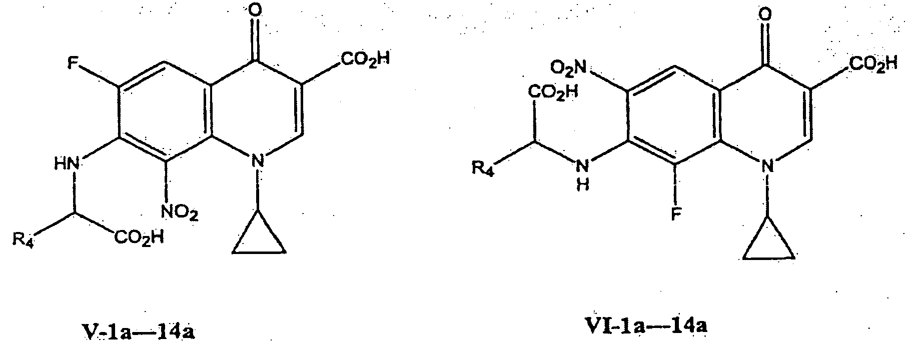 wobei R₄ der Definition in Anspruch 11 entspricht, unter Verwendung von Natriumdithionit in wässriger Kaliumcarbonatlösung bei Raumtemperatur aufweist.

17. Verfahren zur Herstellung einer Verbindung mit der Formel V oder VI gemäß Anspruch 16, das den Schritt zur Reaktion einer Verbindung mit der Formel VII bzw. VIII, 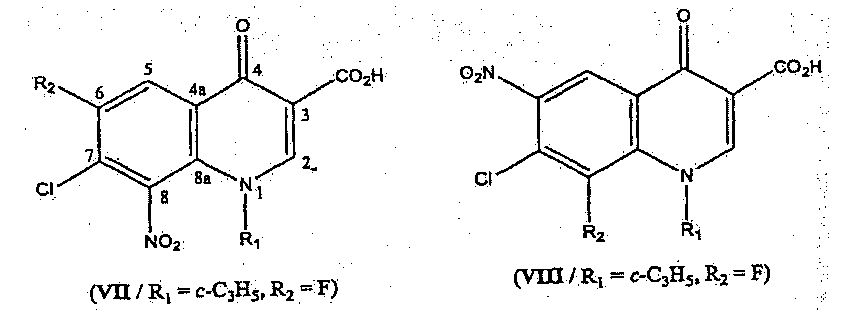 wobei R₁ und R₂ den Definitionen in Anspruch 11 entsprechen, mit einer bestimmten primären α-Aminosäure in wässriger ethanolischer Kaliumhydrogencarbonatlösung bei 60-90°C im Verlauf von 1-5 Tagen aufweist.

18. Verfahren zur Herstellung einer Verbindung mit der Formel IIIA oder IVA gemäß Anspruch 12, das den Schritt zur reduktiven Cyclisierung einer entsprechenden Verbindung mit der Formel VA oder VIA, 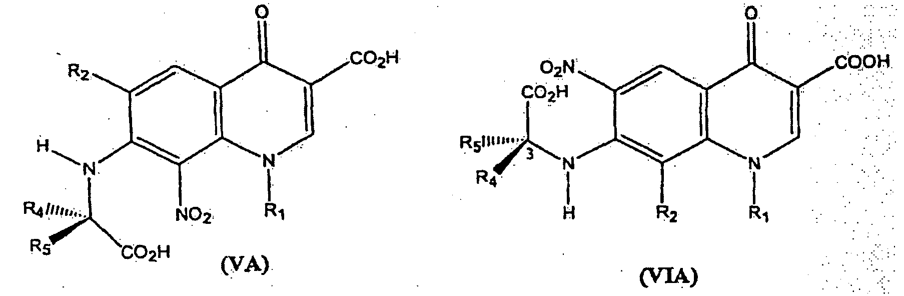 wobei R₁, R₂, R₄ und R₅ den Definitionen in Anspruch 12 entsprechen, unter Verwendung von Natriumdithionit in wässriger Kaliumcarbonatlösung bei Raumtemperatur aufweist.

19. Verfahren zur Herstellung einer Verbindung mit der Formel VA oder VIA gemäß Anspruch 18, das den Schritt zur Reaktion einer Verbindung mit der Formel VII bzw. VIII gemäß Anspruch 17 mit einer bestimmten primären α-Aminosäure in wässriger ethanolischer Kaliumhydrogencarbonatlösung bei 60-90°C im Verlauf von 1-5 Tagen aufweist.

20. Verfahren zur Herstellung einer Verbindung mit der Formel IIIB oder IVB gemäß Anspruch 13, das den Schritt zur reduktiven Cyclisierung einer entsprechenden Verbindung mit der Formel VB oder VIB, 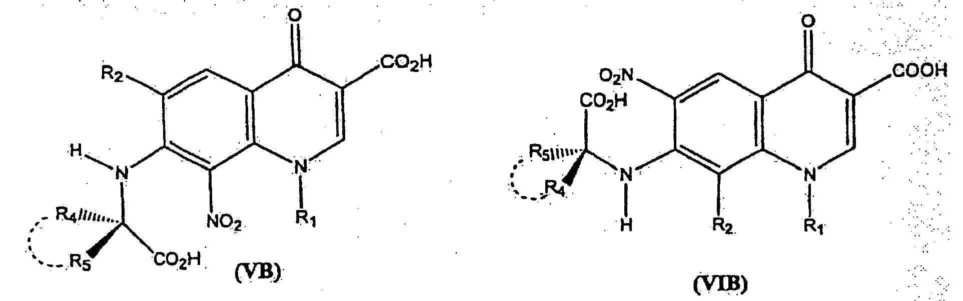 wobei R₁, R₂, R₄ und R₅ den Definitionen in Anspruch 13 entsprechen, unter Verwendung von Natriumdithionit in wässriger Kaliumcarbonatlösung bei Raumtemperatur aufweist.

21. Verfahren zur Herstellung einer Verbindung mit der Formel VB oder VIB gemäß Anspruch 20, das den Schritt zur Reaktion einer Verbindung mit der Formel VII bzw. VIII gemäß Anspruch 17 mit einer bestimmten primären α-Aminosäure in wässriger ethanolischer Kaliumhydrogencarbonatlösung bei 60-90°C im Verlauf von 1-5 Tagen aufweist.

22. Verfahren zur Herstellung einer Verbindung mit der Formel IX oder X gemäß Anspruch 14, das den Schritt zur reduktiven Cyclisierung einer entsprechenden Verbindung mit der Formel XI oder XII, 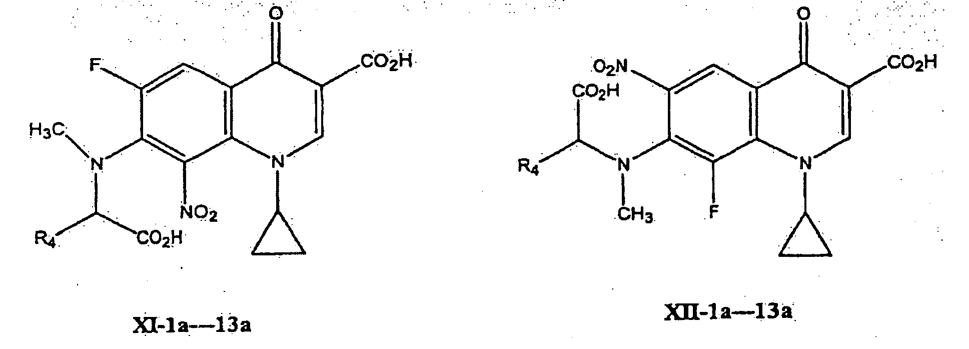 wobei R₄ der Definition in Anspruch 14 entspricht, unter Verwendung von Natriumdithionit in wässriger Kaliumcarbonatlösung bei Raumtemperatur aufweist.

23. Verfahren zur Herstellung einer Verbindung mit der Formel XI oder XII gemäß Anspruch 22, das den Schritt zur Reaktion einer Verbindung mit der Formel VII bzw. VIII mit einer bestimmten N-Methyl-α-Aminosäure in wässriger ethanolischer Kaliumhydrogencarbonatlösung bei 60-90°C im Verlauf von 1-5 Tagen aufweist.

24. Verfahren zur Herstellung eines Methxlcarboxylatesters (als Prodrug seiner Carboxyverbindung) mit der Formel I oder II gemäß Anspruch 8, IA oder IIA gemäß Anspruch 9, IB oder IIB gemäß Anspruch 10, 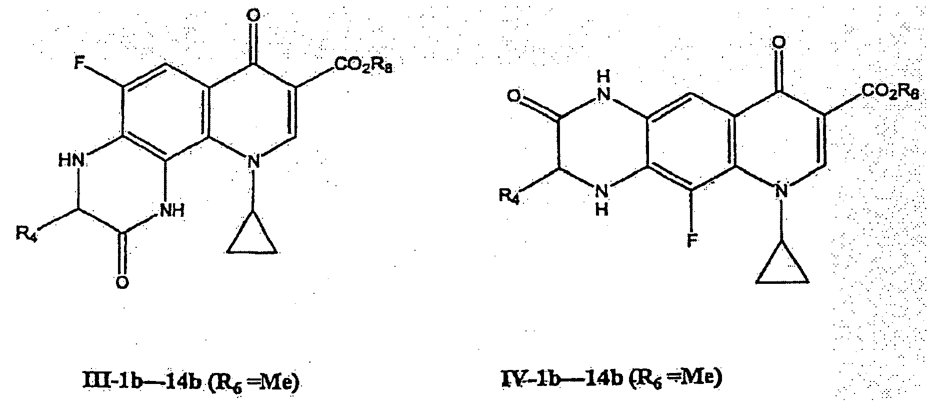 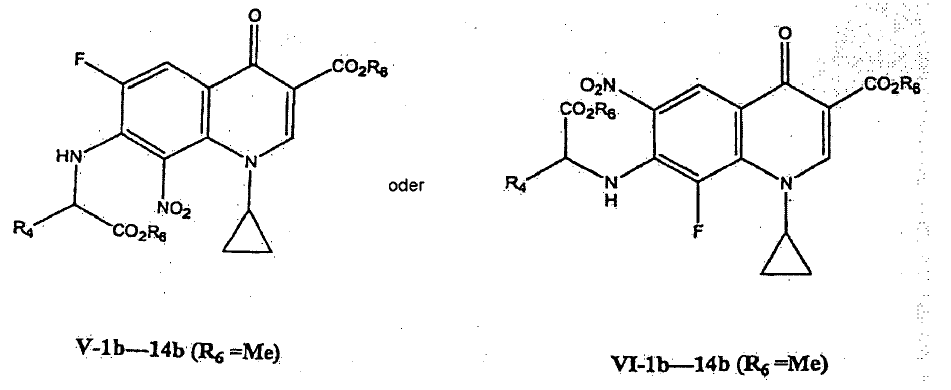 mit
| Eintrag III/IV/V/VI | α-Aminosäure (L-) | R₄ |
|---|---|---|
| 1 | Gly | H |
| 2 | Ala | Me |
| 3 | Val | (Me)₂CH |
| 4 | Leu | (Me)₂CH₂CH |
| 5 | Ile | Et(Me)CH |
| 6 | Ser | CH₂OH |
| 7 | Thr | MeCHOH |
| 8 | Met | MeSCH₂CH₂ |
| 9 | N-Ac Lys | Ac-NH(CH₂)₄ |
| 10 | Asp | CH₂CO₂H |
| 11 | Glu | CH₂CH₂CO₂H |
| 12 | Phe | CH₂Ph |
| 13 | Phenylglycin* | Ph* |
| 14 | Aminobuttersäure* | Et* |
| | | |
|---|---|---|
| * nicht proteirihaltig | | |

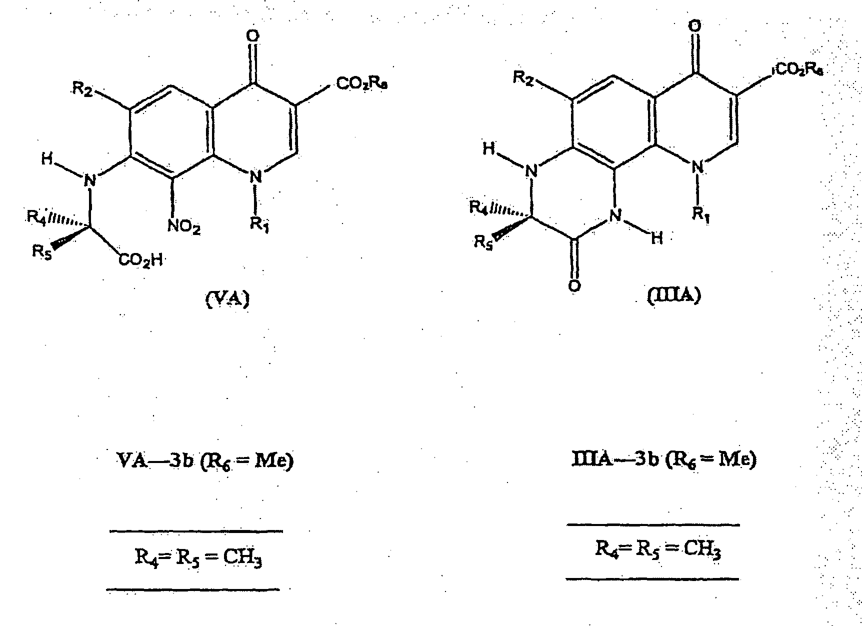

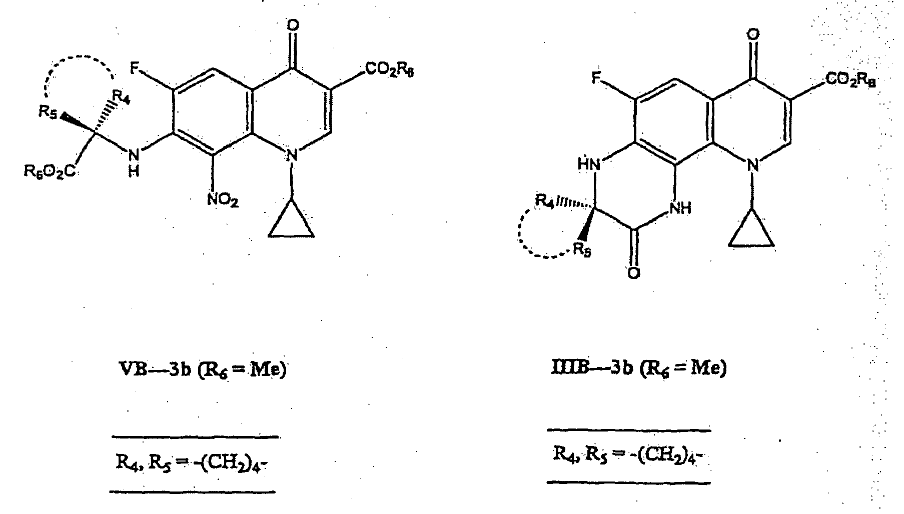

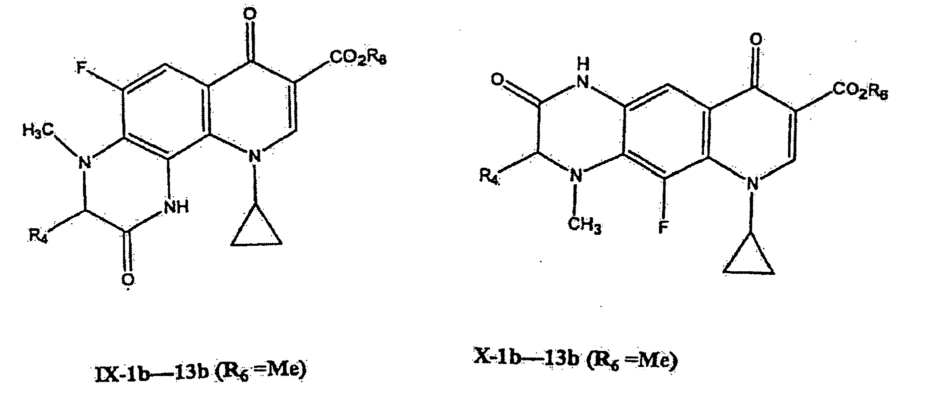

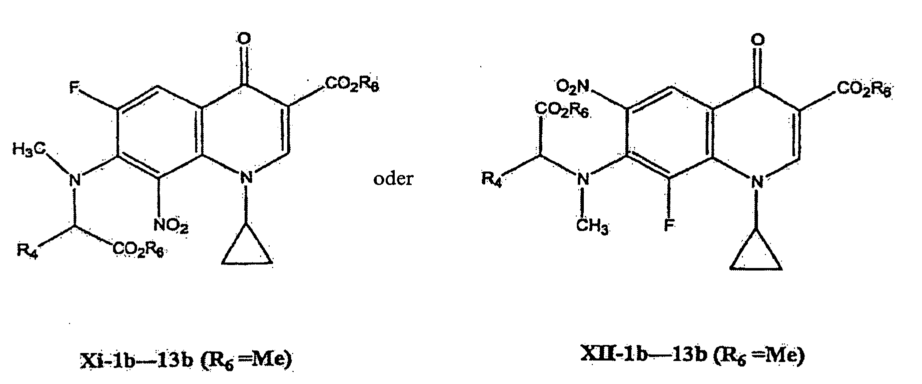
mit
| Eintrag IX/X/XI/XII | N-Me-α-Aminosäure (L-) | R₄ |
|---|---|---|
| 1 | N-Me Gly (Sarcosin) | H |
| 2 | N-Me Ala | Me |
| 3 | N-Me Val | (Me)₂CH |
| 4 | N-Me Leu | (Me)₂CH₂CH |
| 5 | N-Me Ile | Et(Me)CH |
| 6 | N-Me Ser | CH₂OH |
| 7 | N-Me Thr | MeCHOH |
| 8 | N-Me Met | MeSCH₂CH₂ |
| 9 | N-Me Asp | CH₂CO₂H |
| 10 | N-Me Glu | CH₂CH₂CO₂H |
| 11 | N-Me Phe | CH₂Ph |
| 12 | N-Me Phenylglycin* | Ph* |
| 13 | N-Me Aminobuttersäure* | Et* |
| | | |
|---|---|---|
| * nicht proteinhaltig | | |
wobei das Verfahren den Schritt zur Reaktion der entsprechenden Carboxyverbindung mit Diazomethanetherat bei 0-10°C über 20-60 Minuten aufweist.

25. Pharmazeutische Zusammensetzung, die eine Verbindung mit der allgemeinen Formel A oder B oder ein physiologisch toleriertes Salz oder einen physiologisch tolerierten Ester davon gemäß Anspruch I und einen Träger aufweist.

26. Verfahren zur Herstellung einer pharmazeutischen Zusammensetzung nach Anspruch 25, wobei eine Verbindung mit der allgemeinen Formel A oder B oder ein physiologisch toleriertes Salz oder ein physiologisch tolerierter Ester davon gemäß Anspruch 1 mit einem Träger vereinigt wird.

27. Verbindung mit der allgemeinen Formel A oder B oder ein physiologisch toleriertes Salz oder ein physiologisch tolerierter Ester davon gemäß Anspruch 1 zur Verwendung in der Medizin.

28. Verbindung nach Anspruch 27 zur Verwendung als antibakterielles Mittel.

29. Verbindung mit der Formel III, IIIB, V oder VB nach Anspruch 11, Anspruch 13, Anspruch 16 bzw. Anspruch 20 zur Verwendung als antibakterielles Mittel, vorzugsweise gegen gramnegative Bakterien (E. coli) und grampositive Bakterien (S. aureus).

30. Verbindung mit der Formel IX bzw. XI gemäß Anspruch 14 bzw. Anspruch 22 zur Verwendung als antibakterielles Mittel, vorzugsweise gegen gramnegative Bakterien (E. coli) und grampositive Bakterien (S. aureus).

31. Verwendung einer Verbindung mit der allgemeinen Formel A oder B oder eines physiologisch tolerierten Salzes oder Esters davon gemäß Anspruch 1 für die Herstellung eines Medikaments zur Verwendung als antibakterielles Mittel.

32. Verfahren nach Anspruch 15, wobei der optionale Alkylierungsschritt unter Verwendung von Diazomethanetherat bei einer Temperatur von 0 bis 20°C, vorzugsweise 0 bis 10°C im Verlauf von 10 bis 90, vorzugsweise 20 bis 60 Minuten ausgeführt wird.

## Revendications

1. Composé de formule générale 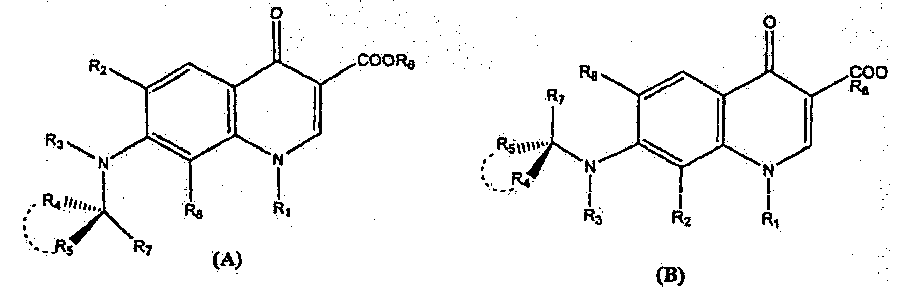 ou un de ses sels ou esters physiologiquement tolérés,
dans lequel
R₁ représente un groupe alkyle en C₁₋₁₂, alcényle en C₂₋₁₂, cycloalkyle en C₃₋₁₂, alcoxy en C₁₋₁₂, amino, (alkyl en C₁₋₁₂)amino, di(alkyl en C₁₋₁₂)amino, aryle en C₆₋₂₄ ou aralkyle en C₇₋₃₀ éventuellement substitué;
R₂ représente un atome d'halogène, un groupe hydroxyle ou un groupe alcoxy en C₁₋₁₂ éventuellement substitué;
R₃ représente un atome d'hydrogène ou un groupe alkyle en C₁₋₁₂ éventuellement substitué;
R₄ et R₅ représentent chacun indépendamment un atome d'hydrogène ou un groupe alkyle en C₁₋₁₂, aryle en C₆₋₂₄, aralkyle en C₇₋₃₀, hétérocyclyle à 3-18 chaînons ou (hétérocyclyl à 3-18 chaînons)-(alkyle en C₁₋₆), chaque groupe étant éventuellement substitué, ou
R₄ et R₅ ensemble représentent un groupe alkylène éventuellement substitué;
R₆ représente un atome d'hydrogène ou un groupe alkyle en C₁₋₁₂ éventuellement substitué;
R₇ représente un groupe -COOR₆, où R₆ est tel que défini ci-dessus, et
R₈ représente un groupe nitro, ou R₇ et R₈ ensemble représentent un groupe - C(O)-NR₉-, où R₉ est un atome d'hydrogène ou un groupe alkyle en C₁₋₁₂ éventuellement substitué;
les substituants facultatifs étant choisis parmi les atomes d'halogène et les groupes nitro, cyano, hydroxyle, cycloalkyle en C₃₋₈, alkyle en C₁₋₁₂, halogénoalkyle en C₁₋₁₂, alcoxy en C₁₋₁₂, halogénoalcoxy en C₁₋₁₂, amino, (alkyl en C₁₋₁₂)amino, di(alkyl en C₁₋₁₂)amino, formyle, (alcoxy en C₁₋₁₂)carbonyle, carboxyle, alcanoyle en C₁₋₁₂, alkylthio en C₁₋₁₂, alkylsulfinyle en C₁₋₁₂, alkylsulfonyle en C₁₋₁₂, alkylsulfonato en C₁₋₁₂, carbamoyle, alkylamido en C₁₋₁₂, aryle en C₆₋₁₀ et aralkyle en C₇₋₁₁.

2. Composé selon la revendication 1 dans lequel R₁ représente un groupe alkyle en C₁₋₆, alcényle en C₂₋₆, cycloalkyle en C₃₋₆, alcoxy en C₁₋₆, (alkyl en C₁₋₆)amino ou aryle en C₆₋₁₄, chaque groupe étant éventuellement substitué par un ou plusieurs substituants choisis dans le groupe constitué des atomes d'halogène et des groupes nitro, cyano, hydroxyle, alkyle en C₁₋₄, halogénoalkyle en C₁₋₄, alcoxy en C₁₋₄ et halogénoalcoxy en C₁₋₄.

3. Composé selon la revendication 1 ou la revendication 2 dans lequel R₂ représente un atome d'halogène, un groupe hydroxyle ou un groupe alcoxy en C₁₋₄.

4. Composé selon l'une quelconque des revendications précédentes dans lequel R₃ représente un atome d'hydrogène ou un groupe alkyle en C₁₋₄.

5. Composé selon l'une quelconque des revendications précédentes dans lequel R₄ et R₅ représentent chacun indépendamment un atome d'hydrogène ou un groupe alkyle en C₁₋₆, aryle en C₆₋₁₄, aralkyle en C₇₋₁₈, hétérocyclyle à 3-10 chaînons ou (hétérocyclyl à 3-10 chaînons)-(alkyle en C₁₋₆), chaque groupe étant éventuellement substitué par un ou plusieurs substituants choisis dans le groupe constitué des atomes d'halogène et des groupes hydroxyle, halogénoalkyle en C₁₋₄, alcoxy en C₁₋₄, halogénoalcoxy en C₁₋₄, alkylthio en C₁₋₄, halogénoalkylthio en C₁₋₄, carboxyle, amino, carbamoyle, alkylamido en C₁₋₄ et -NH-CO-R', où R' est un hydrogène, un groupe alkyle en C₁₋₄ ou phényle, ou R₄ et R₅ ensemble représentent un groupe -(CH₂)ₙ- où n est un entier de 2 à 5.

6. Composé selon l'une quelconque des revendications précédentes dans lequel R₆ représente un atome d'hydrogène ou un groupe alkyle en C₁₋₄.

7. Composé selon l'une quelconque des revendications précédentes dans lequel R₇ représente un groupe -COOR₆, où R₆ est un atome d'hydrogène ou un groupe alkyle en C₁₋₄, et R₈ représente un groupe nitro, ou R₇ et R₈ ensemble représentent un groupe - C(O)-NH-.

8. Composé selon l'une quelconque des revendications précédentes, ledit composé répondant à la formule I ou II, 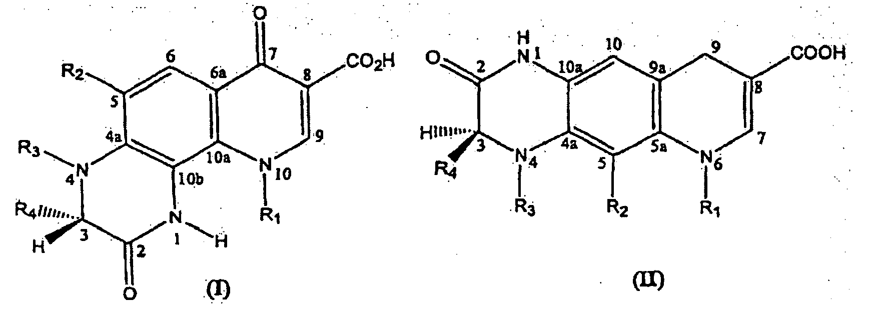 ou un de ses sels ou esters physiologiquement tolérés, dans lequel R₁ = *c-*C₃H₅, Et, 2,4-(F)₂C₆H₃;
R₂ = F, Cl, OH ou OMe;
R₃ = H, Me;
R₄ = H, un groupe alkyle en C₁₋₄ qui est éventuellement substitué par un groupe hydroxyle, alcoxy en C₁₋₄ ou alkylthio en C₁₋₄, -CH₂CO₂H, -CH₂CONH₂, -CH₂CH₂CO₂H, -CH₂CH₂CONH₂, -(CH₂)₄NHCOR' [R' = H, Me, Ph], Ph, *p*-(X)C₆H₄ [X = F, Cl, OH, OMe], PhCH₂, *p-*(OH)C₆H₄CH₂-, 2,4-(OH)₂C₆H₃CH₂-, 3-(indolyl)CH₂ ou 4-(imidazolyl)CH₂-, où Me est un groupe méthyle et Ph un groupe phényle.

9. Composé selon l'une quelconque des revendications 1 à 7, ledit composé répondant à la formule IA ou IIA, 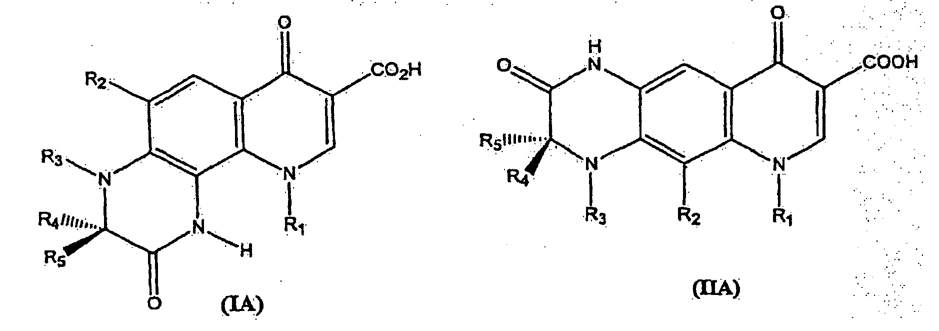 ou un de ses sels ou esters physiologiquement tolérés, dans lequel
R₁ = *c*-C₃H₅, Et, 2,4-(F)₂C₆H₃;
R₂ = F, Cl, OH ou OMe;
R₃ = H, Me;
R₄ = R₅ = Me, Ph, 2-Py, un groupe 2-thiényle ou 2-furyle, où Me est un groupe méthyle, Ph un groupe phényle et Py un groupe pyridyle.

10. Composé selon l'une quelconque des revendications 1 à 7, ledit composé répondant à la formule IB ou IIB: 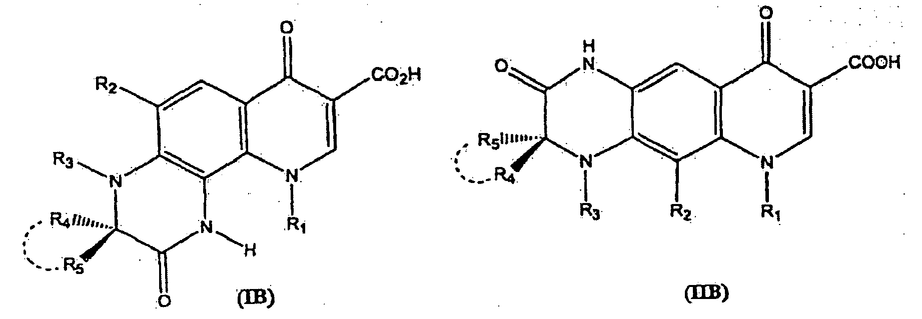 ou un de ses sels ou esters physiologiquement tolérés, dans lequel
R₁ = *c-*C₃H₅, Et, 2,4-(F)₂C₆H₃;
R₂ = F, Cl, OH ou OMe;
R₃ = H, Me;
R₄ et R₅ = [CH₂]ₙ, où n = 2, 3, 4 ou 5.

11. Composé selon l'une quelconque des revendications 1 à 7, ledit composé répondant à la formule III ou IV: 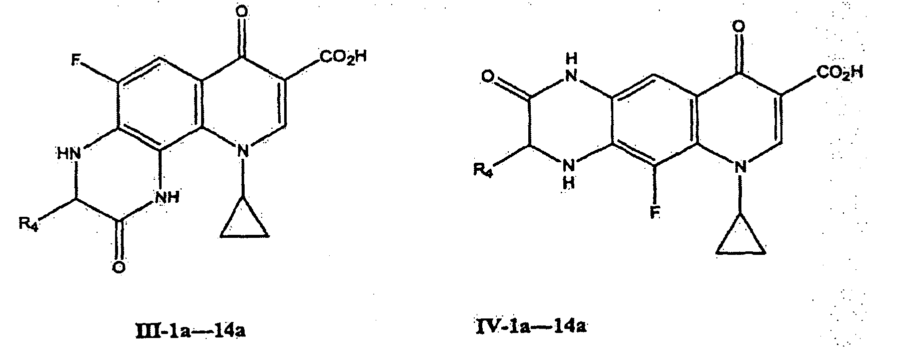 ou un de ses sels ou esters physiologiquement tolérés, dans lequel R₄ est tel que défini dans le tableau ci-dessous.
| Entrée III/IV | Acide α-aminé (L-) | R₄ |
|---|---|---|
| 1 | Gly | H |
| 2 | Ala | Me |
| 3 | Val | (Me)₂CH |
| 4 | Leu | (Me)₂CH₂CH |
| 5 | Ile | Et(Me)CH |
| 6 | Ser | CH₂OH |
| 7 | Thr | MeCHOH |
| 8 | Met | MeSCH₂CH₂ |
| 9 | N-Ac-Lys | Ac-NH(CH₂)₄ |
| 10 | Asp | CH₂CO₂H |
| 11 | Glu | CH₂CH₂CO₂H |
| 12 | Phe | CH₂Ph |
| 13 | Phénylglycine* | Ph* |
| 14 | Acide aminobutyrique* | Et* |
| | | |
|---|---|---|
| * non protéinique | | |

12. Composé selon l'une quelconque des revendications 1 à 7, ledit composé répondant à la formule IIIA ou IVA, 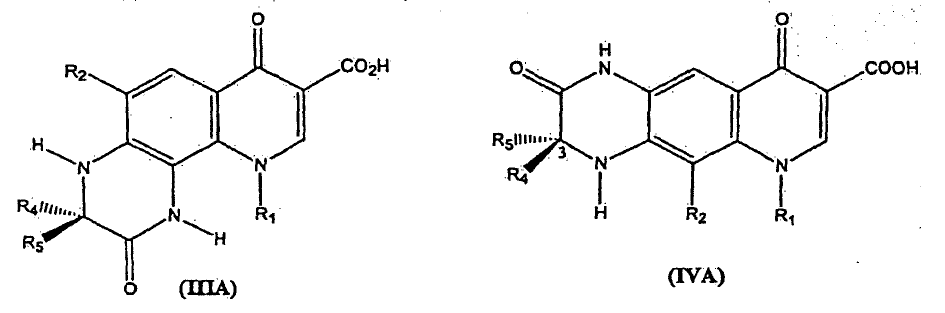 ou un de ses sels ou esters physiologiquement tolérés, dans lequel
R₁ = *c-*C₃H₅ ou Et;
R₂ = F, Cl, OH ou OMe;
R₄ = R₅: Me ou Ph.

13. Composé selon l'une quelconque des revendications 1 à 7, ledit composé répondant à la formule IIIB ou IVB: 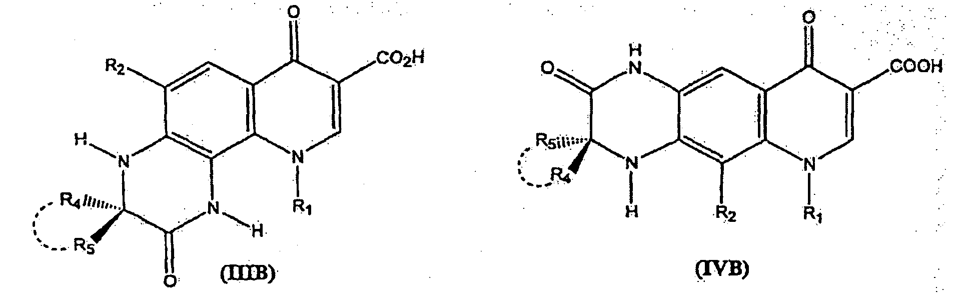 ou un de ses sels ou esters physiologiquement tolérés, dans lequel
R₁ = *c-*C₃H₅ ou Et;
R₂ = F, Cl, OH ou OMe;
R₄ et R₅ = [CH₂]₂, [CH₂]₃ ou [CH₂]₄.

14. Composé selon l'une quelconque des revendications 1 à 7, ledit composé répondant à la formule IX ou X 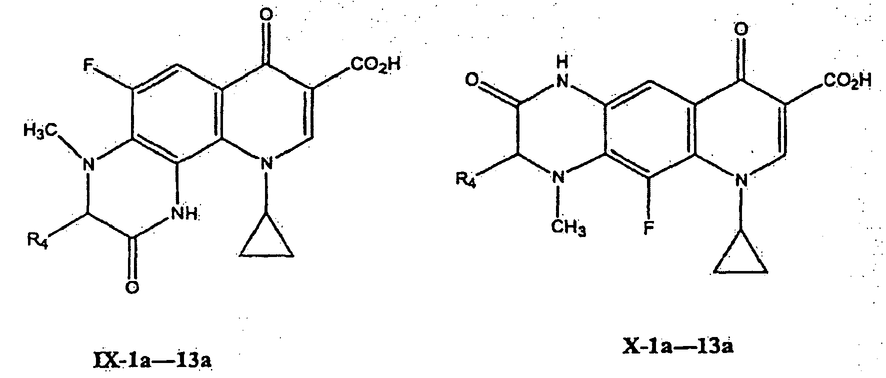 ou un de ses sels ou esters physiologiquement tolérés, dans lequel R₄ est tel que défini dans le tableau ci-dessous:
| Entrée IX/X | Acide N-Me-α-aminé (L-) | R₄ |
|---|---|---|
| 1 | N-Me-Gly (sarcosine) | H |
| 2 | N-Me-Ala | Me |
| 3 | N-Me-Val | (Me)₂CH |
| 4 | N-Me-Leu | (Me)₂CH₂CH |
| 5 | N-Me-Ile | Et(Me)CH |
| 6 | N-Me-Ser | CH₂OH |
| 7 | N-Me-Thr | MeCHOH |
| 8 | N-Me-Met | MeSCH₂CH₂ |
| 9 | N-Me-Asp | CH₂CO₂H |
| 10 | N-Me-Glu | CH₂CH₂CO₂H |
| 11 | N-Me-Phe | CH₂Ph |
| 12 | N-Me-Phénylglycine* | Ph* |
| 13 | Acide N-Me-aminobutyrique* | Et* |
| | | |
|---|---|---|
| * non protéinique | | |

15. Procédé de préparation d'un composé de formule générale A ou B tel que défini dans l'une quelconque des revendications précédentes qui comprend la mise en réaction d'un composé de formule générale 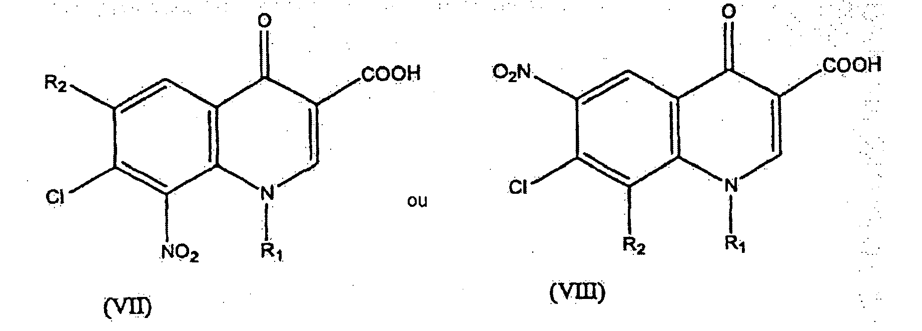 dans lesquelles R₁ et R₂ sont tels que définis dans l'une quelconque des revendications précédentes, avec un composé de formule générale 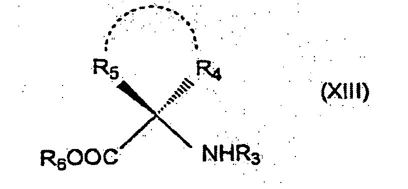 dans laquelle R₃, R₄, R₅ et R₆ sont tels que définis dans l'une quelconque des revendications précédentes, pour former un composé de formule générale A ou B dans lequel R₆ est un atome d'hydrogène, R₇ représente un groupe -COOR₆ et R₈ représente un groupe nitro;
si on le souhaite, l'exposition du composé obtenu à une cyclisation réductrice pour former un composé de formule générale A ou B dans lequel R₇ et R₈ ensemble représentent un groupe -C(O)-NR₉-, où R₉ est tel que défini dans l'une quelconque des revendications précédentes;
si on le souhaite, la mise en réaction du composé obtenu avec un agent alkylant pour former un composé de formule générale A ou B dans lequel R₆ est un groupe alkyle;
et/ou si on le souhaite, la conversion du composé obtenu en un de ses sels ou esters physiologiquement tolérés.

16. Procédé de préparation d'un composé de formule III ou IV telle que définie dans la revendication 11, qui comprend l'étape de cyclisation réductrice d'un composé respectif de formule V ou VI 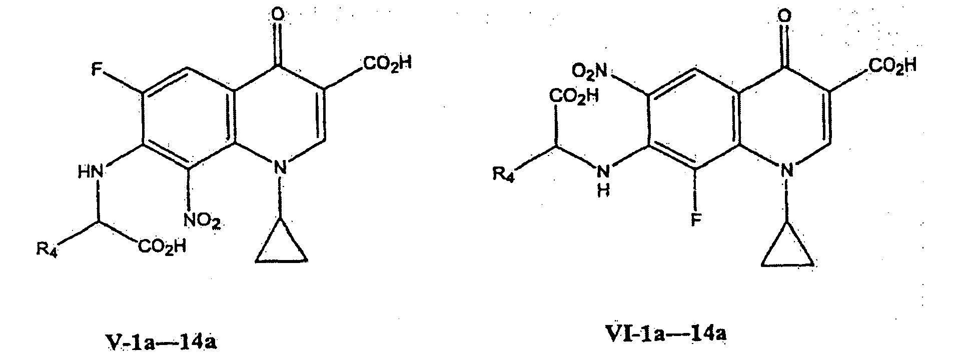 dans lesquelles R₄ est tel que défini dans la revendication 11, à l'aide de dithionite de sodium dans une solution aqueuse de carbonate de potassium à température ambiante.

17. Procédé de préparation d'un composé de formule V ou VI telle que définie dans la revendication 16, qui comprend l'étape de mise en réaction d'un composé de formule VII ou VIII respectivement, 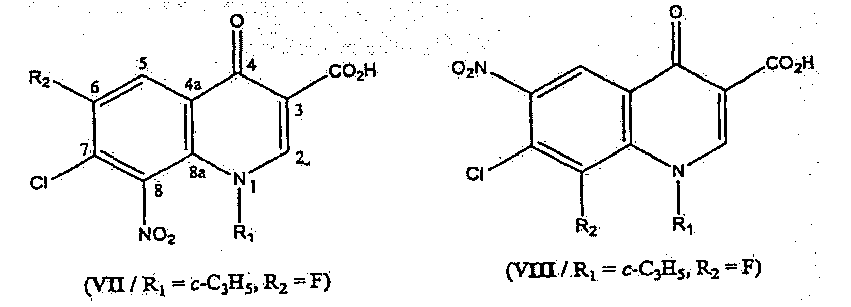 dans lesquelles R₁ et R₂ sont tels que définis dans la revendication 11, avec un acide α-aminé primaire particulier dans une solution aqueuse éthanolique d'hydrogénocarbonate de potassium à 60-90°C pendant 1 à 5 jours.

18. Procédé de préparation d'un composé de formule IIIA ou IVA telle que définie dans la revendication 12, qui comprend l'étape de cyclisation réductrice d'un composé respectif de formule VA ou VIA 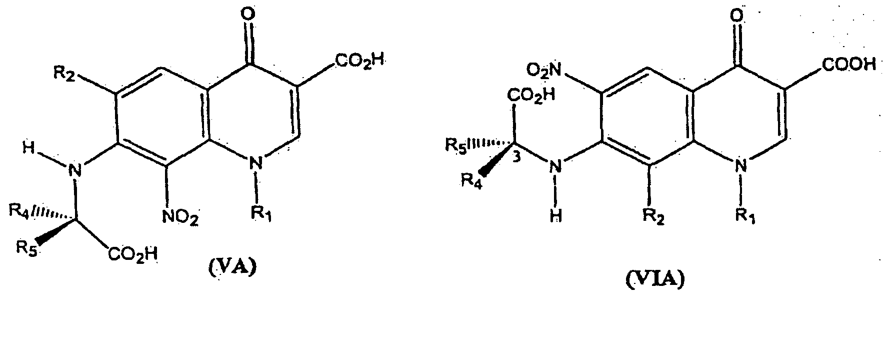 dans lesquelles R₁, R₂, R₄ et R₅ sont tels que définis dans la revendication 12, à l'aide de dithionite de sodium dans une solution aqueuse de carbonate de potassium à température ambiante.

19. Procédé de préparation d'un composé de formule VA ou VIA telle que définie dans la revendication 18, qui comprend l'étape de mise en réaction d'un composé de formule VII ou VIII telles que définies respectivement dans la revendication 17, avec un acide α-aminé primaire particulier dans une solution aqueuse éthanolique d'hydrogénocarbonate de potassium à 60-90°C pendant 1 à 5 jours.

20. Procédé de préparation d'un composé de formule IIIB ou IVB telle que définie dans la revendication 13, qui comprend l'étape de cyclisation réductrice d'un composé respectif de formule VB ou VIB 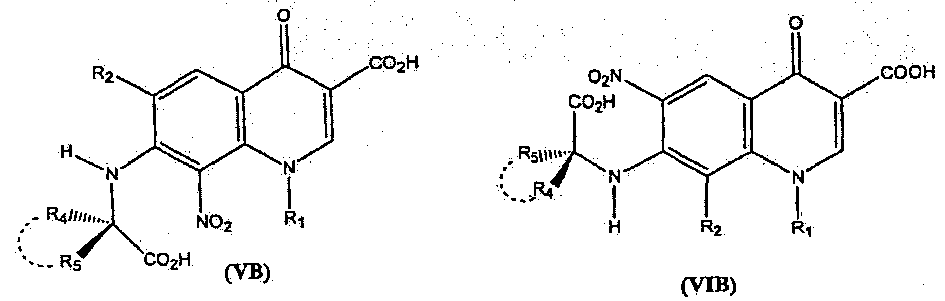 dans lesquelles R₁, R₂, R₄ et R₅ sont tels que définis dans la revendication 13, à l'aide de dithionite de sodium dans une solution aqueuse de carbonate de potassium à température ambiante.

21. Procédé de préparation d'un composé de formule VB ou VIB telle que définie dans la revendication 20, qui comprend l'étape de mise en réaction d'un composé de formule VII ou VIII telles que définies respectivement dans la revendication 17, avec un acide α-aminé primaire particulier dans une solution aqueuse éthanolique d'hydrogénocarbonate de potassium à 60-90°C pendant 1 à 5 jours.

22. Procédé de préparation d'un composé de formule IX ou X telle que définie dans la revendication 14, qui comprend l'étape de cyclisation réductrice d'un composé respectif de formule XI ou XII 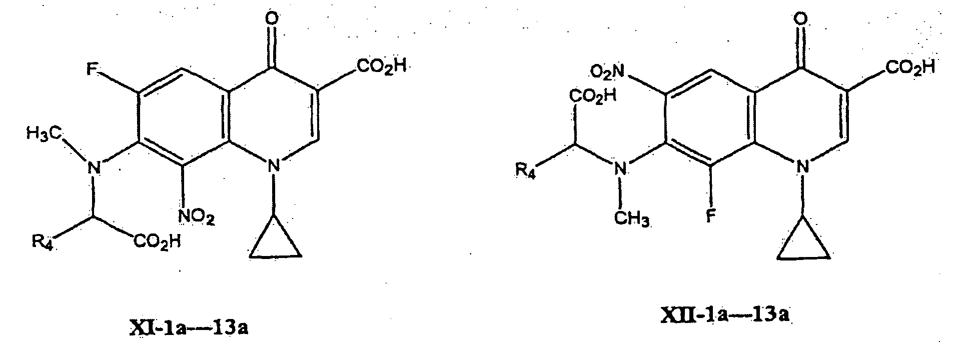 dans lesquelles R₄ est tel que défini dans la revendication 14, à l'aide de dithionite de sodium dans une solution aqueuse de carbonate de potassium à température ambiante.

23. Procédé de préparation d'un composé de formule XI ou XII telle que définie dans la revendication 22, qui comprend l'étape de mise en réaction d'un composé de formule VII ou VIII respectivement, avec un acide N-méthyl-α-aminé particulier dans une solution aqueuse éthanolique d'hydrogénocarbonate de potassium à 60-90°C pendant 1 à 5 jours.

24. Procédé de préparation d'un ester carboxylate de méthyle (en tant que promédicament de son composé carboxy) de formule I ou II telle que définie dans la revendication 8, IA ou IIA telle que définie dans la revendication 9, IB ou IIB telle que définie dans la revendication 10, 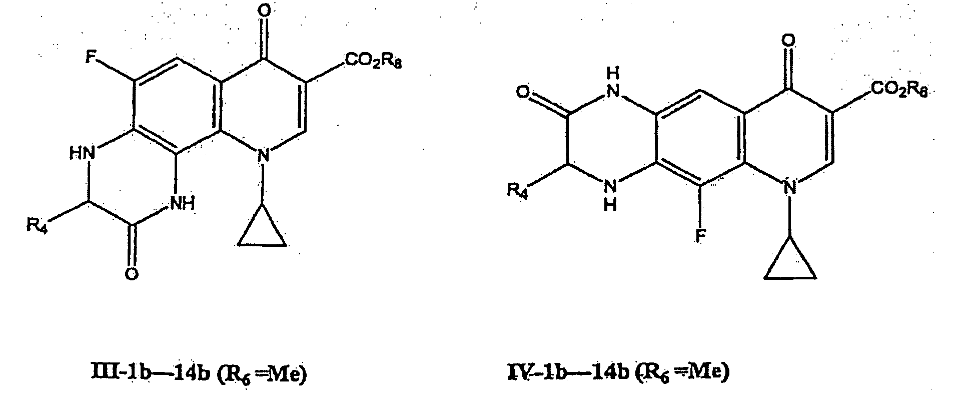 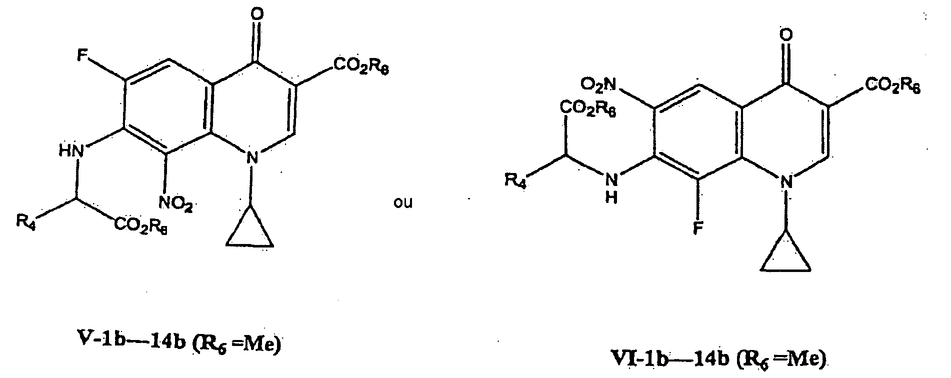 où
| Entrée III/IV/V/VI | Acide α-aminé (L-) | R₄ |
|---|---|---|
| 1 | Gly | H |
| 2 | Ala | Me |
| 3 | Val | (Me)₂CH |
| 4 | Leu | (Me)₂CH₂CH |
| 5 | Ile | Et(Me)CH |
| 6 | Ser | CH₂OH |
| 7 | Thr | MeCHOH |
| 8 | Met | MeSCH₂CH₂ |
| 9 | N-Ac-Lys | Ac-NH(CH₂)₄ |
| 10 | Asp | CH₂CO₂H |
| 11 | Glu | CH₂CH₂CO₂H |
| 12 | Phe | CH₂Ph |
| 13 | Phénylglycine* | Ph* |
| 14 | Acide aminobutyrique* | Et* |
| | | |
|---|---|---|
| * non protéinique | | |

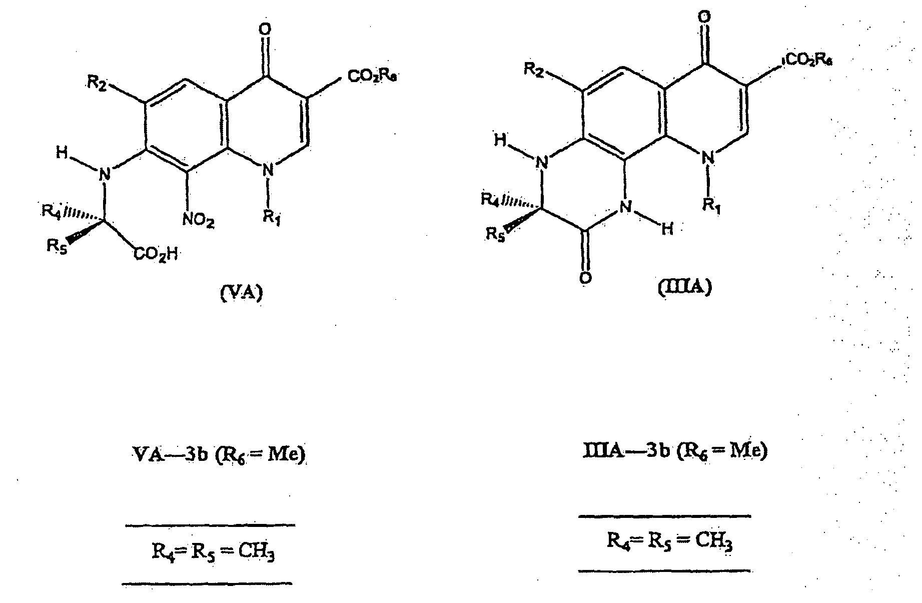

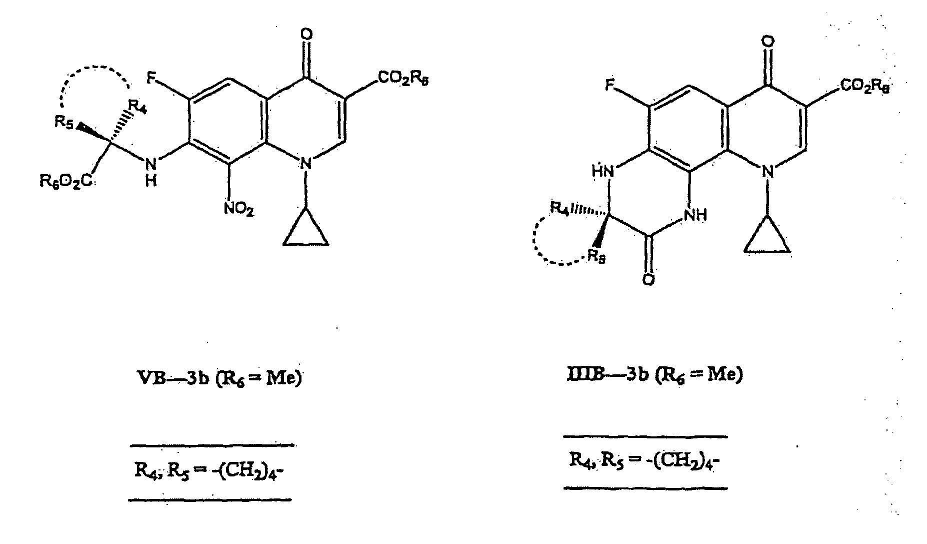

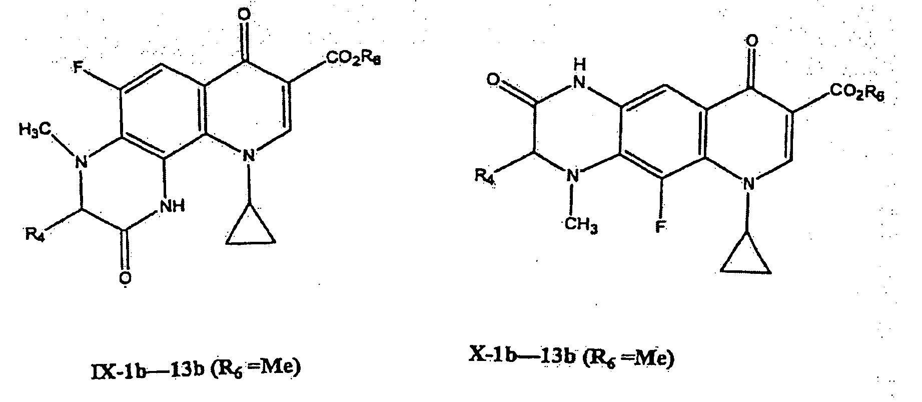

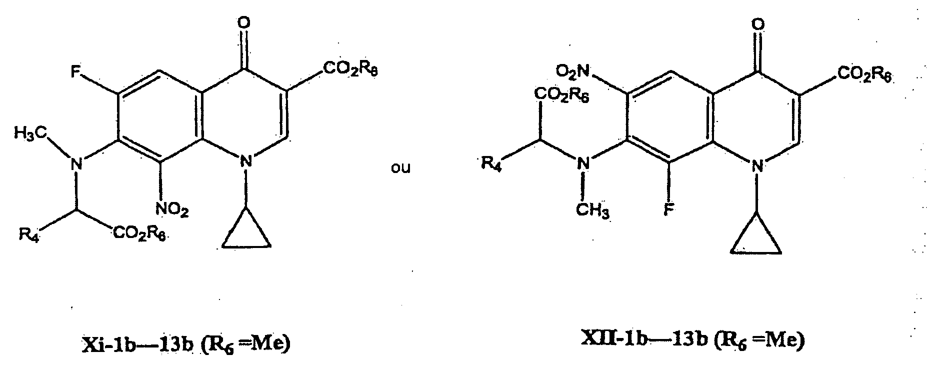
où
| Entrée IX/X/XI/XII | Acide N-Me-α-aminé (L-) | R₄ |
|---|---|---|
| 1 | N-Me-Gly (sarcosine) | H |
| 2 | N-Me-Ala | Me |
| 3 | N-Me-Val | (Me)₂CH |
| 4 | N-Me-Leu | (Me)₂CH₂CH |
| 5 | N-Me-Ile | Et(Me)CH |
| 6 | N-Me-Ser | CH₂OH |
| 7 | N-Me-Thr | MeCHOH |
| 8 | N-Me-Met | MeSCH₂CH₂ |
| 9 | N-Me-Asp | CH₂CO₂H |
| 10 | N-Me-Glu | CH₂CH₂CO₂H |
| 11 | N-Me-Phe | CH₂Ph |
| 12 | N-Me-Phénylglycine* | Ph* |
| 13 | Acide N-Me-aminobutyrique* | Et* |
| | | |
|---|---|---|
| * non protéinique | | |
qui comprend l'étape de mise en réaction du composé carboxy respectif avec du diazométhane éthérate à 0-10°C pendant 20 à 60 minutes.

25. Composition pharmaceutique comprenant un composé de formule générale A ou B ou un de ses sels ou esters physiologiquement tolérés tel que défini dans la revendication 1 et un véhicule.

26. Procédé de préparation d'une composition pharmaceutique selon la revendication 25 qui comprend la mise en association d'un composé de formule générale A ou B ou d'un de ses sels ou esters physiologiquement tolérés tel que défini dans la revendication 1 avec un véhicule.

27. Composé de formule générale A ou B ou un de ses sels ou esters physiologiquement tolérés tel que défini dans la revendication 1 destiné à être utilisé en médecine.

28. Composé selon la revendication 27 destiné à être utilisé en tant qu'agent antibactérien.

29. Composé de formule III, IIIB, V ou VB telles que définies respectivement dans la revendication 11, la revendication 13, la revendication 16 ou la revendication 20, destiné à être utilisé en tant qu'agent antibactérien, de préférence contre les bactéries à Gram négatif (E. *coli)* et les bactéries à Gram positif (S. *aureus*).

30. Composé de formule IX ou XI telles que définies respectivement dans la revendication 14 ou la revendication 22, destiné à être utilisé en tant qu'agent antibactérien, de préférence contre les bactéries à Gram négatif (E. *coli*) et les bactéries à Gram positif (S. *aureus*).

31. Utilisation d'un composé de formule générale A ou B ou d'un de ses sels ou esters physiologiquement tolérés tel que défini dans la revendication 1 pour la fabrication d'un médicament destiné à être utilisé en tant qu'agent antibactérien.

32. Procédé selon la revendication 15 dans lequel l'étape d'alkylation facultative est effectuée à l'aide de diazométhane éthérate à une température de 0 à 20°C, de préférence de 0 à 10°C, pendant 10 à 90, de préférence 20 à 60, minutes.
